# EUROPEAN PATENT APPLICATION

(11) **EP 1 454 989 A1**
(43) Date of publication of application: **08.09.2004**
(21) Application number: 03290505.1
(22) Date of filing: 03.03.2003
(51) Int. Cl.: C12N 15/86, A61K 39/12, C12N 7/04

(54) **Infectious HCV pseudo-particles containing native functional E1 and E2 envelope proteins**

(71) Applicant: INSTITUT NATIONAL DE LA SANTE ET DE LA RECHERCHE MEDICALE (INSERM), 75013 Paris (FR)
(72) Inventor: Bartosch, Birke, 38200 Chuzelles (FR); Cosset, Francois-Loic, 69007 Lyon (FR)
(74) Representative: Bernasconi, Jean

(57) **Abstract**

The invention relates to the generation and the use of hepacivirus pseudo-particles containing native functional E1, E2 envelope glycoproteins assembled onto retroviral core particles. These particles are highly infectious and constitute a valid model of hepacivirus virion.

## Description

The invention relates to the generation and the use of hepacivirus pseudo-particles containing native functional E1, E2 envelope glycoproteins assembled onto retroviral core particles. These particles are highly infectious and constitute a valid model of hepacivirus virion.

World-wide several hundred millions of people are infected with hepatitis C virus (HCV) (Lavanchy et al., 1999). Progression to chronic disease occurs in the majority of HCV infected persons. infection is associated with an increased risk for liver diseases and hepato-cellular carcinoma and has become the main indication for liver transplantation. HCV infection also increases the number of complications in HIV infected people (Dieterich, 2002). No vaccine is currently available to prevent new infections and the only treatment for chronic hepatitis C is interferon-α therapy, either alone or in combination with the guanosine analogue ribavirin. However, only ∼ 40% of patients respond to treatment. Clearly, novel therapeutic strategies are urgently required as the health costs for HCV infected people are predicted to spiral dramatically in the next few decades.

Hepacivirus proteins structural and non-structural proteins are expressed from a single polyprotein precursor and individually released in their respective cell compartments upon cleavage by cellular and viral proteases (Lindenbach and Rice, 2001). By analogy with other members of the Flaviviridae hepacivirus, and in particular HCV genomic organization suggests a virus consisting of a nucleocapsid comprising a viral genome and core protein (C) coated by a lipid envelop containing the two envelope glycoproteins E1 and E2.

So far, the study of hepatitis C virus (HCV), has been hampered by the low level of HCV particles in infected patients and by the lack of available efficient and reliable culture system for amplifying the virus (Lindenbach and Rice, 2001). Recently, a model for HCV replication, based on the self-replication of engineered minigenomes in cell culture, has been established (Blight et *al*., 2000; Lohmann *et al*., 1999). Although very useful to study HCV genomic replication, this system does not support production of HCV particles (Pietschmann *et al.,* 2002).

Production of HCV virus-like particles (VLP) in insect cells has already been reported (WO 98/21338, Wellnitz et *al*., 2002; Baumert et *al*.; 1998, Owsianka et *al*., 2001). However these particles were not secreted and their extraction from intracellular compartments yielded VLP preparations that were not infectious. Pseudotyped Vesicular Stomatitis Virus (VSV) viral particles have also been engineered with chimeric E1 and/or E2 glycoproteins whose transmembrane domains were modified to allow their transport to the cell surface (Buonocore et *al., 2002*; Matsuura et *al*., 2001). However, such modifications are likely to disturb conformation and functions of the E1E2 complexes (Matsuura et *al*., 2001) and although such pseudo-particles stand among candidate HCV vaccines (Rose et *al*., 2001), their use as a tool to investigate HCV assembly and cell entry remains controversial (Buonocore et *al*., 2002). Chimeric HCV-BVDV (Bovine Viral Diarrhea Virus) particles were shown to be infectious for the human hepatocyte line Huh-7 (WO 00/75352). However, infectivity could not be neutralised by an anti-serum against HCV which indicated that these particles did not constitute a valid model of HCV virion.

New approaches are therefore sorely needed to study HCV assembly and cell entry in order to design HCV cell entry inhibitors and to study the humoral immune response against HCV. Availability of infectious, amplifiable HCV particles would also provide useful material for the development of diagnostic application as well as therapeutical drugs.

The invention thus overcomes these hurdles by proposing infectious hepacivirus pseudo-particles, and in particular HCV pseudo-particles harboring unmodified E1 and E2 glycoproteins. The infectious pseudo-particles described herein further constitute a valid model of hepacivirus virions.

### Definitions

The terms *"vector", "cloning vector"* and *"expression vector"* mean the vehicle by which a DNA or RNA sequence (e.g. a foreign gene) can be introduced into a host cell, so as to transform the host and promote expression (e.g. transcription and translation) of the introduced sequence. Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a *"DNA construct".* A common type of vector is *a "plasmid",* which generally is a self-contained molecule of double-stranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence that initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts.

A *"coding sequence"* or a sequence *"encoding"* an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme.

The term *"transfection"* means the introduction of a foreign nucleic acid (DNA, cDNA or RNA) into a cell so that the host cell will express the introduced gene or sequence to produce a desired substance, typically a protein coded by the introduced gene or sequence. The introduced gene may include regulatory or control sequences, such as start, stop, promoter, signal, secretion, or other sequences used by a cell's genetic machinery. A host cell that receives and expresses introduced DNA or RNA has been *"transformed".*

The term *"host cell*" means any cell of any organism that is selected, modified, transformed, grown, or used or manipulated in any way, for the production of a substance by the cell, for example the expression by the cell of a gene, a DNA sequence, a protein, a virion. In the context of the invention, the host cell is a mammalian cell. Suitable host cells include Huh-7 human hepatocellular carcinoma (Nakabayashi et *al.,* 1982), Hep3B human hepatocellular carcinoma (ATCC HB-8064), HepG2 human hepatocellular carcinoma (HB-8065), HT-1080 human fibrosarcoma (CCL-121), 293T human embryo kidney cells (ATCC CRL-1573), TE671 human rhabdomyosarcoma (ATCC CRL-8805), Jurkat human T cell leukemia (TIB-152), CEM human lymphoblastic leukemia (CCL-119), COS-7 African green monkey fibroblasts kidney (CRL-1651), VERO African green monkey kidney (CCL-81), PG-4 feline astrocyte (CRL-2032), BHK-21 golden hamster kidney (CCL-10), CHO Chinese hamster ovary (ATCC CCL-61), and NIH3T3 mouse fibroblasts. In a specific embodiment said host cell is 293T.

As used herein, the term *"permissive cell"* is meant for a cell that is permissive for a hepacivirus infection.

*"Hepacivirus"* denotes hepatitis C virus, GB viruses, *i.e.* GB virus A, GB virus B, GB virus C, and GBV-A like agents, and hepatitis G virus. Preferably, said hepacivirus is hepatitis C virus (HCV). In the context of the invention, said hepacivirus may be of any specie, genotype and subtype, where appropriate, and variants thereof. *"Hepatitis C Virus"* or *"HCV"* is a member of the *Flaviviridae* family. HCV is the type specie of the genus hepacivirus. HCV genome, like other hepaciviruses genome, encodes a single polyprotein NH₂-C-E1-E2-P7-NS2-NS3-NS4a-NS4b-NS5a-NS5b-COOH that is processed co and post-translationally into both structural (N-terminal nucleocapsid protein termed "Core" (C), and glycoproteins E1 and E2) and non-structural (NS) proteins. The amino-terminal part of the polyprotein is cleaved by host cell proteases and its products, core and envelope (E1 and E2) proteins, are believed to be the major constituents of HCV particles (virions).

Although most cleavages in the polyprotein precursor proceed to completion during or immediately after translation, processing between E2 and p7, a hydrophobic domain found at the carboxy terminus of E2, is incomplete and results in the production of fully processed E2 and uncleaved E2-p7. The p7 polypeptide of HCV is a small hydrophobic protein which has not been well characterized yet. Indeed, the structure of this 63-amino-acid-long polypeptide has not been determined, and its putative function(s) remains unknown.

In the context of the invention, HCV is of any genotype, *e.g.* 1, 2, 3, 4, 5, 6, and subtype, *e.g.* a, b, c, d, e, f, g, h, k, and variants thereof.

The term *"variant"* refers to the homologous polynucleotide sequences and corresponding amino acid sequences found in the different HCV strains owing to HCV hypervariability.

Preferably, HCV has either 1a or 1b genotype (Dubuisson *et al.,* 1994) that stand among HCV genotypes that are the most prevalent and the most resistant to interferon-α therapy (Zein, 2000).

The term *"hepacivirus-like particles"* as used herein refers to non naturally occurring viral particles that comprise an envelope protein of an hepacivirus.

The term *"HCV-like particles"* or *"HCV pseudo-particles"* as used herein refers to non naturally occurring viral particles that comprise an envelope protein of HCV.

The hepacivirus, in particular HCV, pseudo-particles of the invention are infectious for a target cell. The particles of the invention more particularly comprise retroviral core proteins. Such particles may be readily produced by one skilled in genetic engineering techniques. One can for instance refer to EP 1 201 750 that describes production of synthetic retroviral particles expressing an antigen for modulating an immune response.

In the context of the invention, the term *"infectious"* is used to describe the capacity of the particles of the invention to complete the initial steps of viral cycle that lead to cell entry. However, upon interaction with the host cell, hepacivirus-like particles may or may not produce progeny viruses.

The term *"an envelope protein of a hepacivirus"* denotes the native E1 or E2 glycoprotein of a hepacivirus, or a mutant thereof.

By an *"E1 glycoprotein"* or *"E1 protein"* is meant an envelope 1 protein (E1) from any specie, genotype, subtype and variants of hepacivirus strains.

By an *"E2 glycoprotein"* or *"E2 protein"* is meant an envelope 2 protein (E2) from any specie, genotype, subtype and variants of hepacivirus strains.

Preferably, E1 and E2 glycoproteins are derived from a same hepacivirus strain. Preferably, E1 and E2 glycoproteins are native.

By a *"p7 protein"* is meant a native p7 protein, or a mutant thereof, from any specie, genotype, subtype and variants of hepacivirus strains. Preferably, p7 protein and E1 and/or E2 glycoproteins are derived from a same hepacivirus strain. Preferably, p7 protein and E1 and/or E2 glycoproteins are native.

The term *"an envelope protein of HCV"* denotes the E1 or E2 glycoprotein of HCV, or a mutant thereof.

By a *"HCV E1 glycoprotein"* or *"HCV E1 protein"* is meant an envelope 1 protein (E1) from any genotype, subtype and variant of HCV strains.

By a *"HCV E2 glycoprotein"* or *"HCV E2 protein"* is meant an envelope 2 protein (E2) from any genotype subtype and variant of HCV strains.

Preferably, HCV E1 and E2 glycoproteins are derived from a same HCV strain. Preferably, HCV E1 and E2 glycoproteins are native.

By a *"HCV p7 protein"* is meant a native p7 protein, or a mutant thereof, from any genotype, subtype and variant of HCV strains. Preferably, HCV p7 protein and E1 and/or E2 glycoproteins are derived from a same HCV strain. Preferably, HCV p7 protein and E1 and/or E2 glycoproteins are native.

The term *"mutant"* or *"mutation"* is meant for alteration of the DNA sequence that result in a modification of the amino acid sequence of native E1, E2, or p7 proteins. Such a modification can be for instance the substitution and/or deletion of one or more amino acids. Mutants notably include fragments of native E1, E2 and p7 proteins. Variants are particular examples of naturally occurring mutants. Mutants are more particularly contemplated as useful for identifying the structural elements of E1 and/or E2 proteins, and optionally p7 protein, necessary for maintaining cell infectivity or for increasing E1 and/or E2 antigenicity for vaccination purposes. In a preferred embodiment, the mutants encompass E2 glycoproteins wherein hypervariable region I has been deleted, while the particles produced therefrom remain infectious.

The term *"hepacivirus core"* is meant for a native core protein of a hepacivirus strains, a fragment thereof, or a variant thereof. According to an embodiment, the core protein is a N-terminally truncated form of hepacivirus core (ΔC) that comprises the core signal peptide.

As used herein, the term *"HCV core"* denotes a native core protein of the various HCV strains, a fragment thereof, or a variant thereof. The HCV core provides a signal peptide for the E1 or optionally E2 linked thereto that allows protein translocation to the endoplasmic reticulum. HCV core signal peptide corresponds to the last 21 residues of the carboxy-terminus of HCV core (GCSFSIFLLALLSCLTVPASA, SEQ ID N°1). According to an embodiment, the core protein is thus a N-terminally truncated form of HCV core (ΔC). Preferably ΔC comprises the last 21 residues of the carboxy-terminus of HCV core. In particular, HCV core may consist in the last 60 residues of the carboxy-terminus of HCV core.

In the context of the invention, the terms *"native"* or *"unmodified"* are indifferently used to describe a wild-type, full-length protein.

The term *"polyprotein"* as used herein is used to describe a protein construct made up of individual proteins that are joined together in a sequence whereby they retain their original relevant biological activities.

The term *"a polyprotein comprising a hepacivirus core protein linked to hepacivirus E1 protein and*/*or hepacivirus E2 protein",* or "*a polyprotein comprising successively a hepacivirus core protein, and* a *hepacivirus E1protein and*/*or hepacivirus E2 protein"* includes the CE1E2, CE2E1, CE1, CE2, ΔCE1E2, ΔCE2E1, ΔCE1, and ΔCE2 polyproteins. Optionally, said polyproteins further contain the p7 protein. The polyprotein comprising a hepacivirus core protein linked to hepacivirus E1 protein and/or hepacivirus E2 protein thus additionally includes the CE1 E2p7, CE2p7E1, CE1p7, CE2p7, ΔCE1E2p7, ΔCE2p7E1, ΔCE1p7, and ΔCE2p7 polyproteins. *"CE1E2"* denotes a polyprotein comprising successively a hepacivirus core protein, a hepacivirus E1 protein and a hepacivirus E2 protein. *"CE2E1"* denotes a polyprotein comprising successively a hepacivirus core protein, a hepacivirus E2 protein and a hepacivirus E1 protein. *"CE1"* denotes a polyprotein comprising a hepacivirus core protein linked to a hepacivirus E1 protein. *"CE2"* denotes a polyprotein comprising a hepacivirus core protein linked to a hepacivirus E2 protein. *"ΔCE1E2"* denotes a polyprotein comprising a carboxy terminus of hepacivirus core protein, and hepacivirus E1 and hepacivirus E2 proteins. *"ΔCE2E1"* denotes a polyprotein comprising a carboxy terminus of hepacivirus core protein, and hepacivirus E2 and hepacivirus E1 proteins. *"ΔCE1"* denotes a polyprotein comprising a carboxy terminus of hepacivirus core protein, linked to hepacivirus E1 protein. *"ΔCE2"* denotes a polyprotein comprising a carboxy terminus of hepacivirus core protein, linked to hepacivirus E2 protein: *ΔCE1E2,* as well as *ΔCE2,* have been built by inserting a stop codon at the end of E2, whereas *ΔCE2E1* and *ΔCE1* have been built by inserting a stop codon at the end of E1. *"CE1E2p7"* denotes a polyprotein comprising successively a hepacivirus core protein, a hepacivirus E1 protein, a hepacivirus E2 protein, and a hepacivirus p7 protein. *"CE2p7E1"* denotes a polyprotein comprising successively a hepacivirus core protein, a hepacivirus E2 protein, a hepacivirus p7 protein, and a hepacivirus E2 protein. *"CE1p7"* denotes a polyprotein comprising successively a hepacivirus core protein, a hepacivirus E1 protein, and a hepacivirus p7 protein. *"CE2p7"* denotes a polyprotein comprising successively a hepacivirus core protein, a hepacivirus E2 protein, and a hepacivirus p7 protein. *"ΔCE1E2p7"* denotes a polyprotein comprising a carboxy terminus of hepacivirus core protein, a hepacivirus E1 protein, a hepacivirus E2 protein, and a hepacivirus p7 protein. *"ΔCE2p7E1"* denotes a polyprotein comprising a carboxy terminus of hepacivirus core protein, a hepacivirus E2 protein, a hepacivirus p7 protein and a hepacivirus E1 protein. *"ΔCE1p7"* denotes a polyprotein comprising a carboxy terminus of hepacivirus core protein, a hepacivirus E1 protein, and a p7 protein. *"ΔCE2p7"* denotes a polyprotein comprising a carboxy terminus of hepacivirus core protein, a hepacivirus E2 protein, and a p7 protein. *ΔCE1E2p7, ΔCE1p7* and *ΔCE2p7,* have been built by inserting a stop codon at the end of p7 whereas ΔCE2p7E1 has been built by inserting a stop codon at the end of E1.

The term *"a polyprotein comprising a HCV core protein linked to HCV E1 protein and*/*or HCV E2 protein",* or "*a polyprotein comprising successively a HCV core protein and a HCV E1 protein and*/*or a HCV E2 protein",* includes the HCV CE1E2, CE2E1, CE1, CE2, ΔCE1E2, ΔCE2E1, ΔCE1, and ΔCE2 polyproteins. Optionally, said polyproteins further contain the p7 protein. The polyprotein comprising a HCV core protein linked to HCV E1 protein and/or HCV E2 protein thus additionally includes the HCV CE1E2p7, CE2p7E1, CE1p7, CE2p7, ΔCE1E2p7, ΔCE2p7E1, ΔCE1p7, and ΔCE2p7 polyproteins. *"HCV CE1E2"* denotes a polyprotein comprising successively a HCV core protein, a HCV E1 protein and a HCV E2 protein. *"HCV CE2E1"* denotes a polyprotein comprising successively a HCV core protein, a HCV E2 protein and a HCV E1 protein. *"HCV CE1"* denotes a polyprotein comprising a HCV core protein linked to a HCV E1 protein. *"HCV CE2"* denotes a polyprotein comprising a HCV core protein linked to a HCV E2 protein. *"HCV ΔCE1E2"* denotes a polyprotein comprising a carboxy terminus of HCV core protein, and HCV E1 and HCV E2 proteins. *"HCV ΔCE2E1"* denotes a polyprotein comprising a carboxy terminus of HCV core protein, and HCV E2 and HCV E1 proteins. *"HCV ΔCE1"* denotes a polyprotein comprising a carboxy terminus of HCV core protein, and a HCV E1 protein. *"HCV ΔCE2"* denotes a polyprotein comprising a carboxy terminus of HCV core protein, and a HCV E2 protein. *HCV ΔCE1E2,* as well as *HCV* Δ*CE2*, have been built by inserting a stop codon at the end of E2, whereas *ΔCE2E1* and *ΔCE1* have been built by inserting a stop codon at the end of E1. *"HCV CE1E2p7"* denotes a polyprotein comprising successively a HCV core protein, a HCV E1 protein, a HCV E2 protein, and a HCV p7 protein. *"HCV CE2p7E1"* denotes a polyprotein comprising successively a HCV core protein, a HCV E2 protein, a HCV p7 protein, and a HCV E2 protein. *"HCV CE1p7"* denotes a polyprotein comprising successively a HCV core protein, a HCV E1 protein, and a HCV p7 protein. *"HCV CE2p7"* denotes a polyprotein comprising successively a HCV core protein, a HCV E2 protein, and a HCV p7 protein. *"HCV ΔCE1E2p7"* denotes a polyprotein comprising a carboxy terminus of HCV core protein, a HCV E1 protein, a HCV E2 protein, and a HCV p7 protein. "*HCV ΔCF2p7E1*" denotes a polyprotein comprising a carboxy terminus of HCV core protein, a HCV E2 protein, a HCV p7 protein, and a HCV E1 protein. *"HCV ΔCE1p7"* denotes a polyprotein comprising a carboxy terminus of HCV core protein, a HCV E1 protein, and a p7 protein. *"HCV ΔCE2p7"* denotes a polyprotein comprising a carboxy terminus of HCV core protein, a HCV E2 protein, and a p7 protein. HCV ΔCE1E2p7, HCV ΔCE1p7, and HCV ΔCE2p7, have been built by inserting a stop codon at the end of p7 whereas ΔCE2p7E1 has been built by inserting a stop codon at the end of E1.

By *"retrovirus"* is meant a virus whose genome consists of a RNA molecule and that comprises a reverse-transcriptase, *i.e.* a member of the Retroviridae family. Retroviruses are divided into Oncovirus, Lentivirus and Spumavirus. Preferably said retrovirus is an oncovirus, *e.g*. MLV, ALV, RSV, or MPMV, a lentivirus, *e.g.* HIV-1, HIV-2, SIV, EIAV, or CAEV, or a spumavirus such as HFV. Genomes of these retroviruses are readily available in databanks.

In the context of the invention "*a nucleic sequence comprising a packaging competent retrovirus-derived genome"* is intended for a sequence that comprises the retroviral nucleic acid sequences known as "cis-acting" sequences. These include the Long Terminal Repeats (LTRs) for the control of transcription and integration, the psi sequence necessary for encapsidation, and the Primer Binding site (PBS) and polypurine track (PPT) sequences necessary for reverse transcription of th e retroviral genome. Advantageously, said nucleic acid sequence comprising a packaging competent retrovirus-derived genome further comprises a transgene.

Said retroviral genome may be replication-defective or replication-competent, in the absence of any trans-complementing function. A replication-competent genome would further comprise the gag, pol, and env retroviral genes. in a replication-defective genome, the viral genes gag, pol, and env are deleted. However, assembly of viral pseudo-particles may be achieved by providing another vector that comprises gag, pol and env but that is defective for the "cis" sequences. Their expression allows the encapsidation of the transgene, excluding the genes necessary for the multiplication of the viral genome and for the formation of complete viral particles.

As used herein, the term *"transgene"* designates the gene that is expressed in the target cell upon infection by the particles of the invention.

Examples of transgenes include a gene encoding a molecule of therapeutic interest, a marker gene, a gene coding for an immune modulator, an antigen, or a suicide gene.

A *"marker gene"* denotes a gene whose expression is detectable. For instance marker gene expression can generate a detectable signal, such as a fluorescence emission, a chromogenic reaction, or confer a growth advantage to the cells wherein it is expressed (antibiotic resistance genes).

An *"immune modulator"* refers to the product of a gene that modifies the activity of the immune system of a subject *in vivo.* Examples of immune modulators include cytokines, (*e.g.* interleukins, interferons, or haematopoietic colony stimulating factors), chemokines, and the like. Expression of an immune modulator by transformed cells may change the cellular environment and alter differentiation of immune cells and thus modify the type and the strength of immune response elicited against a given antigen.

An *"antigen"* refers to a molecule, such as a peptide, a polypeptide or a protein, against which an immune response is sought. Said antigen may be for instance a tumor, a bacterial, a pathogenic, a proteic, or a viral antigen.

A *"suicide gene"* is meant for a gene whose expression in cells induces programmed-cell death (apoptosis) such as the conditional Herpes Simplex virus type I thymidine kinase gene.

The *"core protein from a retrovirus"* refers to proteins encoded by the gag and pol genes. The gag gene encodes a polyprotein which is further processed by the retroviral protease into structural proteins that comprise the core. The pol gene encodes the retroviral protease, reverse-transcriptase, and integrase.

A *"pharmaceutically acceptable carrier"* refers to any vehicle wherein the vaccine composition according to the invention may be formulated. It includes a saline solution such as phosphate buffer saline. In general, a diluent or carrier is selected on the basis of the mode and route of administration, and standard pharmaceutical practice.

In the context of the present application, *"vaccination"* is intended for prophylactic or therapeutical vaccination. *"Therapeutical vaccination"* is meant for vaccination of a patient with HCV infection.

According to the invention, the term *"subject"* or *"patient"* is meant for any mammal likely to be infected with a hepacivirus, in particular with HCV. Human, chimpanzee, tamarin, and mice, especially human liver-xenogratfed mice are examples of hosts for hepaciviruses, and in particular HCV.

### Production of hepacivirus pseudo- particles

The inventors have generated infectious pseudo-particles that contain functional, and more particularly unmodified, hepacivirus glycoproteins, in particular HCV glycoproteins, assembled onto retroviral core particles. Hepacivirus (HCV) E1 E2, and optionally p7, are expressed from a polyprotein containing the core (C) protein or a fragment thereof, in particular the carboxy-terminus of the C protein, which served as signal peptide for E1 or E2, and the E1 and/or E2 glycoproteins.

The invention thus provides a method for producing hepacivirus-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a hepacivirus core protein, and a hepacivirus E1 protein and/or a hepacivirus E2 protein;
- transfecting host cells with said nucleic acid sequences and maintaining the transfected cells in culture for sufficient time to allow expression of the cDNAs to produce structural proteins from hepacivirus and retrovirus; and allowing the structural proteins to form virus-like particles.

The invention further provides a method for producing hepacivirus-like particles *in vivo,* which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a hepacivirus core protein, and a hepacivirus E1 protein and/or a hepacivirus E2 protein;
- transfecting cells of a subject *in vivo* with said nucleic acid sequences, to allow expression of the cDNAs to produce structural proteins from hepacivirus and retrovirus; and to allow the structural proteins to form virus-like particles.

Another aspect of the invention is the use of three nucleic acid sequences for the preparation of a medicament useful as a vaccine against a hepacivirus infection, *i.e.* hepatitis, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a hepacivirus core protein, and a hepacivirus E1 protein and/or a hepacivirus E2 protein ;
and, when transferred into cells of a subject, the nucleic acid sequences allow the production of structural proteins from hepacivirus and retrovirus, wherein the structural proteins form virus-like particles that are immunogenic.

Preferably, said third nucleic acid sequence comprises a cDNA encoding a polyprotein that further comprises a hepacivirus p7 protein. Thus, preferably said polyprotein comprises successively a hepacivirus core protein, a hepacivirus E1 protein and/or a hepacivirus E2 protein, and optionally a hepacivirus p7 protein.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator.

In the method of the invention, said polyprotein may comprise a hepacivirus core protein linked to a hepacivirus E1 protein, or a hepacivirus core protein linked to a hepacivirus E2 protein, or successively a hepacivirus core protein, a hepacivirus E1 protein and a hepacivirus E2 protein, or successively a hepacivirus core protein, a hepacivirus E2 protein and a hepacivirus E1 protein. Said polyprotein may further comprise successively a hepacivirus core protein, a hepacivirus E1 protein and a p7 protein, or successively a hepacivirus core protein, a hepacivirus E2 protein and a hepacivirus p7 protein, or successively hepacivirus core protein, a hepacivirus E1 protein, a hepacivirus E2 protein and a hepacivirus p7 protein, or successively a hepacivirus core protein, a hepacivirus E2 protein, a hepacivirus p7 protein, and a hepacivirus E1 protein.

According to an embodiment, E1 and/or E2, and optionally p7 protein, are native proteins. According to another embodiment, E1 and/or E2 glycoproteins, and optionally p7 protein, are mutated to obtain particles that are useful for characterizing the glycoprotein determinants for hepacivirus infectivity.

Preferably, said E1 and E2 glycoproteins are both derived from a same hepacivirus strain. Preferably, said E1 or E2 glycoprotein and p7 protein are both derived from a same hepacivirus strain. Still preferably, said E1 and E2 glycoproteins, and p7 protein are derived from a same hepacivirus strain.

According to another embodiment said hepacivirus core protein is a carboxy terminus form (ΔC) of hepacivirus core protein, comprising the core protein signal peptide.

Preferably said hepacivirus is a hepatitis C virus (HCV).

The invention thus provides a method for producing hepatitis C virus (HCV)-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid Sequence comprising a cDNA encoding a polyprotein comprising successively a HCV core protein, and a HCV E1 protein and/or a HCV E2 protein;
- transfecting host cells with said nucleic acid sequences and maintaining the transfected cells in culture for sufficient time to allow expression of the cDNAs to produce structural proteins from hepatitis C virus and retrovirus; and allowing the structural proteins to form virus-like particles.

The invention further provides a method for producing hepatitis C-virus (HCV)-like particles *in vivo,* which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a HCV core protein, and a HCV E1 protein and/or a HCV E2 protein;
- transfecting cells of a subject *in vivo* with said nucleic acid sequences, to allow expression of the cDNAs to produce structural proteins from hepatitis C virus and retrovirus; and to allow the structural proteins to form virus-like particles.

Another aspect of the invention is the use of three nucleic acid sequences for the preparation of a medicament useful as a vaccine against hepatitis C, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a HCV core protein, and a HCV E1 protein and/or a HCV E2 protein ;
and, when transferred into cells of a subject, the nucleic acid sequences allow the production of structural proteins from hepatitis C virus and retrovirus, wherein the structural proteins form virus-like particles that are immunogenic.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator

Preferably, said third nucleic acid sequence comprises a cDNA encoding a polyprotein that further comprises a HCV p7 protein. Thus, preferably said polyprotein comprises successively a HCV core protein, a HCV E1 protein and/or a HCV E2 protein, and optionally a HCV p7 protein.

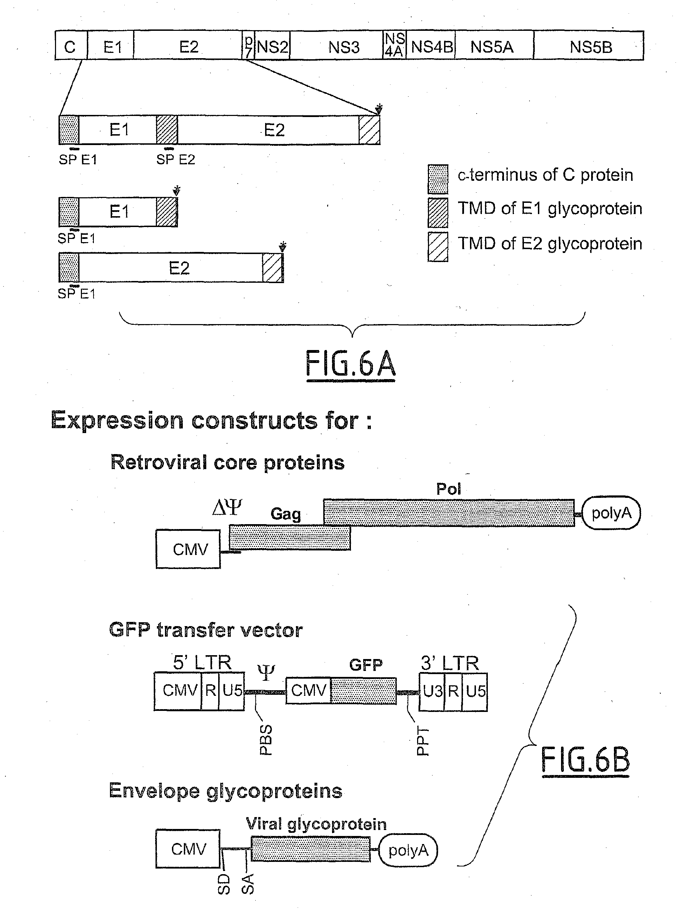

An example of HCV E1E2 and retroviral expression constructs is shown in figures 6A and 6B.

In the method of the invention, said polyprotein may comprise a HCV core protein linked to a HCV E1 protein, a HCV core protein linked to a HCV E2 protein, or successively a HCV core protein, a HCV E1 protein and a HCV E2 protein, or successively a HCV core protein, a HCV E2 protein and a HCV E1 protein. Said polyprotein may further comprise successively a HCV core protein, a HCV E1 protein and a p7 protein, or successively a HCV core protein, a HCV E2 protein and a HCV p7 protein, or successively HCV core protein, a HCV E1 protein, a HCV E2 protein and a HCV p7 protein, or successively a HCV core protein, a HCV E2 protein, a HCV p7 protein, and a HCV E1 protein.

According to an embodiment, HCV E1 and/or E2, and optionally HCV p7 protein, are native proteins. According to another embodiment, HCV E1 and/or E2 glycoproteins, and optionally HCV p7 protein, are mutated to obtain particles that are useful for characterizing the glycoprotein determinants for HCV infectivity.

Preferably, said E1 and E2 glycoproteins are both derived from a same HCV strain. Preferably, said E1 or E2 glycoprotein, and p7 protein are both derived from a same HCV strain. Still preferably, said E1 and E2 glycoproteins, and p7 protein are derived from a same HCV strain.

According to another embodiment said HCV core protein is a carboxy terminus form (ΔC) of HCV core protein. In particular, said HCV core protein may comprise the last 21 amino acids of the carboxy-terminus of HCV core.

For the purpose of transfection, said first, second and third nucleic acid sequences may be carried on a same vector, or on two or three separated vectors.

In particular, plasmoviruses, adenoretroviruses and replicating pseudo-viruses are examples of vectors suitable for carrying the above-mentioned sequences. A plasmovirus vaccine consists in such a plasmid DNA preparation, that allow expression of hepacivirus pseudo-particles after administration in an patient in order to elicit a immune response against said hepacivirus. Administration of such a plasmovirus vaccine being achieved for preventive vaccination into people at risk for hepacivirus-induced disease or for therapeutic vaccination into hepacivirus-infected patients. Adenoretroviruses consist in an alternative way to provide the above-mentioned nucleic acid sequences encoding hepacivirus pseudo-particles. in this case, it is possible to design three independent adenoretroviruses, *i.e*. recombinant adenoviruses, that encode the three nucleic acid sequences mentioned above (retroviral core and genome and hepacivirus glycoproteins), or, alternatively, it is also possible to design a single adenoretrovirus, derived from "guttless" recombinant adenoviruses, that contains the different nucleic acid sequences. Such adenoretroviruses can be administered to patient as for plasmoviruses, in order to elicit an anti-hepacivirus immune response. Replicating pseudo-retroviruses are another alternative possibility to express all the above-mentioned nucleic acid sequences encoding the hepacivirus pseudo-particles. Such structures are in fact hepacivirus pseudo-particles whose genome is engineered to allow, following infection, its propagation into cells of an inoculated patient, thereby inducing the production of further replicating hepacivirus-pseudo-particles. In this case the genome of a retrovirus is modified so as to express the hepacivirus E1 E2 glycoproteins in place of the retroviral Env gene (encoding the retroviral glycoproteins). The genes encoding the retroviral core proteins are left unchanged. Futhermore an additional gene, encoding a marker gene or an immunomodulator, for example, can be expressed from this genome.

In accordance with the present invention there may be employed conventional molecular biology, microbiology, and recombinant DNA techniques within the skill of the art. Such techniques are explained fully in the literature. See, *e.g*., Sambrook et *al*., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et *al*., 1994.

In particular, the vectors of the invention may be introduced into the target cell by means of any technique known for the delivery of nucleic acids to the nucleus of cells, either in culture, *ex vivo,* or *in vivo.*

Introduction of the nucleic acid sequences may be performed by any standard method well known by one skilled in the art, *e.g.* transfection, electroporation, microinjection, transduction, cell fusion, DEAE dextran, calcium phosphate precipitation, or use of a gene gun (see for instance Wu et al., 1992 ; Wu et al, 1988).

The donor nucleic acid targeting system can also be introduced by lipofection. In certain embodiments, the use of liposomes and/or nanoparticles is contemplated for the introduction of the donor nucleic acid targeting system into host cells. Nanocapsules can generally entrap compounds in a stable and reproducible way. Ultrafine particles (sized around 0.1 µm) that can be designed using biodegradable polyalkyl-cyanoacrylate polymers are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The use of cationic lipids may promote encapsulation of negatively charged nucleic acids, and also promote fusion with negatively charged cell membranes (Felgner et al., 1989).

*In vivo* targeted gene delivery is described in international patent publication WO 95/28 494. Alternatively, the vector can be introduced *in vivo* by lipofection, using liposomes or nanoparticles as above described. It is also possible to introduce the vector *in vivo* using techniques that are similar to the techniques that are employed *in vitro* (e.g. transfection, electroporation...).

### Transformed cells

The invention further relates to a transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a hepacivirus core protein, and a hepacivirus E1 protein and/or a hepacivirus E2 protein.

Pfreferably, said third nucleic acid sequence comprises a cDNA encoding a polyprotein that further comprises a hepacivirus p7 protein. Thsu, preferably said polyprotein comprises successively a hepacivirus core protein, a hepacivirus E1 protein and/or a hepacivirus E2 protein, and optionally a hepacivirus p7 protein.

Such a transformed host cell is obtainable as described in a method above.

In another aspect, the invention relates to the use of a transformed host cell as defined above, for the identification of molecules capable of interfering with hepacivirus entry in cells. The invention provides in particular a method of *ex vivo* screening or identification of molecules capable of interfering with hepacivirus entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule; under conditions that allow syncytia formation, *i.e.* cell-cell fusion, and hepacivirus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with hepacivirus entry the candidate molecule for which syncytia formation, as measured in the presence of said molecule, is decreased as compared to syncytia formation measured in the absence of any candidate molecule.

Preferably, said hepacivirus is a hepatitis C virus. The invention thus also relates to a transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a HCV core protein, and a HCV E1 protein and/or a HCV E2 protein.

According to another embodiment, said third nucleic acid sequence comprises a cDNA encoding a polyprotein that further comprises a HCV p7 protein. Preferably said polyprotein comprises successively a HCV core protein, a HCV E1 protein and/or a HCV E2 protein, and optionally a HCV p7 protein.

Such a transformed host cell is obtainable as described in a method above.

In another aspect, the invention relates to the use of a transformed host cell as defined above, for the identification of molecules capable of interfering with HCV entry in cells. The invention provides in particular a method of *ex vivo* screening or identification of molecules capable of interfering with HCV entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule, under conditions that allow syncytia formation, *i.e.* cell-cell fusion, and HCV-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with HCV entry the candidate molecule for which syncytia formation, as measured in the presence of said molecule, is decreased as compared to syncytia formation measured in the absence of any candidate molecule.

Contacting a transformed host cell with a target host cell, and a candidate molecule can be carried out by contacting simultaneously said transformed host cell, target host cell and candidate molecule. Otherwise, two of these three elements can be contacted under conditions sufficient to allow their interaction before addition of the third missing element.

Preferably said target host cell is not transformed , i.e. said target host cell does not contains at least one of the first, second, and third nucleic acid sequence as defined above.

Syncytia formation can be readily assessed by one skilled in the art. Briefly, the coculture is submitted to a acidic pH drop by incubation for 5 min at pH-5 and incubated in a nomal medium for an additional 12 hrs. Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

### Hepacivirus-like particles

In the method described above no structural modifications of the E1 E2 glycoproteins are required for their correct assembly on retroviral cores. The method of the invention thus makes it possible to generate high titre infectious hepacivirus pseudo-particles, and in particular HCV pseud-particles, with functional E1E2 glycoproteins. As demonstrated herein, these particles constitute a valid model of hepacivirus virions, and in particular of HCV virions, as regards to early steps of viral infection cycle.

The invention further relates to an infectious hepacivirus-like particle, comprising the core proteins from a retrovirus, E1 and/or E2 hepacivirus glycoprotein(s), and optionally hepacivirus p7 protein. Such a particle is obtainable by a method as described above.

According to an embodiment, the infectious particle of the invention may comprise native hepacivirus E1 protein, or native hepacivirus E2 protein, or native hepacivirus E1 protein and native hepacivirus E2 protein. Preferably said E1 and E2 glycoproteins are both derived from a same hepacivirus strain. According to another embodiment, E1 and/or E2 glycoproteins are mutated.

According to another embodiment the infectious particle of the invention may comprise native hepacivirus E1 and native hepacivirus p7 proteins, or native hepacivirus E2 and native hepacivirus p7 proteins, or native hepacivirus E1 protein, native hepacivirus E2 protein and native hepacivirus p7 protein Preferably, said E1 or E2 glycoprotein and p7 protein are both derived from a same hepacivirus strain. Still preferably, said E1 and E2 glycoproteins, and p7 protein are derived from a same hepacivirus strain. According to another embodiment, E1 and/or E2 glycoproteins and/or p7 protein are mutated.

Preferably, said hepacivirus is a hepatitis C virus. The invention thus also relates to an infectious HCV-like particle, comprising the core proteins from a retrovirus, E1 and/or E2 HCV glycoprotein(s), and optionally p7 protein. Such a particle is obtainable by a method as described above.

According to an embodiment, the infectious particle of the invention may comprise native HCV E1 protein, or native HCV E2 protein, or native HCV E1 protein and native HCV E2 protein. Preferably said E1 and E2 glycoproteins are both derived from a same HCV strain. According to another embodiment, E1 and/or E2 glycoproteins are mutated.

According to another embodiment the infectious particle of the invention may comprise native HCV E1 and native HCV p7 proteins, or native HCV E2 and native HCV p7 proteins, or native HCV E1 protein, native HCV E2 protein and native HCV p7 protein Preferably, said E1 or E2 glycoprotein and p7 protein are both derived from a same HCV strain. Still preferably, said E1 and E2 glycoproteins, and p7 protein are derived from a same HCV strain. According to another embodiment, E1 and/or E2 glycoproteins and/or p7 protein are mutated.

In a particular embodiment, the hypervariable region I (located in the N-terminus region, *i.e*. the first 27 aminoacids of the E2 protein after the signal peptide) is deleted. The HCV-like particles (called ΔHVR1) produced with E2 protein deleted from this region are particularly advantageous as a diagnostic tool or in vaccination, to enhance induction or binding of neutralizing antibodies. The ΔHVR1 HCV-like particles are also of interest to identify the epitopes/mechanisms within the HCV glycoproteins that can be targeted/inhibited by neutralizing antibodies or other therapeutic agents.

Said retrovirus may be selected from the group consisting of MLV, ALV, RSV, MPMV, HIV-1, HIV-2, SIV, EIAV, CAEV, and HFV.

Advantageously, said infectious particles further carry a transgene. For instance said transgene may be a marker gene which make it possible to follow-up cell infection by the infectious particles of the invention and can find application for instance in the identification of a cell receptor involved in hepacivirus entry, and in particular HCV entry. Said transgene can also be a gene encoding a molecule of therapeutic interest and/or a suicide gene. Accordingly, the particles of the invention that specifically target primary or cancerous hepatocytes comprise a useful vector for gene transfer and/or gene therapy.

### Use of the infectious hepacivirus-like particles of the invention

High infectivity of these particles makes it possible for the investigation of the role of hepacivirus (HCV) E1 and E2 glycoproteins and their potential receptors in cell entry, heapcivirus host-range and neutralisation by antibodies from hepacivirus patient sera. These particles makes it possible for the investigation of the role of p7 protein in E1 E2 maturation and functions, viral assembly, budding and release.

The invention therefore concerns the use of a hepacivirus-like infectious particle as described above, for *ex vivo* identification of a cell receptor for hepacivirus E1 and/or E2 glycoprotein.

According to an embodiment, the invention provides a method for *ex vivo* identification of a receptor for hepacivirus E1 and/or E2 glycoprotein comprising detection of the binding of said particle to a cell receptor. More specifically, the method may comprise the steps consisting of:
- contacting a cell susceptible to hepacivirus infection with an infectious hepacivirus-like particle of the invention, under conditions sufficient to allow specific binding of said particle to a receptor expressed at the surface of said cell;
- detecting binding of said particle to a receptor; and
- identifying said receptor.

Preferably, said hepacivirus is a hepatitis C virus. The invention therefore concerns the use of an HCV-like infectious particle as described above, for *ex vivo* identification of a cell receptor for HCV E1 and/or E2 glycoprotein.

According to an embodiment, the invention provides a method for *ex vivo* identification of a receptor for HCV E1 and/or E2 glycoprotein comprising detection of the binding of said particle to a cell receptor. More specifically, the method may comprise the steps consisting of:
- contacting a cell susceptible to HCV infection with an infectious HCV-like particle of the invention, under conditions sufficient to allow specific binding of said particle to a receptor expressed at the surface of said cell;
- detecting binding of said particle to a receptor; and
- identifying said receptor.

A cell susceptible to a hepacivirus infection, and in particular to a HCV infection, may preferably be selected from the group consisting of a hepatocyte cell line, such as Huh-7 human hepatocellular carcinoma (Nakabayashi et *al.*, 1982), PLC/PRF/5 human hepatoma (CRL-8024), Hep3B human hepatocellular carcinoma (ATCC HB-8064), or HepG2 human hepatocellular carcinoma (HB-8065), and a primary human hepatocyte. Primary human hepatocytes may be isolated from human adult biopsy samples according to procedures well-known by one skilled in the art. One can for example refer to Guguen-Guillouzo and Guillouzo (1986). Otherwise such cells are commercially available, and can be purchased for instance from Biopredic International (Rennes, France).

Detection of particle binding to a receptor can be achieved according to classical procedures well known by one skilled in the art. For instance, this could involve radioactive, enzyme or fluorescent labelling of the particles of the invention, and subsequent detection with an appropriate method. A number of fluorescent materials are known and can be utilized as labels. These include, for example, fluorescein, rhodamine, auramine, Texas Red. Enzyme labels consist in conjugation of an enzyme to a molecule of interest, *e.g*. a polypeptide, and can be detected by any of colorimetric, spectrophotometric, or fluorospectrophotometric techniques. Flow cytometry analysis (FACS) together with labelled antibodies directed against E1 or E2 proteins harboured by the pseudo-particles of the invention is also appropriate.

According to another embodiment, the invention provides a method for *ex vivo* identifying a cell receptor for a hepacivirus comprising the step consisting of:
- transfecting a cell which is not permissive for hepacivirus infection with a nucleic acid sequence encoding a protein likely to be a receptor for hepacivirus;
- contacting said transformed cell with a hepacivirus-like particle of the invention;
- determining whether said transformed cell has become permissive or not for hepacivirus infection; and
- identifying as a cell receptor for a hepacivirus said protein expressed by the transformed cell that has become permissive.

Preferably, the invention provides a method for *ex vivo* identifying a cell receptor for HCV comprising the step consisting of:
- transfecting a cell which is not permissive for HCV infection with a nucleic acid sequence encoding a protein likely to be a receptor for HCV;
- contacting said transformed cell with a HCV-like particle of the invention;
- determining whether said transformed cell has become permissive or not for HCV infection; and
- identifying as a cell receptor for HCV said protein expressed by the transformed cell that has become permissive.

Determination of whether the transformed cell has become permissive for hepacivirus infection, and in particular HCV infection, can be readily achieved using the hepacivirus-like (HCV-like) particles of the invention. In particular, where said particles carry a marker gene, such as GFP, permissivity *(i.e.* the capacity of cells to be infected with a hepacivirus, or with a hepacivirus-like particle, in particular with HCV or with HCV-like particles) can be assessed by FACS analysis of the transformed cells. Where the marker gene is an antibiotic resistance gene, identification of cells infected by the hepacivirus-like (HCV-like) particle is readily achieved through exposure to said antibiotic.

Where one does not suspect a given protein to be a receptor for hepacivirus entry, in cells, the above method can advantageously be adapted for the screening and the identification of a cell receptor for a hepacivirus, such as HCV. In particular, an expression cDNA library can be prepared, for instance from a cDNA library obtained by reverse-transcription of cellular mRNAs from a cell permissive for hepacivirus infection, in particular for HCV infection. Expression of such a cDNA library would be driven by a constitutive promoter whose nucleic acid sequence has been fused to the cDNA library in suitable vectors. Such a library would contain a vector encoding a cell receptor for a hepacivirus, for instance for HCV. Non permissive cells can then be transfected with this expression library and further screened for the identification of a cell receptor for a hepacivirus.

To this end, the invention proposes a method for *ex vivo* identifying a cell receptor for hepacivirus comprising the step consisting of:
- providing an expression cDNA library obtained from a cell permissive for hepacivirus infection;
- transfecting cells that are not permissive for hepacivirus infection with said expression cDNA library;
- contacting said transformed cells with hepacivirus-like particles of the invention;
- identifying and isolating those transformed cells that have become permissive for hepacivirus infection;
- isolating the expression vector transfected in cells that have become permissive; and
- identifying as a receptor for hepacivirus the proteins encoded by the cDNA sequence of said isolated expression vectors.

Preferably said hepacivirus is a hepatitis C virus. The invention thus proposes a method for *ex vivo* identifying a cell receptor for HCV comprising the step consisting of:
- providing an expression cDNA library obtained from a cell permissive for HCV infection;
- transfecting cells that are not permissive for HCV infection with said expression cDNA library;
- contacting said transformed cells with HCV-like particles of the invention;
- identifying and isolating those transformed cells that have become permissive for HCV infection;
- isolating the expression vector transfected in cells that have become permissive; and
- identifying as a receptor for HCV the proteins encoded by the cDNA sequence of said isolated expression vectors.

Determination of whether the transformed cell has become permissive for hepacivirus (HCV) infection can be readily achieved using the hepacivirus-like (HCV-like) particles of the invention. In particular, where said particles carry a marker gene, such as GFP, permissivity (*i.e*. the capacity of cells to be infected with hepacivirus, and in particular with hepacivirus-like particles, for instance with HCV or with HCV-like particles) can be assessed by FACS analysis of the transformed cells. Where the marker gene is an antibiotic resistance gene, identification of cells infected by hepacivirus-like particles, in particular by HCV-like particles, is readily achieved through exposure to said antibiotic.

Advantageously, the expression cDNA library is expressed from retroviral vectors that comprise glycoproteins that allow infection of the hepacivirus (HCV) non permissive cells. Such glycoproteins can be the VSV-G glycoprotein derived from vesicular stomatitis virus (VSV) whose receptor is expressed in most cell types *ex vivo.* Such viral particles can be assembled using a packaging competent retrovirus-derived genome that comprises the expression cDNA library, and optionally a marker gene. According to this embodiment the method for isolating the expression vector expressed in cells that have become permissive to infection by the hepacivirus-like (HCV-like) particles of the invention is greatly facilitated. Indeed this latter embodiment is particularly advantageous in that the process of cell infection with retroviral vectors has greater efficacy, as compared to cell transfection. Furthermore, cell infection leads to stable integration of viral genome in the cellular genome. Accordingly, transgenes, *i.e.* cDNA and marker gene that are carried by the pseudo-particles of the invention, are found to be stably expressed by infected cells. This in contrast with classical vectors used for transfection that do not integrate into cellular genome and for which expression may be transient.

In another aspect, the invention relates to the use of an infectious particle as defined above, for the identification of molecules capable of interfering with hepacivirus, and in particular HCV, entry in cells.

In particular, herein is provided a method of *ex vivo* screening or identification of molecules capable of interfering with hepacivirus entry in cells comprising comparison of the level of cell infection by the particles of the invention in the presence or the absence of a candidate molecule. Said method preferably comprises the steps consisting of:
- contacting a cell susceptible to hepacivirus infection with an infectious hepacivirus-like particle, in the absence or presence of a candidate molecule, under conditions that allow cell infection with hepacivirus-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with hepacivirus entry the candidate molecule for which cell infectivity, as measured in the presence of said molecule, is decreased as compared to cell infectivity measured in the absence of any candidate molecule.

Contacting a cell susceptible to hepacivirus infection with an infectious hepacivirus-like particle, and a candidate molecule can be carried out by contacting simultaneously said cell, hepacivirus-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious hepacivirus-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with hepacivirus cell entry may be selected from the group consisting of a hepatocyte cell line and a primary human hepatocyte, as described above,

Preferably said hepacivirus is a hepatitis C virus. The invention thus further provides a method of *ex vivo* screening or identification of molecules capable of interfering with HCV entry in cells comprising comparison of the level of cell infection by the particles of the invention in the presence or the absence of a candidate molecule. Said method preferably comprises the steps consisting of:
- contacting a cell susceptible to HCV infection with an infectious HCV-like particle, in the absence or presence of a candidate molecule, under conditions that allow cell infection with HCV-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with HCV entry the candidate molecule for which cell infectivity, as measured in the presence of said molecule, is decreased as compared to cell infectivity measured in the absence of any candidate molecule.

Contacting a cell susceptible to HCV infection with an infectious HCV-like particle, and a candidate molecule can be carried out by contacting simultaneously said cell, HCV-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious HCV-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with HCV cell entry may be selected from the group consisting of a hepatocyte cell line and a primary human hepatocyte, as described above.

Such molecules capable of interfering with hepacivirus, and in particular with HCV, entry in cells may constitute new antiviral drugs.

The infectious particles of the invention are further useful for diagnosis of hepacivirus infection and follow-up of hepacivirus infection, for instance to assess efficacy of a therapy in a patient.

The invention thus concerns the use of an infectious hepacivirus-like particle for the *in vitro* detection of antibodies directed against hepacivirus in a biological sample from a subject susceptible to be infected with hepacivirus. Said biological sample may be a biological fluid, such as blood or serum, or a tissue biopsy. In a specific embodiment, said antibodies are directed against E1 and/or E2 hepacivirus glycoproteins.

Accordingly, the invention provides a method of *in vitro* diagnosis of a hepacivirus infection in a patient comprising detecting immune complexes formed by interaction of anti-hepacivirus antibodies likely to be present in a biological sample of the patient, with hepacivirus-like particle of the invention. Said method may in particular comprise the steps consisting of:
- contacting a biological sample with an infectious hepacivirus-like particle of the invention under conditions sufficient to allow formation of complexes by binding of said infectious particle to antibodies directed against hepacivirus present in the biological sample;
- detecting said complexes, which presence is indicative of a hepacivirus infection.

The presence of antibodies reactive with hepacivirus-like particles can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the hepacivirus-like particle and the antibody or antibodies reacted therewith.

in another embodiment, said method of *in vitro* diagnosis of a hepacivirus infection in a patient comprises detecting an inhibitory effect of anti-hepacivirus antibodies likely to be present in a biological sample of the patient, on the infection of a permissive cell by a hepacivirus-like particle of the invention. Said method may in particular comprise the steps consisting of:
- contacting a cell permissive for hepacivirus infection with a hepacivirus-like particle and a biological sample;
- comparing cell infectivity measured in presence of said biological sample with cell infectivity measured in absence of said biological sample;
- detecting the inhibition of hepacivirus-like particle infection of a permissive cell as a decrease in cell infectivity measured in presence of said biological sample compared with cell infectivity measured in absence of said biological sample, said inhibition being indicative of a hepacivirus infection.

This embodiment is advantageous in that the method relies on the detection of the specific antibodies that are neutralizing for cell infection, that is those patient's antibodies that are effective against viraemia.

In a further embodiment of this invention, commercial diagnostic kits may be useful to carry out the above diagnosis methods, by detecting the presence or absence of immune complexes formed by hepacivirus particles and antibodies directed against hepacivirus in a biological sample from a subject susceptible to be infected with hepacivirus, or by detecting an inhibition of hepacivirus-like particle infection of a permissive cell by anti-hepacivirus neutralizing antibodies likely to be present in a biological sample of the patient. Such kits may comprise at least a hepacivirus-like particle of the present invention. Where the method involves detection of immune complexes, the kits may further comprise appropriate means of detection of said immune complexes. Preferably the kit of the invention further comprises directions, and protocols, depending upon the method selected, *e.g.*, "competitive", "sandwich", and the like. The kits may also contain peripheral reagents such as buffers, stabilizers, etc..

Preferably said hepacivirus is a hepatitis C virus. The infectious particles of the invention are further useful for diagnosis of HCV infection and follow-up of HCV infection, for instance to assess efficacy of a therapy in a patient.

The invention thus concerns the use of an infectious HCV-like particle for the *in vitro* detection of antibodies directed against HCV in a biological sample from a subject susceptible to be infected with HCV. Said biological sample may be a biological fluid, such as blood or serum, or a tissue biopsy. In a specific embodiment, said antibodies are directed against E1 and/or E2 HCV glycoproteins.

Accordingly, the invention provides a method of *in vitro* diagnosis of a HCV infection in a patient comprising detecting immune complexes formed by interaction of anti-HCV antibodies likely to be present in a biological sample of the patient, with HCV-like particle of the invention. Said method may in particular comprise the steps consisting of:
- contacting a biological sample with an infectious HCV-like particle of the invention under conditions sufficient to allow formation of complexes by binding of said infectious particle to antibodies directed against HCV present in the biological sample;
- detecting said complexes, which presence is indicative of a HCV infection.

The presence of antibodies reactive with HCV-like particles can be detected using standard electrophoretic and immunodiagnostic techniques, including immunoassays such as competition, direct reaction, or sandwich type assays, as described above. The reactions generally include revealing labels such as fluorescent, chemiluminescent, radioactive, enzymatic labels or dye molecules, or other methods for detecting the formation of a complex between the HCV-like particle and the antibody or antibodies reacted therewith.

in another embodiment, said method of *in vitro* diagnosis of a HCV infection in a patient comprises detecting an inhibitory effect of anti-HCV antibodies likely to be present in a biological sample of the patient, on the infection of a permissive cell by a HCV-like particle of the invention. Said method may in particular comprise the steps consisting of:
- contacting a cell permissive for HCV infection with a HCV-like particle and a biological sample;
- comparing cell infectivity measured in presence of said biological sample with cell infectivity measured in absence of said biological sample;
- detecting the inhibition of HCV-like particle infection of a permissive cell as a decrease in cell infectivity measured in presence of said biological sample compared with cell infectivity measured in absence of said biological sample, said inhibition being indicative of a HCV infection.

This embodiment is advantageous in that the method relies on the detection of the specific antibodies that are neutralizing for cell infection, that is those patient's antibodies that are effective against viraemia.

In a further embodiment of this invention, commercial diagnostic kits may be useful to carry out the above diagnosis methods, by detecting the presence or absence of immune complexes formed by HCV particles and antibodies directed against HCV in a biological sample from a subject susceptible to be infected with HCV, or by detecting an inhibition of HCV-like particle infection of a permissive cell by anti-HCV neutralizing antibodies likely to be present in a biological sample of the patient. Such kits may comprise at least a HCV-like particle of the present invention. Where the method involves detection of immune complexes, the kits may further comprise appropriate means of detection of said immune complexes. Preferably the kit of the invention further comprises directions, and protocols, depending upon the method selected, *e.g*., "competitive", "sandwich", and the like. The kits may also contain peripheral reagents such as buffers, stabilizers, etc..

In another aspect of the invention, the infectious hepacivirus-like particles may be used for vaccination purposes.

According to an embodiment, the invention thus proposes a method of vaccination, notably against hepacivirus infection, that comprises administration of a hepacivirus-like particle to a subject in need thereof. The invention also relates to a vaccine composition comprising a hepacivirus-like particle and a pharmaceutically acceptable carrier. The invention further provides a immunogenic composition comprising in a pharmaceutical acceptable carrier, a hepacivirus-like particle disclosed herein.

The vaccine and immunogenic compositions of the invention may be drawn to confer immunity, or elicit an immune response against hepacivirus.

However, where the hepacivirus-like particles of the invention further carry an additional gene encoding another antigen, different from hepacivirus antigens, the invention provides a recombinant viral vaccine useful to raise an immune response against said antigen. Actually, the use of pseudo-particles described herein makes it possible to improve the elicited immune response through combining several presentation and processing pathways of an antigen. For instance, a vaccine composition of the invention, when administered, results in the hepacivirus-like particles infecting cells of the host. The transgene encoding the antigen is then integrated in the cellular genome, and subsequently expressed by the cell, such that there is both a cellular and a humoral immune response elicited by the vaccine composition.

Advantageously, the hepacivirus-like particles may further carry a transgene encoding an immune modulator, which allows for enhancement of the raised immune reaction.

Preferably said hepacivirus is a hepatitis C virus. The invention thus proposes a method of vaccination, notably against HCV infection, that comprises administration of a HCV-like particle to a subject in need thereof. The invention also relates to a vaccine composition comprising a HCV-like particle and a pharmaceutically acceptable carrier. The invention further provides a immunogenic composition comprising in a pharmaceutical acceptable carrier, a HCV-like particle disclosed herein.

The vaccine and immunogenic compositions of the invention may be drawn to confer immunity, or elicit an immune response against HCV.

However, where the HCV-like particles of the invention further carry an additional gene encoding another antigen, different from HCV antigens, the invention provides a recombinant viral vaccine useful to raise an immune response against said antigen. Actually, the use of pseudo-particles described herein makes it possible to improve the elicited immune response through combining several presentation and processing pathways of an antigen. For instance, a vaccine composition of the invention, when administered, results in the HCV-like particles infecting cells of the host. The transgene encoding the antigen is then integrated in the cellular genome, and subsequently expressed by the cell, such that there is both a cellular and a humoral immune response elicited by the vaccine composition.

Advantageously, the HCV-like particles may further carry a transgene encoding an immune modulator, which allows for enhancement of the raised immune reaction.

The vaccination or immunogenic composition of the present invention may additionally contain an adjuvant. A number of adjuvants are known to those skilled in the art. Examples of suitable adjuvants include, for example, include aluminum hydroxide; Saponin; detergents such as Tween 80; animal, mineral or vegetable oils, Corynebacterium or Propionibacterium -derived adjuvants; Mycobacterium bovis (Bacillus Calmette and Guerinn, or BCG); cytokines; acrylic acid polymers such as carbomer; EMA; or combinations thereof.

The route of administration is any conventional route used in the vaccine field. As general guidance, a vaccine composition of the invention is administered via a mucosal surface, *e.g*., an ocular, intranasal, pulmonary, oral, intestinal, rectal, vaginal, and urinary tract surface; or via a parenteral route, *e.g*., by an intravenous, subcutaneous, intraperitoneal, intradermal, intraepidermal, or intramuscular route. The choice of administration route depends on the formulation that is selected.

In still another embodiment the particles of the invention may be used as vectors for gene transfer and/or gene therapy. Gene therapy is defined as the introduction of genetic material into a cell in order to either change its phenotype or genotype. Owing to their tropism for hepatic cells, either primary or cancerous, the hepacivirus-like particles, and in particular the HCV-like particles, described herein comprise an efficient gene delivery system specific for hepatocytes. Furthermore, such a delivery system is amenable to scale up for reproducibly producing large titers of infectious, replication-defective hepacivirus-like particles, in particular HCV-like particles.

Accordingly, the invention relates to a method for *in vivo* or *in vitro* transferring a transgene of interest in a cell, which method comprises infecting a cell with a hepacivirus-like particle of the invention, wherein the particle carries a transgene of interest.

The invention further relates to the use of a hepacivirus-like particle of the invention, that carries a transgene of interest, for the preparation of a medicament for the prevention or treatment of a disease in a patient, wherein the hepacivirus-like particle allows the transfer of the transgene of interest into a cell of the patient, and encodes a product that has a prophylactic or therapeutic effect against the disease.

Preferably said hepacivirus is a hepatitis C virus. The invention thus proposes a method for *in vivo* or *in vitro* transferring a transgene of interest in a cell, which method comprises infecting a cell with a HCV-like particle of the invention, wherein the particle carries a transgene of interest.

The invention further relates to the use of a HCV-like particle of the invention, that carries a transgene of interest, for the preparation of a medicament for the prevention or treatment of a disease in a patient, wherein the HCV-like particle allows the transfer of the transgene of interest into a cell of the patient, and encodes a product that has a prophylactic or therapeutic effect against the disease.

Preferably, the targeted cell is a hepatic cell.

The invention will be further understood in view of the following examples and the annexed figures.

### LEGEND TO THE FIGURES:

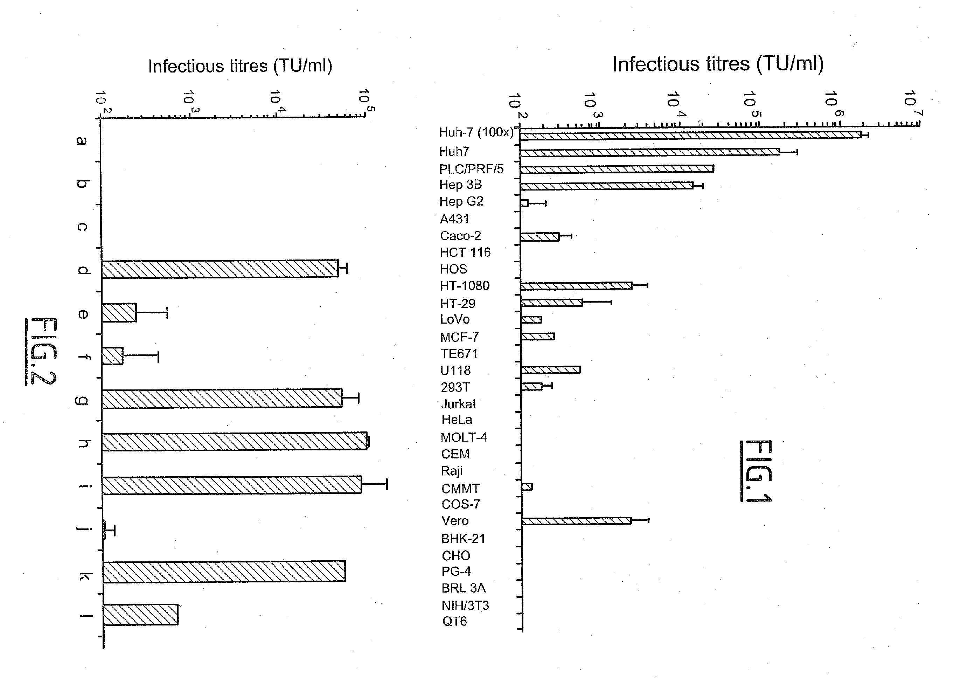

Figure 1 depicts the results of infectivity experiments performed with HCV pseudo-particles of 1 a genotype and different target cell types. Results are displayed as transducing units (TU) per ml of supernatant (mean ± standard deviations of up to six experiments) for HCVpp. The infectivity on Huh-7 cells of HCVpp concentrated 100 times by ultra-centrifugation is shown (100x).

Figure 2 represents the infectious titres determined on Huh-7 target cells with HCV pseudo-particles generated without E1E2 (lane a), without retroviral core proteins (lane b), with MLV-G2A assembly-defective core proteins (lane c), with HIV-1 core proteins (lane d), with E1 or E2 alone (lane e or f, respectively), with E1+E2 expressed in *trans* from two independent vectors (lane g), with HCV-1a E1E2 expressed from the same vector in *cis* (lane h), or with HCV-1b E1E2 (lane i). HCVpp were treated with 25 µM AZT (3'-azido-3'-deoxythymidine; Sigma-Aldrich, France) before and during infection of target cells (lane j). Infectivity of HCV pseudo-particles generated with E1 from 1a HCV genotype and E2 form 1b HCV or genotype (lane k) or with E1 from 1b HCV genotype and E2 form 1a HCV or genotype (lane I). Results are expressed as TU/ml and are displayed as mean ± standard deviations of up to four experiments.

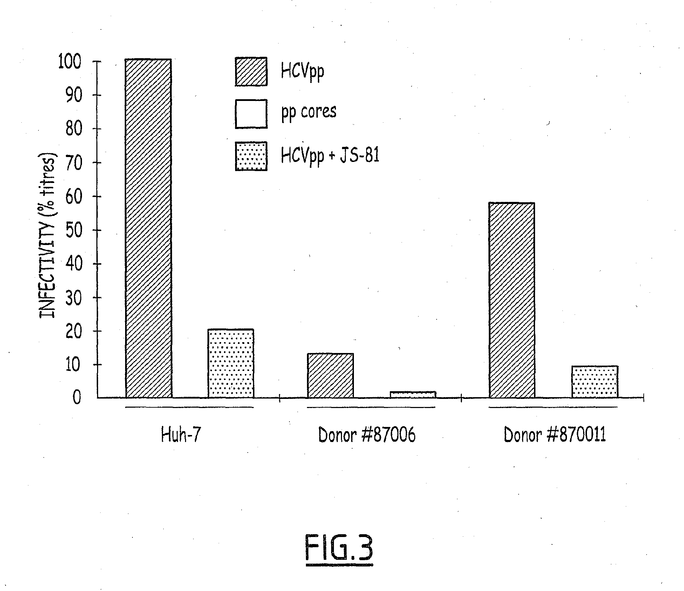

Figure 3 shows the results of infection on human primary hepatocytes derived from two donors with of HCVpp of genotype 1a. Infectivity is expressed as percentage of infectivity determined on Huh-7 cells.

Fiaure 4 displays neutralisation of HCV pseudo-particles with monoclonal antibodies against E1 (A4) or E2 (H31, H33, H35, H44, H48, H53, H54, H60 and H61) glycoproteins of genotype 1a, with pooled antibodies (Hmix), with no antibodies or using pseudo-particles generated with VSV-G (control). Neutralization of the control was achieved with the VSV-G neutralising 41A.1 monoclonal antibody. Results are expressed as the percentages of inhibition of the average infectious titres ± standard deviations relative to incubation in the absence of antibodies.

Figure 5 represents neutralisation of HCVpp with HCV patient sera. The genotype of HCV diagnosed in these patients is indicated in brackets. Results are expressed as percentages of inhibition of the average infectious titres ± standard deviations relative to incubation with control sera from healthy individuals.

Figures 6A and 6B show HCV E1E2 and retroviral expression constructs. (A) : A cDNA derived from the HCV polyprotein gene was used to express the E1E2 glycoproteins and the carboxy-terminus of the C protein, which provides the signal peptide for E1 (SP E1). The position of stop codons (star) inserted in the expression constructs to terminate translation of the proteins is shown. The transmembrane domain (TMD) of E1 provides the signal peptide (SP E2) for the E2 glycoprotein. (B) : The expression constructs encoding the different components required to assemble infectious pseudo-particles are shown. The filled boxes represent the viral genes and the marker gene (GFP) transferred to the infected cells. The open boxes show the cis-acting sequences. LTR, long terminal repeat ; CMV, human cytomegalovirus immediate-early promoter; PBS, primer binding site ; ψ, packaging sequence ; PPT, poly-purine track ; polyA, polyadenylation site ; SD, splice donor site ; SA, splice acceptor site. Vector particles were produced by cotransfection of plasmids harboring the packaging functions, the transfer vector and the viral glycoproteins into 293 T cells. The viral glycoproteins were the HCV E1 and E2 glycoproteins, expressed individually or as a CE1 E2, the VSV-G or the RD114 glycoproteins. The supernatants of transfected cells were collected during transient expression, concentrated by ultracentrifugation, and used for target cell transduction.

Figure 7 shows incorporation of ΔHVR1 HCV pseudo-particles. Immunoblots of lysates of 293 T transfected cells and of pseudo-particles pelleted through 20 % sucrose-cushions are shown. The positions of the molecular weight markers are shown (kDa).

Figure 8 represents the infectivity of ΔHVR1 HCV pseudo-particles. Supernatants from producer cells were diluted and used to infect Huh-7 target cells before assessing transduction efficiency three days post-infection by FACS analysis.

### EXAMPLES:

### EXAMPLE 1 : Generation of HCV pseudo-particles (HCVpp)

HCV pseudo-particles (HCVpp) were generated by assembling full-length, unmodified E1 and E2 glycoproteins onto retroviral core proteins derived from murine leukemia virus (MLV). To investigate further whether functional HCVpp could also be produced with E1 and E2 expressed in *trans* or with only one of the two glycoproteins, expression vectors that encoded individually either E1 or E2 glycoproteins were designed.

### Construction of expression vectors encoding the viral components, i.e., E1, E2, or E1E2 glycoproteins and viral core proteins

Plasmids expressing wild type E1E2 polyproteins were constructed by standard methods (Sambrook et *al*., 1989).

Briefly, the phCMV-7a expression vector encoding the E1 and E2 glycoproteins from a 1a type HCV was generated by inserting the blunted *Cl*al and *Stu*l restriction fragment encoding the last 60 residues of HCV core (C) and all of E1 and E2 proteins from the pTM1p5E1E2(745) vector (Op De Beeck *et al.,* 2000) into the *Bam*Hl digested and Klenow blunted vector phCMV-G (Nègre *et al.,* 2000).

The phCMV-E1 expression vector, expressing only HCV E1 glycoprotein, was derived from phCMV-7a by adding a stop codon to the C-terminus of E1 with primers 5'-actggacgacgcaaagctgc (SEQ ID N°2) and 5'-cgcggatcctacgcgtcgacgccggcaaa (SEQ ID N°3). The resulting PCR fragment was digested with *Bam*Hl and ligated into *Bam*Hl-digested phCMV-7a.

phCMV-E2, expressing only HCV E2 glycoprotein, was obtained by fusing the N-terminus of E2 to the C-terminus of the HCV core using two PCR fragments generated with the two primer pairs 5'-tgcccgcttcagccgaaacccacgtcaccggggga (SEQ ID N°4) + 5'-gccagaagtcagatgctcaagg (SEQ ID N°5) and 5'-tactctgagtccaaaccg (SEQ ID N°6) + 5'-gtgacgtgggtttcggctgaagcgggcacagtcag (SEQ ID N°7). The two PCR fragments were then fused in a second round PCR. The resulting DNA fragment was digested with *Bam*Hl and ligated into *Bam*Hl cut phCMV-7a.

The sequence of the phCMV-ΔC E1 vector is shown in SEQ ID No 8, whereas the aminoacid sequence of E1 protein is shown in SEQ ID No 9. The sequence of the phCMV-ΔC E1E2 vector is shown in SEQ ID No 10, whereas the aminoacid sequence if the E1E2 polyprotein is shown in SEQ ID No 11. The sequence of the phCMV-ΔC E2 vector is shown in SEQ ID No 12, whereas the aminoacid sequence of E2 protein is shown in SEQ ID No 13.

Expression vectors for E1E2 glycoproteins of 1b genotype were constructed by similar strategies.

HCV E1E2 were therefore expressed from a polyprotein containing the carboxy-terminus of the core (ΔC) protein, which served as signal peptide for E1, E2 or E1 and E2 glycoproteins.

### Generation of HCV pseudo-particles

Retroviruses were chosen as platforms for assembly of HCVpp because their cores can incorporate a variety of different cellular and viral glycoproteins and because they can easily package and integrate genetic markers into host cell DNA.

HCVpp were produced by transfecting 293T human embryo kidney cells (ATCC CRL-1573) with three expression vectors encoding a ΔCE1E2, ΔCE1 or ΔCE2 polyprotein, the MLV core proteins and a packaging-competent MLV-derived genome harbouring the GFP (green fluorescent protein) marker gene. This construct contains the GFP marker gene, whose expression is driven by the CMV (cytomegalovirus) immediate early promoter. Both CMV and GFP nucleic acid sequences where inserted in a retroviral vector derived from MLV in which the gag, pol and env viral gene where removed and in which the retroviral cis-acting elements that control vector genome packaging, reverse transcription and integration were retained.

Control pseudo-particles were generated with the VSV-G glycoprotein (Nègre *et al.,* 2000), the RD114 virus envelope glycoprotein (Sandrin *et al.,* 2002), and/or with assembly-defective MLV core proteins (MLV-G2A) (Swanstrom et *al.,* 1997).

Briefly, expression constructs were transfected into 2.5×10⁶ 293T cells seeded the day before in 10 cm plates using a calcium-phosphate transfection protocol (Clontech, France) according to the manufacturer's recommendations. The medium (8 ml/plate) was replaced 16 hrs after transfection. Supernatants containing the pseudo-particles were harvested 24 hrs later, filtered through 0.45µm-pore-sized membranes and processed as described before (Nègre *et al.,* 2000).

### Immunoblot analysis of structural components of the pseudo-particles

Lysates of transfected cells and of pseudo-particles pelleted through 20% sucrose-cushions were immunobloted. Expression of E1 and E2 glycoproteins from HCV-1a genotype and of MLV core proteins was revealed in reducing and denaturing conditions with monoclonal antibodies against E1 (A4) (Dubuisson et *al*., 1994) and E2 (H52) (Flint *et al.,* 1999) or with an anti-capsid (MLV CA) antiserum (ViroMed Biosafety Laboratories, USA), as described previously (Sandrin *et al.,* 2002). VSV-G expressed in control pseudo-particles was detected with the monoclonal antibody P5D4 (Sigma-Aldrich, France).

Analysis of immunoblots of transfected cells showed that the structural components of the pseudo-particles were readily detected at the expected molecular weights; *i.e.* 30 kDa for E1, 60 kDa for E2, 60 kDa for VSV-G and 70 kDa for the RD114 glycoprotein. MLV core proteins were detected as a Gag protein precusor of 65 kDa that was partially processed by the MLV protease into mature core components.

The E1 and E2 glycoproteins were readily detected in the pellets of purified virions generated with the wild-type MLV core particles but not with the viral assembly-deficient MLV-G2A mutant. E2 present within the viral pellets migrated slightly slower than the cell-associated forms, due to modifications of the associated glycans by Golgi enzymes.

The presence of VSV-G in viral pellets generated with MLV-G2A assembly-defective core proteins is due to empty vesicles formed by VSV-G itself (Nègre *et al.,* 2000).

Comparison of the relative levels of VSV-G or HCV glycoproteins in producer cell lysates versus viral pellets suggests efficient incorporation of E1 and E2 into viral particles. Likewise, could E1 and E2 glycoproteins efficiently assemble on retroviral core proteins derived from HIV-1, raising the possibility of pathogenic interactions between the two parental viruses, *in vivo,* as co-infection of patients with HCV and HIV is prevalent (Dieterich, 2002).

Altogether, these results indicate that transient over-expression of E1 and E2 in 293T cells leads to specific and efficient incorporation of HCV glycoproteins into pseudo-particles generated with retroviral cores. Since HCV envelope glycoproteins have been shown to be retained in the ER (Op De Beeck et al. 2001), this implies that HCVpp form by budding into the ER lumen or, alternatively, that saturation/leakiness of the ER retention allows a fraction of E1E2 to reach the cell surface where MLV budding normally occurs (Swanstrom and Wills, 1997).

Individual expression or co-expression in *trans* of E1 and E2, from distinct expression units, led to normal levels of synthesis, as compared to expression of both glycoproteins in cis, from a single E1E2 polyprotein. Moreover, HCVpp were found to incorporate similar levels of E2 glycoprotein, whether it was expressed alone, or co-expressed in *cis* or in *trans* with E1. Finally, incorporation of E1 expressed alone or co-expressed in *trans*, with E2, occurred but at reduced levels, consistent with the chaperone activity of E2 (Op De Beeck et *al*., 2001). Formation of HCVpp carrying E1 or E2 only will allow investigation of their respective roles in HCV cell entry and infectivity.

### EXAMPLE 2 : HCVpp cell line infectivity

Infectivity of HCVpp was assessed on a panel of target cell lines : Huh-7 human hepatocellular carcinoma (Nakabayashi et *al.,* 1982), PLC/PRF/5 human hepatoma (CRL-8024), Hep3B human hepatocellular carcinoma (ATCC HB-8064), HepG2 human hepatocellular carcinoma (HB-8065), A431 human epidermoid carcinoma (CRL-1555), Caco-2 human colon adenocarcinoma (HTB-37), HCT 116 human colorectal carcinoma (CCL-247), HOS human osteosarcoma (CRL-1543), HT-1080 human fibrosarcoma (CCL-121), HT-29 human colorectal adenocarcinoma (HTB-38), LoVo human colorectal adenocarcinoma (CCL-229), MCF-7 human breast adenocarcinoma (HTB-22), 293T, TE671 human rhabdomyosarcoma (ATCC CRL-8805), U118 human glioblastoma (HTB-15), Jurkat human T cell leukemia (TIB-152), CEM human lymphoblastic leukemia (CCL-119), Molt-4 human lymphoblastic leukemia (CRL-1582), Raji Burkitt's lymphoma (CCL-86), CMMT Rhesus monkey mammalian carcinoma (CRL-6299), COS-7 African green monkey fibroblasts kidney (CRL-1651), VERO African green monkey kidney (CCL-81), PG-4 feline astrocyte (CRL-2032), BHK-21 golden hamster kidney (CCL-10), CHO Chinese hamster ovary (ATCC CCL-61), BRL 3A rat hepatocytes (CRL-1442), NIH3T3 mouse fibroblasts (CRL-1658) and QT6 quail fibrosarcoma (CRL-1708). Target cells were grown as recommended by the ATCC (American Type Culture Collection, Rockville, MD, USA)..

Target cells (seeded the day before 8×10⁴ cells/well in 12-well plates) were incubated for 3 hrs with dilutions of supernatants from producer cells containing the HCVpp, then washed and cultured until expression of the GFP marker gene harboured by the virions was assessed by FACS analysis 72 hrs later (Sandrin *et al*., 2002). Since the HCVpp were generated with replication-defective viral components, this procedure allowed evaluation of the specific infectivity of the pseudo-particles after a one-round infection process.

Infectious titres of up to 1.1×10⁵ TU/ml were detected for the HCVpp on Huh-7 human hepato-carcinoma cells (Figure 1). Upon concentration of the producer cell supernatants by ultra-centrifugation (Sandrin *et al.*, 2002), infectious titres of 2×10⁶ TU/ml, on average, could be readily obtained. The other target cell types used in the infection assays displayed weaker (PLC/PRF/5, Hep3B, HepG2, Caco-2, HT1080, HT-29, LoVo, MCF-7, U118, 293T, Vero) or undetectable (A431, HCT 116, HOS, TE671, Jurkat, Molt-4, CEM, Raji, CMMT, Cos-7, BHK-21, CHO, PG-4, BRL 3A, NIH3T3, QT6) levels of infection with the HCVpp. The infectivity of control pseudo-particles generated with VSV-G ranged from 7×10⁶ to 2×10⁷ TU/ml depending on the target cell type indicating that all these cells were readily infected with the control pseudo-particles. This suggested that all the molecules necessary for HCV entry were specifically expressed in the former cell types only.

Infectious HCVpp could be generated at comparable efficiencies with E1E2 glycoproteins derived from HCV genotypes 1a and 1b (lanes h and i; Figure 2) and/or with retroviral core proteins derived from either HIV-1 or MLV (lanes d and h; Figure 2).

Incubation of HCVpp and target cells with AZT, a reverse-transcriptase inhibitor that prevents conversion of the retroviral RNA genome of the HCVpp as integration-competent proviral DNA, inhibited transduction (lane j; Figure 2).

Moreover, long-term expression of GFP could be demonstrated after serial passaging of the infected cells for more than one month.

These results indicated that infection of the target cells led to integration in host cell DNA and stable expression of the GFP marker gene transduced by the HCVpp. Additionally, no infection could be obtained with viral particles lacking both E1 and E2 glycoproteins, or lacking core proteins, or, alternatively, when using the MLV-G2A assembly-defective core proteins (lanes a-c; Figure 2).

Altogether, these results demonstrated that HCVpp harbouring the E1 and E2 glycoproteins and retroviral core proteins were infectious, leading to retroviral-mediated integration of their vector genome.

Finally, despite reduced levels of E1 incorporation, HCVpp generated with E1 and E2 glycoproteins expressed in *trans,* from distinct vectors, were nearly as infectious as those generated with the E1 E2 polyprotein construct (lanes g and h; Figure 2). However, HCVpp assembled with either E1 or E2 glycoproteins only were 500-fold less infectious (lanes e-f; Figure 2), demonstrating that both glycoproteins need to be co-incorporated on the HCVpp to allow efficient virus entry and infection.

### EXAMPLE 3 : HCVpp primary hepatocyte infectivity

Hepatocytes represent the principal site of HCV replication *in vivo,* yet *ex vivo* studies have suggested that HCV may also infect lymphoid cells (Lindenbach and Rice, 2001). To address whether either cell types could be infected, human adult hepatocytes (Figure 3) and peripheral blood mononuclear cells (PBMCs) have been transduced. Pseudo-particles were generated with core proteins derived from HIV-1 rather than from MLV, which do not permit transduction of non-proliferating target cells (Nègre *et al.,* 2000).

Cryopreserved primary hepatocytes, isolated from human adult biopsy samples checked for absence of HBV, HCV and HIV, were purchased from Biopredic International (Rennes, France) and cultured on collagen I coated plates according to recommendations of the supplier. Transduction of hepatocytes was determined by counting GFP-positive cells under UV microscope. Specificity of infection was demonstrated by absence of transduction when target cells were infected with pseudo-particles lacking glycoproteins (pp cores) or by the reduced levels of transduction when target cells were pre-incubated with JS-81 anti-CD81 antibodies (30 µg/ml) before infection. Infection of human PBMCs, isolated from healthy donors, was conducted as described previously (Sandrin *et al*., 2002).

Relative to infection of Huh-7 cells, the HCVpp could readily infect the primary hepatocytes derived from different donors (Figure 3), yet transduction efficiencies varied with quality and cell culture viability of the individual biopsies. In contrast, poor or undetectable infection of PBMCs was found with the HCVpp although control pseudo-particles generated with VSV-G could readily infect these primary cells.

Altogether these data indicate that HCVpp closely mimic the tropism and early events of infection by wild-type HCV and preferentially infect hepatocytes and hepato-carcinoma cells.

### EXAMPLE 4 : HCVpp is a valid model of early steps of HCV infection

### Infectivity of HCVpp is mediated by E1 and E2 glycoproteins

It was further determined whether infectivity of the HCVpp is specifically mediated by E1 and E2 and their interaction with cell surface receptors. A panel of monoclonal antibodies previously shown to specifically react against HCV-1a glycoproteins (Dubuisson et *al.,* 1994; Flint et *al.*, 1999; Patel et *al*., 2000) was used.

HCVpp generated with HCV-1a E1E2 and with MLV core proteins were pre-incubated before infection of Huh-7 cells with saturating concentrations (20 µg/ml) of monoclonal antibodies against E1 (A4) or E2 (H31, H33, H35, H44, H48, H53, H54, H60 and H61) glycoproteins of genotype 1 a, or with pooled antibodies (Hmix). Control experiments were performed using no antibodies or using pseudo-particles generated with VSV-G (VSV-Gpp).

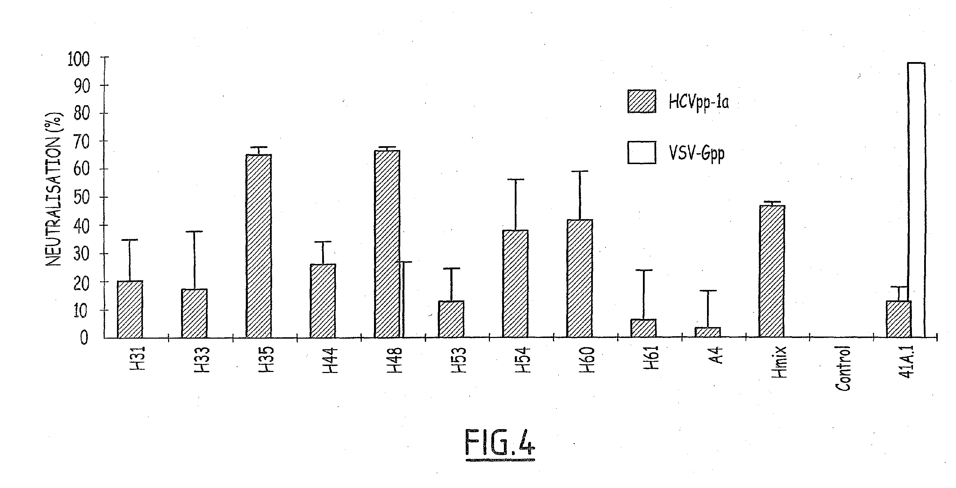

Some of these monoclonal antibodies, like H35 and H48, for example, could neutralise the HCVpp of genotype 1a and reduce their infectivity by up to 70% (Figure 4). That incomplete neutralisation might be due to the fact that these monoclonal antibodies, which were developed and selected for binding to intra-cellular E1E2 complexes, have been shown to be sensitive to post-translational modifications of E2 (Flint M. et *al.,* 2000). Indeed, compared to its intra-cellular counterpart, E2 associated to HCVpp was found to have undergone sugar modifications, most likely as a result of its export through the cell secretory pathway.

In contrast, none of these genotype 1a-specific antibodies could neutralise the HCVpp of genotype 1b or the control pseudo-particles generated with VSV-G (Figure 4). Neutralisation of the infectivity of these control pseudo-particles was achieved by using the VSV-G neutralising 41A.1 monoclonal antibody.

These data therefore demonstrate that infectivity of the pseudo-particles is specifically due to the incorporation of E1 and E2 glycoproteins, indicating that these HCVpp represent a valid model to investigate the early steps of HCV infection, *i.e*. receptor binding, membrane fusion and envelope uncoating.

### HCV infection is neutralised by serum from HCV-infected patients

The capacity of sera derived from chronically HCV-infected patients to neutralise infectivity of HCVpp was further assessed.

HCVpp of genotypes 1 a or 1b were pre-incubated for 30 min at room temperature with sera from chronically HCV-infected patients diluted 1/50 before infection of Huh-7 target cells. Control experiments were performed using pseudo-particles generated with RD114 glycoproteins (RD114pp), rather than with VSV-G that exhibits sensitivity to human complement (Sandrin et *al*., 2002). Efficient neutralisation of the control pseudo-particles was demonstrated using a hyperimmune goat serum raised against the RD114 SU glycoprotein (ViroMed Biosafety Laboratories, USA).

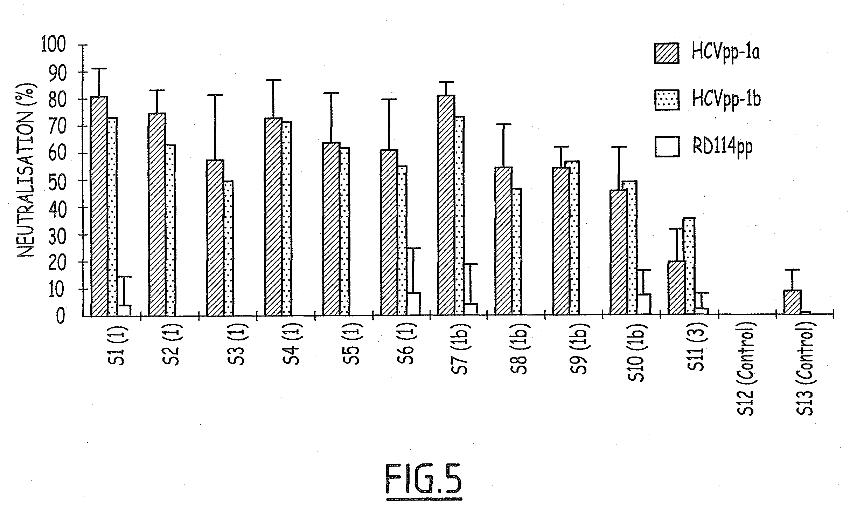

No or only weak neutralisation of control pseudo-particles could be detected using the sera of the HCV-infected patients. In contrast, most if not all of these sera could neutralise the infectivity of the HCVpp, in contrast to sera derived from healthy donors. Variable levels of neutralisation were detected depending on the donor and ranged from 20% to up to 90% of inhibition for HCVpp of both genotypes 1a and 1b. Sera derived from patients infected with HCV of the 1b genotype could neutralise the HCVpp generated with E1E2 of genotype 1a or 1b with similar efficiencies (Figure 5).

### EXAMPLE 5 : Cell entry receptor usage by HCV pseudo-particles

Both the LDL receptor (LDLr) and CD81, a member of the tetraspanin family of receptors, have been proposed as putative HCV receptors (Pileri et *al*., 1998; Agnello et *al*., 1999). However, recent studies have questioned the role of these molecules as cell entry receptors despite their unequivocal capacity to bind HCV particles and/or glycoproteins (2023). Thus, the contribution of either cell surface molecules in the early stages of HCV cell entry was investigated by performing receptor-competition assays.

More recently, SR-B1 the human scavenger receptor class B type I expressed in steroidogenic tissues has been put forward as another putative HCV receptor (Scarselli, Ansuini et al. 2002). Thus, the contribution of SR-BI in the early stages of HCV cell entry was investigated by performing infection assays in non-permissive target cells engineered to express this HCV receptor candidate.

### LDLr

LDLr binds apoliprotein B within LDL and apoliproteins B and E (ApoB and ApoE) within VLDL complexes; both complexes have been found associated with HCV particles in plasma of infected patients (Andre et *al*., 2002).

LDL receptor competition assays were performed using purified LDLs (10 µg/ml, 100µg/ml; Sigma-Aldrich, France) or with the monoclonal antibodies 5E11 (10 µg/ml, 50 µg/ml), 4G3 (anti-ApoB; 10 µg/ml, 50 µg/ml) or 1B7 (anti-ApoE; 1 µg/ml ,10 µg/ml, 50 µg/ml, 100 µg/ml) (Ottawa Heart Institute Research corporation, Ottawa, Ontario, Canada), or mixed 5E11, 4G3 and 1B7 antibodies (with individual concentrations 10 µg/ml, 50 µg/ml), pre-incubated with the pseudo-particles prior to infection.

Purified LDLs were not found to out-compete infection of HCVpp on Huh-7 LDLr-positive cells, even when concentrations higher than 100 µg/ml were used. When monoclonal antibodies targeted to the receptor binding sites of ApoB and ApoE were pre-incubated with the HCVpp, only the anti-ApoE antibodies could inhibit, albeit weakly, the infectivity of the HCVpp. This is consistent with our observation that HCVpp could not or hardly infect LDLr-positive cell lines such as HepG2, Jurkat, CEM, Molt-4, Raji and TE671 (Figure 1). Thus, although these results do not exclude a role of LDLr in HCV entry, possibly *via* association of LDLs with HCV particles (Andre et *al.*, 2002), they suggest that LDLr is not the major receptor of HCVpp entry.

### CD81

Recombinant GST-fusion polypeptides encompassing the large extra-cellular loop (LEL) of human CD81 which has been shown to bind HCV E2 (Flint *et al.,* 1999, (Pileri et *al*., 1998) were then used to investigate the role of hCD81 in HCVpp cell entry.

hCD81-LEL GST-fusion polypeptides (4 µg/ml, 8 µg/ml, 16 µg/ml) were pre-incubated with the pseudo-particles before infection or JS-8-1 anti-CD81 antibodies (4 µg/ml, 10 µg/ml, 30 µg/ml; Pharmingen, France) were pre-incubated with the target cells prior to infection. Percentages of inhibition of the infectious titres obtained on Huh-7 cells relative to titres obtained in the absence of inhibitors were calculated. Control experiments were performed using pseudo-particles generated with VSV-G (VSV-Gpp). Comparative experiments were further conducted with NIH3T3, NIH3T3-hCD81 and Huh-7 cells infected with HCVpp and with control pseudo-particles generated with VSV-G.

The GST-fusion hCD81-LEL polypeptides pre-incubated with the HCVpp could specifically precipitate E1 E2 complexes, confirming their capacity to bind the E2 glycoprotein (Flint *et al.,* 1999; Pileri et *al*., 1998), and were found to neutralise the infectivity of the HCVpp on Huh-7 cells, yet with a relatively poor efficiency inhibition ranged from 38% to 54% inhibition of infectivity and appeared to be dose-dependent.

Consistently, pre-incubation of Huh-7 cells with JS-81 monoclonal antibody, that blocks binding of recombinant E2 to hCD81 (Flint *et al.,* 1999), was found to inhibit infectivity of HCVpp in both Huh-7 target cells and human primary hepatocytes. At high antibody concentrations (30 µg/ml), over 90% inhibition of infection could be obtained for HCVpp based on genotypes 1a as well as 1b. However, several target cells non-permissive to infection by HCVpp, like TE671, Jurkat, CEM, Molt-4 and Raji cells (Figure 1) were found to express high densities of hCD81, indicating the lack of correlation between CD81 expression and infectivity.

Moreover, expression of hCD81 in non-permissive NIH3T3 mouse fibroblasts did not allow infection with the HCVpp (Figure 4C).

Altogether these results demonstrate that although hCD81 binds HCV E2 and might help cell surface attachment of HCV, it is not sufficient by itself to allow infection with HCVpp.

### SR-BI

A cDNA encoding SR-BI, also known as CLA-1 (CD36 and LIMPII Analogous-1) (Calvo and Vega, 1993; Webb et al., 1998) was introduced in an MLV retroviral vector and vector particles were generated and pseudotyped with the VSV-G glycoprotein. They were used to transduce CHO and 3T3 cells, that are non permissive to HCVpp infection, as shown in Figure 1. Expression of SR-BI in these transduced cells was verified by FACS analysis using antibodies, directed against the SR-BI ectodomain. The SR-BI-transduced cells as well as the parental cells were used as target cells for infection assays using the HCVpp. Huh7 cells were used as control permissive target cells.

In contrast to the Huh-7 cells, neither the SR-BI-transduced cells nor the parental CHO or 3T3 cells could be infected with HCVpp. These data indicates that although SR-BI binds E2, it is not sufficient to allow entry of HCVpp in cells.

Therefore, as for hLDLR and hCD81, expression of SR-BI expression alone is not enough to render cell permissive to infection.

To address the possibility that HCV target cells need to co-express all molecules to allow HCVpp infection, CHO and 3T3 target cells, non permissive to infection with the HCVpp, were co-infected with two VSV-G-pseudotyped vectors carrying CD81 and SR-BI, repectively. Co-expression of both cell suface molecules was verified two days after transduction, by FACS analysis.

The CD81 and SR-BI co-expressing CHO or 3T3 cells were then used as target cells for infection assays using the HCVpp. In contrast to parental CHO or 3T3 cells and to the same cells individually transduced by either CD81 or SR-BI, this resulted in high levels of infectivity, similar to those obtained in the most permissive target cell type, Huh-7 cells.

These results demonstrate that co-expression of both CD81 and SR-BI is necessary and sufficient to allow infection in HCV non-permissive rodent cells.

Since HepG2 human hepato-carcinoma cells are non-permissive to infection by the HCVpp, the inventors further thought that their non-permissiveness could be due to lack of co-expression of both CD81 and SR-BI. Indeed, HepG2 cells express SR-BI but lack CD81 expression.

Thus the HepG2 cells were transduced with a CD81-carrying MLV retroviral vector pseudotyped with VSV-G. CD81-expression was verified by FACS analysis two days after transduction and the CD81-transduced HepG2 cells were then used as target for infection assays using the HCVpp.

Compared to the parental HepG2 cells which were poorly infected with the HCVpp, ectopic expression of CD81 in these cells resulted in high infectivity of the HCVpp, similar to that observed in Huh-7 cells.

### EXAMPLE 6 : Deletion of hypervariable region I

HCV infection is not cleared by over 80% of patients and chronicity is associated with various forms of liver disease. Chronic infection is thought to be caused by the high mutation rate of HCV, which helps the virus to escape the host immune response. One of several mutation hotspots within the HCV genome has been localised to the N-terminus of the E2 glycoprotein. This so-called hypervariable region I is a major antigen and is thought to play an important role in allowing HCV to escape the host's immune response. The antigenic variation of the HVR1 also inhibits the development of a protective immune response against re-infection with heterologous HCV strains/genotypes. Finally does the antigenic dominance and extreme variability of the HVR1 hinder the development of vaccines against HCV. With the long-term aim to develop effective therapeutics which target more conserved parts of the HCV glycoproteins the inventors investigated whether the HVR1 is dispensable for infection.

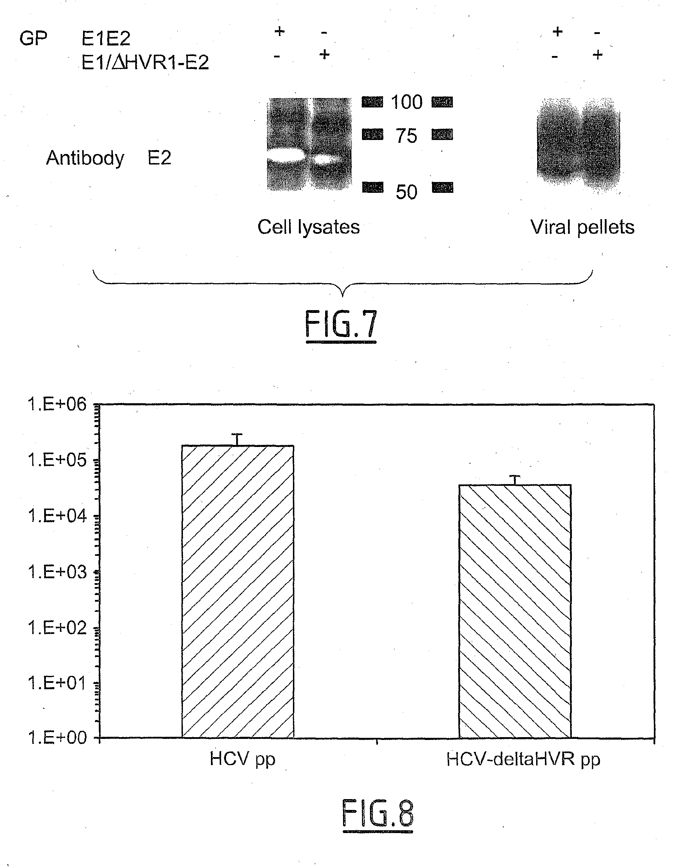

For that purpose expression constructs encoding the polyprotein for wildtype E1E2 or a mutant version in which the HVR1 = the first 27 residues of E2 had been deleted from the polyprotein were generated. HCV pseudo-particles (HCVpp) were produced with either wildtype or ΔHVR1 ΔCE1E2 polyprotein. Analysis of immunoblots of transfected cells showed that both wildtype and mutant E2 proteins were expressed to similar levels. Viral particles were harvested from the supernatant of transfected cells and purified by ultra-centrifugation through high-density sucrose cushions. ΔHVR1 and wildtype E2 were incoporated into particles to a similar level (Fig. 7). The infectivity of the wildtype and mutant HCVpp were tested as before on Huh-7 target cells. Infectious titres of up to 2.6×10⁵ TU/ml were detected for wildtype HCVpp (Fig. 8). ΔHVR1 HCVpp were about 5 to 10 fold less infectious, still a maximal titer of 5.1×10⁵ TU/ml was observed suggesting that the HVR1 aids but is not required for the infection process.

### Example 7 : HCVpp and cell-cell fusion assays

Cell-cell fusion assays were designed by co-culturing E1 E2-transfected 293T (donor) cells with target (effector) cells. The effector cells were hepato-carcinoma cells or, as control, 293T cells.

Transformed cells, transfected with the constructs expressing the envelope glycoproteins, are detached, counted and re-seeded at the same concentration (3×10⁵ cells/well) in six-well plates. Fresh target host cells (1×10⁶ cells per well) are then added onto the transfected cells and are cocultivated for 24 hours.

The coculture is submitted to a acidic pH drop by replacing the culture medium with a pH5-buffered DMEM for 5 min and incubating for 5 min Acidic medium was then replaced with a normal (neutral pH medium) and cultures were grown for 12 hours until cell-cell fusion was evaluated by scoring the syncytia

Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

The results of syncytia scoring are displayed in Table 1.

**Table 1.**

| Results of syncytia assays with Huh-7 cells | | |
|---|---|---|
| Transfected glycoprotein | Syncytia at neutral pH¹ | Syncytia after pH-5 shock¹ |
| None | - | - |
| MLV-A | - | - |
| MLV-A-Rless | ++ | ++ |
| FPV-HA | - | ++ |
| VSV-G | + | +++ |
| HCV E1 | - | - |
| HCV E2 | - | - |
| HCV E1E2 | - | ++² |

| | | |
|---|---|---|
| ¹(-), fusion index of less than 1%, (+),fusion index between 1% and 5%; (++),fusion index between 5% and 20%; (+++),fusion index higher than 20%. | | |
| ²no syncytia were detected when HCV E1E2-transfected cells were co-cultivated with 293 cells after a pH-5 shock | | |

Expression of control amphotropic murine leukaemia virus (MLV-A) pH-independent glycoproteins did not result in syncytia in these experimental conditions. However, extensive syncytia formation was detected when using a mutant form of MLV-A glycoprotein with shortened cytoplasmic tail (MLV-A-Rless).

Expression of pH-dependent fowl plague virus hemagglutinin (FPV-HA) resulted in syncytia formation only when the co-cultures were incubated for 5 min at pH-5.

Expression of the pH-dependent G glycoprotein of VSV (vesicular stomatitis virus) resulted in small syncytia formation at neutral pH and in extensive syncytia formation at acid pH.

Although expression of HCV E1 or HCV E2 alone did not results in syncytia under either experimental conditions, co-expression of both E1 and E2 in 293T cells co-cultivated with Huh-7 cells was sufficient to induce the formation of syncytia. This indicates that under such experimental conditions, HCV E1E2 can fuse cells expressing the appropriate HCV receptors.

Moreover these results indicate that unlike most retroviral glycoproteins (eg., MLV-A), but like influenza virus hemagglutinin (eg., FPV-HA) or VSV-G, fusogenicity of HCV E1E2 is triggered by acid pH. These findings are confirmed by the sensitivity of the HCV pseudo-particles to inhibitors of endosomal acidifications (Table 2).

**Table 2.**

| pH-sensitivity of infection by HCV pseudo-particles | | | | |
|---|---|---|---|---|
| Glycoprotein¹ | Bafilomycin-A concentration (nM)² | | | |
| | 0 | 50 | 100 | 200 |
| MLV-A | 100 | 91.2 | 70 | 45 |
| FPV-HA | 100 | 12.8 | 2.3 | 2.7 |
| VSV-G | 100 | 27 | 7.3 | 0.5 |
| HCV-E1E2 | 100 | 12.8 | 4.3 | 3 |

| | | | | |
|---|---|---|---|---|
| ¹GFP-carrying pseudo-particles generated with the indicated glycoprotein were used to infect Huh-7 cells in the presence of the indicated concentration of Bafilomycin A. The inhibitor was removed after 3 hr and infectivity was determined by FACS analysis 3 days later. | | | | |
| ²results of infection for the indicated concentration of Bafilomycin-A are expressed as percentage of the infectivity determined in the absence of inhibitor. | | | | |

Thus, this novel cell-cell HCV fusion assay is highly valuable for the screening of molecules capable to inhibit HCV binding and cell entry by membrane fusion.

### Example 8: Assembly and increased infectivity of HCV pseudo-particles in the presence of HCV p7

The p7 protein may influence the conformational changes of the HCV envelope proteins that are perhaps important for cell binding and entry of the HCV virion. Accordingly, to gain more information on the p7 protein, expression constructs expressing a E1E2p7 polyprotein were generated.

The phCMV-ΔCE1E2p7 (phCMV 7a p7-1) expression vector expressing HCV 1a proteins delta core, E1, E2 and p7 was constructed using the Stu I, Bcl I digested fragment from plasmid pTM1p5E1E2p7 (Fournillier-Jacob et al, 1996; Cocquerel et al, 2000), comprising E1, E2 and p7 of HCV genotype 1a. This fragment was ligated into the previously described plasmid phCMV-7a after Bsu36 I digestion, blunting and subsequent digestion with Bcl I. The sequence of the phCMV-ΔCE1E2 vector is shown in SEQ ID No 14, whereas the aminoacid sequence of the ΔCE1E2 polyprotein is shown in SEQ ID No 15.

Infectivity of the HCV pseudo-particles generated with p7 (HCV-p7pp) was compared with that of HCVpp. Expression of p7 during assembly of the HCV pseudo-particles resulted in up to 5-fold increased titres. Similar increase of infectious titres mediated by p7 expression were obtained for HCVpp assembled with E1E2 glycoproteins derived from HCV genotypes 1a and 1b.

In conclusion, the invention provides a tool that allows precise investigation of viral assembly of E1E2 glycoproteins (processing, maturation) and their role in cell entry of HCV. No structural modifications of the E1E2 glycoproteins were required for their correct assembly on retroviral and lentiviral cores and to generate high titre infectious HCVpp with functional E1E2 glycoproteins. The insertion of a marker gene into the HCVpp allowed precise and rapid determination of the infectivity of these pseudo-particles by flow-cytometry.

The development of these functional, infectious HCV pseudo-particles make it now possible to investigate early events of HCV infection, such as the identification of novel HCV receptor(s) or co-receptor(s), to carry out diagnostic assays for the detection of neutralising antibodies in seroconverted patients, and to develop efficient inhibitors to HCV infection.

### REFERENCES

Agnello V. et *al., Proc Natl Acad Sci U S A* **96**, 12766-71. (1999).
Andre P. et *al*., *J Virol* ***76****,* 6919-28. (2002).
Ausubel F.M. et *al*. (eds.), Current Protocols in Molecular Biology, John Wiley & Sons, Inc. (1994).
Baumert T. F., Ito S., Wong D. T., Liang T. J., *J Virol* **72*,*** 3827-36. (1998).
Blight K. J., Kolykhalov A. A., Rice C. M., *Science* **290,** 1972-4. (2000).
Buonocore L., Blight K. J., Rice C. M., Rose J. K., *J Virol* **76**, 6865-72. (2002).
Calvo and Vega (1993) *J. Biol. Chem:* ***268****:18929-18935*
Cocquerel et *al*., *J Virol,* **74**/8, 3623-3633 (2000)
Dieterich (2002) *J Infect Dis* **185,** S128-37.
Dubuisson J. et *al., J Virol* **68**, 6147-60. (1994).
Felgner et *al. Science* **337,** 387-388. (1989).
Flint M. et *al*., *J Virol* **73**, 6235-44. (1999).
Flint et *al., J Virol* **74**, 702-709 (2000).
Fournillier-Jacob et *al., J Gen Virol,* **77***,* 1055-1064 (1996)
Guguen-Guillouzo and Guillouzo. Methods for preparation of adult and fetal hepatocytes. p 1-12. In A. Guillouzo and C. Guguen-Guillouzo (ed.), Isolated and cultured hepatocytes. Les Editions INSERM Paris. John Libbey and Co, Ltd., London, United Kingdom (1986).
Lavanchy D. et *al*., *J. Viral Hepat* **6**, 35-47 (1999).
Lindenbach B. D., Rice C. M., *Flaviviridae: The Viruses and Their Replication.* Knipe D. M., Howley P. M., Eds., Fields Virology, 4th ed. (Lippincott Williams & Wilkins, Philadelphia, Pa, 2001).
Lohmann V. et *al., Science* **285,** 110-3. (1999).
Matsuura Y. et al., Virology **286,** 263-75. (2001).
Nakabayashi H. et *al., Cancer Res* **42,** 3858-63. (1982).
Nègre D. et *al.*, *Gene Ther***7**, 1613-1623 (2000).
Op De Beeck A. et *al., J Biol Chem* **275,** 31428-37. (2000).
Op De Beeck A., L. Cocquerel, J. Dubuisson, *J Gen Virol* **82***,* 2589-95. (2001).
Owsianka A. et *al., J Gen Virol* **82**, 1877-83. (2001).
Patel A. H. et *al., J Gen Virol* **81**, 2873-83. (2000).
Pietschmann T. et *al*., *J Virol* **76**, 4008-21. (2002).
Pileri P. et *al*., *Science* **282**, 938-41. (1998).
Rose N. F. et *al., Cell* **106**, 539-49. (2001).
Sambrook, J., Fritsch, E. F., and Maniatis, T. *Molecular Cloning: A Laboratory Manual* 2nd Ed. , Cold Spring Harbor Laboratory, Cold Spring Harbor, NY (1989).
Sandrin V. et *al., Blood* **100**, 823-832 (2002).
Scarselli, E. et *al., EMBO J.* **21:** 5017-25 (2002).
Swanstrom R., Wills J. W., in *Retroviruses* J. M. Coffin, S. H. Hughes, H. E. Varmus, Eds. (Cold Spring Harbor Laboratory Press, New York, USA, 1997) pp. 263-334.
Wellnitz S. et *al*., *J Virol* **76**, 1181-93. (2002).
Webb et al., (1998) *J. Biol. Chem.,* **273***:15241-15248.*
Wu *et al.,* (1988) J. Biol. Chem. 263:14621-14624.
Wu *et al.,* (1992) J. Biol. Chem. 267:963-967.

## Claims

1. A method for producing hepacivirus-like particles *ex vivo* comprising the steps of:
- providing a first nucleic acid sequence comprising a packaging competent retroviral-derived genome;
- providing a second nucleic acid sequence comprising a cDNA encoding core proteins from said retrovirus;
- providing a third nucleic acid sequence comprising a cDNA encoding a polyprotein comprising successively a hepacivirus core protein, and a hepacivirus E1 protein and/or a hepacivirus E2 protein;
- transfecting host cells with said nucleic acid sequences and maintaining the transfected cells in culture for sufficient time to allow expression of the cDNAs to produce structural proteins from hepacivirus and retrovirus; and allowing the structural proteins to form virus-like particles.

2. The method according to claim 1, wherein said packaging competent retroviral-derived genome and core proteins are from a retrovirus selected from the group consisting of MLV, ALV, RSV, MPMV, HIV-1, HIV-2, SIV, EIAV, CAEV, or HFV.

3. The method according to claim 1 or 2, wherein said polyprotein comprises a hepacivirus core protein and a hepacivirus E1 protein.

4. The method according to any of claims 1 to 3, wherein said polyprotein comprises a hepacivirus core protein and a hepacivirus E2 protein.

5. The method according to any of claims 1 to 4, wherein said third nucleic acid sequence comprises a cDNA encoding a polyprotein that further comprises a hepacivirus p7 protein.

6. The method according to any of claims 1 to 5, wherein said polyprotein comprises native hepacivirus E1 and/or E2 protein, and optionally native hepacivirus p7 protein.

7. The method according to any of claims 1 to 6, wherein said polyprotein comprises a native hepacivirus core protein, a native hepacivirus E1 protein and native hepacivirus E2 protein, and optionally a native p7 protein.

8. The method according to any of preceding claims, wherein core protein, E1 protein and E2 protein, and optionally p7 protein, are derived from a same hepacivirus.

9. The method according to claim 8, wherein said hepacivirus is a hepatitis C virus (HCV).

10. The method according to claim 9, wherein said HCV core protein comprises the last 21 amino acids of the carboxy-terminus of HCV core

11. The method according to any of preceding claims, wherein said nucleic acid sequence comprising a packaging competent retroviral-derived genome further comprises a transgene.

12. An infectious hepacivirus-like particle susceptible to be obtained by a method according to any of preceding claims, comprising the core proteins from a retrovirus, and a E1 hepacivirus glycoprotein and/or a E2 hepacivirus glycoprotein.

13. The infectious particle according to claim 12, comprising E1 and E2 hepacivirus glycoproteins.

14. The infectious particle according to claim 12, comprising E1 hepacivirus glycoprotein.

15. The infectious particle according to claim 12, comprising E2 hepacivirus glycoprotein.

16. The infectious particle according to any of claims 12 to 15, further comprising a hepacivirus p7 protein.

17. The infectious particle according to any of claims 12 to 16, comprising native E1 and/or E2 hepacivirus glycoprotein, and optionally native p7 protein.

18. The infectious particle according to any of claims 12 to 17, wherein core E1 and D2 protein, and optionally p7 proteins, are derived from a same hepacivirus.

19. The infectious particles according to claim 18, wherein said hepacivirus is HCV.

20. The infectious particle according to any of claims 12 to 19, wherein said retrovirus is selected from the group consisting of MLV, ALV, RSV, MPMV, HIV-1, HIV-2, SIV, EIAV, CAEV, or HFV.

21. The infectious particle according to any of claims 12 to 20, wherein said nucleic acid sequence comprising a packaging competent retroviral-derived genome further comprises a transgene.

22. Use of three nucleic acid sequences for the preparation of a medicament useful as a vaccine against hepatitis, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a hepacivirus core protein, and a hepacivirus E1 protein and/or a hepacivirus E2 protein ;
and, when transferred into cells of a subject, the nucleic acids sequences allow the production of structural proteins from hepacivirus and retrovirus, wherein the structural proteins form virus-like particles that are immunogenic.

23. The use according to claim 22, wherein said third nucleic acid sequence comprises a cDNA encoding a polyprotein that further comprises a hepacivirus p7 protein.

24. The use according to claim 22 or 23, wherein said hepacivirus is HCV.

25. A method for *ex vivo* identification of a receptor for hepacivirus E1 and/or E2 glycoprotein comprising detection of the binding of an infectious particle according to any of claims 12 to 20, to a cell receptor.

26. A method for *ex vivo* identifying a cell receptor for hepacivirus comprising the step consisting of:
- transfecting a cell which is not permissive for hepacivirus infection with a nucleic acid sequence encoding a protein likely to be a receptor for hepacivirus;
- contacting said transformed cell with a hepacivirus-like particle according to any of claims 12 to 20;
- determining whether said transformed cell has become permissive or not for hepacivirus infection; and
- identifying as a cell receptor for hepacivirus said protein expressed by the transformed cell that has become permissive.

27. A method for ex vivo identifying a cell receptor for a hepacivirus comprising the step consisting of:
- providing an expression cDNA library obtained from a cell permissive for hepacivirus infection;
- transfecting cells that are not permissive for hepacivirus infection with said expression cDNA library;
- contacting said transformed cells with hepacivirus -like particles according to any of claims 12 to 20;
- identifying and isolating those transformed cells that have become permissive for hepacivirus infection;
- isolating the expression vector transfected in cells that have become permissive; and
- identifying as a receptor for hepacivirus the proteins encoded by the cDNA sequence of said isolated expression vectors.

28. A method of *ex vivo* screening or identification of molecules capable of interfering with hepacivirus entry in cells comprising comparison of the level of cell infection by an infectious particle according to any of claims 12 to 20 in the presence or the absence of a candidate molecule.

29. A method of *in vitro* diagnosis of a hepacivirus infection in a patient, comprising detecting immune complexes formed by interaction of anti-hepacivirus antibodies likely to be present in a biological sample of the patient with hepacivirus-like particle according to any of claims 12 to 20.

30. A method of *in vitro* diagnosis of a hepacivirus infection in a patient, comprising detecting an inhibitory effect of anti-hepacivirus antibodies likely to be present in a biological sample of the patient, on the infection of a permissive cell by hepacivirus-like particles according to any of claims 12 to 20.

31. A diagnostic kit useful for the method of claim 30, comprising a hepacivirus-like particle according to any of claim 12 to 20 and appropriate means of detection of said immune complexes.

32. Vaccine composition comprising a hepacivirus-like particle according to any of claims 12 to 21 and a pharmaceutically acceptable carrier.

33. A method for *in vitro* transferring a transgene of interest in a hepatic cell comprising infecting a cell with a hepacivirus-like particle as described in any of claims 12 to 21, wherein the hepacivirus-like particle carries a transgene of interest.

34. Use of a hepacivirus-like particle according to any of claims 12 to 21, that carries a transgene of interest, for the preparation of a medicament for the prevention or treatment of a disease in a patient, wherein the hepacivirus-like particle allows the transfer of the transgene of interest into a cell of the patient, and encodes a product that has a prophylactic or therapeutic effect against the disease.

35. A transformed host cell that contains:
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a hepacivirus core protein, and a hepacivirus E1 protein and/or a hepacivirus E2 protein.

36. The transformed host cell according to claim 35, wherein said third nucleic acid sequence comprises a cDNA encoding a polyprotein that further comprises a HCV p7 protein.

37. The transformed host cell according to claim 35 or 36, wherein said hepacivirus is HCV.

38. A method of *ex vivo* screening or identification of molecules capable of interfering with hepacivirus entry in cells comprising comparison the level of transformed host cell fusion to a target host cell, in the presence or the absence of a candidate molecule.

39. The method according to claim 38, comprising the steps consisting of:
- co-culturing a transformed host cell with a target host cell, in the absence or presence of a candidate molecule, under conditions that allow syncytia formation, *i.e*. cell-cell fusion, and hepacivirus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with hepacivirus entry the candidate molecule for which syncytia formation, as measured in the presence of said molecule, is decreased as compared to syncytia formation measured in the absence of any candidate molecule.

40. The method, according to any of claims 25 to 30, 33, 38 and 39, wherein said hepacivirus is HCV.


SEQUENCE LISTING

<110>  INSERM

<120>  Infectious HCV pseudo-particles containing functionnal E1,
E2 envelope proteins

<130>  BET 03/P0141

<160>  15

<170>  PatentIn version 3.2

<210>  1
<211>  21
<212>  PRT
<213>  Hepatitis C virus : delta C

<400>  1

Gly Cys Ser Phe Ser Ile Phe Leu Leu Ala Leu Leu Ser Cys Leu Thr
1               5                   10                  15

Val Pro Ala Ser Ala
            20

<210>  2
<211>  20
<212>  DNA
<213>  Artificial

<220>
<223>  primer

<400>  2
actggacgac gcaaagctgc
20

<210>  3
<211>  29
<212>  DNA
<213>  Artificial

<220>
<223>  primer

<400>  3
cgcggatcct acgcgtcgac gccggcaaa
29

<210>  4
<211>  35
<212>  DNA
<213>  Artificial

<220>
<223>  primer

<400> 4
tgcccgcttc agccgaaacc cacgtcaccg gggga
35


<210> 5
<211> 22
<212> DNA
<213> Artificial

<220>
<223> primer

<400> 5
gccagaagtc agatgctcaa gg
22


<210> 6
<211> 18
<212> DNA
<213> Artificial

<220>
<223> primer

<400> 6
tactctgagt ccaaaccg
18


<210> 7
<211> 35
<212> DNA
<213> Artificial

<220>
<223> primer

<400> 7
gtgacgtggg tttcggctga agcgggcaca gtcag
35


<210> 8
<211> 6274
<212> DNA
<213> Artificial

<220>
<223> phCMV-deltaCE1-1a plasmid


<220>
<221> promoter
<222> (1)..(768)
<223> human cytomegalovirus (hCMV) immediate-early promoter

<220>
<221> misc_feature

<222>  (769)..(1429)
<223>  rabbit beta-globin intron II

<220>
<221>  misc_feature
<222>  (1425)..(1430)
<223>  BamHI 5'junction after ligation of deltaCE1 fragment in
BamHI
       site of hCMV-G

<220>
<221>  misc_feature
<222>  (1430)..(2115)
<223>  IRES sequence of EMCV

<220>
<221>  CDS
<222>  (2116)..(2880)

<220>
<221>  misc_feature
<222>  (2125)..(2304)
<223>  deltaC part of the polyprotein

<220>
<221>  misc_feature
<222>  (2305)..(2880)
<223>  Mature E1 protein

<220>
<221>  misc_feature
<222>  (2883)..(2888)
<223>  BamHI 3' junction after ligation of deltaCE1 fragment in
BamHI
       site of hCMV-G

<400>  8
gcggccgctc tagagagctt ggcccattgc atacgttgta tccatatcat aatatgtaca
60

tttatattgg ctcatgtcca acattaccgc catgttgaca ttgattattg actagttatt
120

aatagtaatc aattacgggg tcattagttc atagcccata tatggagttc cgcgttacat
180

aacttacggt aaatggcccg cctggctgac cgcccaacga cccccgccca ttgacgtcaa
240

taatgacgta tgttcccata gtaacgccaa tagggacttt ccattgacgt caatgggtgg
300

agtatttacg gtaaactgcc cacttggcag tacatcaagt gtatcatatg ccaagtacgc
360

cccctattga cgtcaatgac ggtaaatggc ccgcctggca ttatgcccag tacatgacct
420

tatgggactt tcctacttgg cagtacatct acgtattagt catcgctatt accatggtga
480

tgcggttttg gcagtacatc aatgggcgtg gatagcggtt tgactcacgg ggatttccaa
540

gtctccaccc cattgacgtc aatgggagtt tgttttggca ccaaaatcaa cgggactttc
600

caaaatgtcg taacaactcc gccccattga cgcaaatggg cggtaggcgt gtacggtggg
660

aggtctatat aagcagagct cgtttagtga accgtcagat cgcctggaga cgccatccac
720

gctgttttga cctccataga agacaccggg accgatccag cctccggtcg accgatcctg
780

agaacttcag ggtgagtttg gggacccttg attgttcttt ctttttcgct attgtaaaat
840

tcatgttata tggagggggc aaagttttca gggtgttgtt tagaatggga agatgtccct
900

tgtatcacca tggaccctca tgataatttt gtttctttca ctttctactc tgttgacaac
960

cattgtctcc tcttattttc ttttcatttt ctgtaacttt ttcgttaaac tttagcttgc
1020

atttgtaacg aatttttaaa ttcacttttg tttatttgtc agattgtaag tactttctct
1080

aatcactttt ttttcaaggc aatcagggta tattatattg tacttcagca cagttttaga
1140

gaacaattgt tataattaaa tgataaggta gaatatttct gcatataaat tctggctggc
1200

gtggaaatat tcttattggt agaaacaact acaccctggt catcatcctg cctttctctt
1260

tatggttaca atgatataca ctgtttgaga tgaggataaa atactctgag tccaaaccgg
1320

gcccctctgc taaccatgtt catgccttct tctctttcct acagctcctg ggcaacgtgc
1380

tggttgttgt gctgtctcat cattttggca aagaattcct cgacggatcc gatgaaggac
1440

agttctttcc agacattgtt gaattgatct cgatcccgcg aaattaatac gactcactat
1500

agggagacca caacggtttc cctctagcgg gatcaattcc gcccctctcc ctcccccccc
1560

cctaacgtta ctggccgaag ccgcttggaa taaggccggt gtgcgtttgt ctatatgtta
1620

ttttccacca tattgccgtc ttttggcaat gtgagggccc ggaaacctgg ccctgtcttc
1680

ttgacgagca ttcctagggg tctttcccct ctcgccaaag gaatgcaagg tctgttgaat
1740

gtcgtgaagg aagcagttcc tctggaagct tcttgaagac aaacaacgtc tgtagcgacc
1800

ctttgcaggc agcggaaccc cccacctggc gacaggtgcc tctgcggcca aaagccacgt
1860

gtataagata cacctgcaaa ggcggcacaa ccccagtgcc acgttgtgag ttggatagtt
1920

gtggaaagag tcaaatggct ctcctcaagc gtattcaaca aggggctgaa ggatgcccag
1980

aaggtacccc attgtatggg atctgatctg gggcctcggt gcacatgctt tacatgtgtt
2040

tagtcgaggt taaaaaacgt ctaggccccc cgaaccacgg ggacgtggtt ttcctttgaa
2100

aaacacgata atacc atg aat tcc gac ctc atg ggg tac ata ccg ctc gtc
2151
Met Asn Ser Asp Leu Met Gly Tyr Ile Pro Leu Val
1               5                   10

ggc gcc cct ctt gga ggc gct gcc agg gcc ctg gcg cat ggc gtc cgg
2199
Gly Ala Pro Leu Gly Gly Ala Ala Arg Ala Leu Ala His Gly Val Arg
        15                  20                  25

gtt ctg gaa gac ggc gtg aac tat gca aca ggg aac ctt cct ggt tgc
2247
Val Leu Glu Asp Gly Val Asn Tyr Ala Thr Gly Asn Leu Pro Gly Cys
    30                  35                  40

ttt ttc tct atc ttc ctt ctg gcc ctg ctc tct tgc ctg act gtg ccc
2295
Phe Phe Ser Ile Phe Leu Leu Ala Leu Leu Ser Cys Leu Thr Val Pro
45                  50                  55                  60

gct tca gcc tac caa gtg cgc aat tcc tcg ggg ctt tac cat gtc acc
2343
Ala Ser Ala Tyr Gln Val Arg Asn Ser Ser Gly Leu Tyr His Val Thr
                65                  70                  75

aat gat tgc cct aat tcg agt att gtg tac gag gcg gcc gat gcc atc
2391
Asn Asp Cys Pro Asn Ser Ser Ile Val Tyr Glu Ala Ala Asp Ala Ile
            80                  85                  90

ctg cac act ccg ggg tgt gtc cct tgc gtt cgc gag ggt aac gcc tcg
2439
Leu His Thr Pro Gly Cys Val Pro Cys Val Arg Glu Gly Asn Ala Ser
        95                  100                 105

agg tgt tgg gtg gcg gtg acc ccc acg gtg gcc acc agg gac ggc aaa
2487
Arg Cys Trp Val Ala Val Thr Pro Thr Val Ala Thr Arg Asp Gly Lys
    110                 115                 120

ctc ccc aca acg cag ctt cga cgt cat atc gat ctg ctt gtc ggg agc
2535
Leu Pro Thr Thr Gln Leu Arg Arg His Ile Asp Leu Leu Val Gly Ser
125                 130                 135                 140

gcc acc ctc tgc tca gcc ctc tac gtg ggg gac ctg tgc ggg tct gtt
2583
Ala Thr Leu Cys Ser Ala Leu Tyr Val Gly Asp Leu Cys Gly Ser Val
                145                 150                 155

ttt ctt gtt ggt caa ctg ttt acc ttc tct ccc agg cgc cac tgg acg
2631
Phe Leu Val Gly Gln Leu Phe Thr Phe Ser Pro Arg Arg His Trp Thr
            160                 165                 170

acg caa agc tgc aat tgt tct atc tat ccc ggc cat ata acg ggt cat
2679
Thr Gln Ser Cys Asn Cys Ser Ile Tyr Pro Gly His Ile Thr Gly His
        175                 180                 185

cgc atg gca tgg gat atg atg atg aac tgg tcc cct acg gca gcg ttg
2727
Arg Met Ala Trp Asp Met Met Met Asn Trp Ser Pro Thr Ala Ala Leu
    190                 195                 200

gtg gta gct cag ctg ctc cgg atc cca caa gcc atc atg gac atg atc
2775
Val Val Ala Gln Leu Leu Arg Ile Pro Gln Ala Ile Met Asp Met Ile
205                 210                 215                 220

gct ggt gct cac tgg gga gtc ctg gcg ggc ata gcg tat ttc tcc atg
2823
Ala Gly Ala His Trp Gly Val Leu Ala Gly Ile Ala Tyr Phe Ser Met
                225                 230                 235

gtg ggg aac tgg gcg aag gtc ctg gta gtg ctg ctg tta ttt gcc ggc
2871
Val Gly Asn Trp Ala Lys Val Leu Val Val Leu Leu Leu Phe Ala Gly
            240                 245                 250

gtc gac gcg taggatccgt cgaggaattc actcctcagg tgcaggctgc
2920
Val Asp Ala
        255

ctatcagaag gtggtggctg gtgtggccaa tgccctggct cacaaatacc actgagatct
2980

ttttccctct gccaaaaatt atggggacat catgaagccc cttgagcatc tgacttctgg
3040

ctaataaagg aaatttattt tcattgcaat agtgtgttgg aattttttgt gtctctcact
3100

cggaaggaca tatgggaggg caaatcattt aaaacatcag aatgagtatt tggtttagag
3160

tttggcaaca tatgcccata tgctggctgc catgaacaaa ggttggctat aaagaggtca
3220

tcagtatatg aaacagcccc ctgctgtcca ttccttattc catagaaaag ccttgacttg
3280

aggttagatt ttttttatat tttgttttgt gttatttttt tctttaacat ccctaaaatt
3340

ttccttacat gttttactag ccagattttt cctcctctcc tgactactcc cagtcatagc
3400

tgtccctctt ctcttatgga gatccctcga cggatcggcc gcaattcgta atcatgtcat
3460

agctgtttcc tgtgtgaaat tgttatccgc tcacaattcc acacaacata cgagccggaa
3520

gcataaagtg taaagcctgg ggtgcctaat gagtgagcta actcacatta attgcgttgc
3580

gctcactgcc cgctttccag tcgggaaacc tgtcgtgcca gctgcattaa tgaatcggcc
3640

aacgcgcggg gagaggcggt ttgcgtattg ggcgctcttc cgcttcctcg ctcactgact
3700

cgctgcgctc ggtcgttcgg ctgcggcgag cggtatcagc tcactcaaag gcggtaatac
3760

ggttatccac agaatcaggg gataacgcag gaaagaacat gtgagcaaaa ggccagcaaa
3820

aggccaggaa ccgtaaaaag gccgcgttgc tggcgttttt ccataggctc cgcccccctg
3880

acgagcatca caaaaatcga cgctcaagtc agaggtggcg aaacccgaca ggactataaa
3940

gataccaggc gtttccccct ggaagctccc tcgtgcgctc tcctgttccg accctgccgc
4000

ttaccggata cctgtccgcc tttctccctt cgggaagcgt ggcgctttct catagctcac
4060

gctgtaggta tctcagttcg gtgtaggtcg ttcgctccaa gctgggctgt gtgcacgaac
4120

cccccgttca gcccgaccgc tgcgccttat ccggtaacta tcgtcttgag tccaacccgg
4180

taagacacga cttatcgcca ctggcagcag ccactggtaa caggattagc agagcgaggt
4240

atgtaggcgg tgctacagag ttcttgaagt ggtggcctaa ctacggctac actagaagaa
4300

cagtatttgg tatctgcgct ctgctgaagc cagttacctt cggaaaaaga gttggtagct
4360

cttgatccgg caaacaaacc accgctggta gcggtggttt ttttgtttgc aagcagcaga
4420

ttacgcgcag aaaaaaagga tctcaagaag atcctttgat cttttctacg gggtctgacg
4480

ctcagtggaa cgaaaactca cgttaaggga ttttggtcat gagattatca aaaaggatct
4540

tcacctagat ccttttaaat taaaaatgaa gttttaaatc aatctaaagt atatatgagt
4600

aaacttggtc tgacagttac caatgcttaa tcagtgaggc acctatctca gcgatctgtc
4660

tatttcgttc atccatagtt gcctgactcc ccgtcgtgta gataactacg atacgggagg
4720

gcttaccatc tggccccagt gctgcaatga taccgcgaga cccacgctca ccggctccag
4780

atttatcagc aataaaccag ccagccggaa gggccgagcg cagaagtggt cctgcaactt
4840

tatccgcctc catccagtct attaattgtt gccgggaagc tagagtaagt agttcgccag
4900

ttaatagttt gcgcaacgtt gttgccattg ctacaggcat cgtggtgtca cgctcgtcgt
4960

ttggtatggc ttcattcagc tccggttccc aacgatcaag gcgagttaca tgatccccca
5020

tgttgtgcaa aaaagcgggt tagctccttc ggtcctccga tcgttgtcag aagtaagttg
5080

gccgcagtgt tatcactcat ggttatggca gcactgcata attctcttac tgtcatgcca
5140

tccgtaagat gcttttctgt gactggtgag tactcaacca agtcattctg agaatagtgt
5200

atgcggcgac cgagttgctc ttgcccggcg tcaatacggg ataataccgc gccacatagc
5260

agaactttaa aagtgctcat cattggaaaa cgttcttcgg ggcgaaaact ctcaaggatc
5320

ttaccgctgt tgagatccag ttcgatgtaa cccactcgtg cacccaactg atcttcagca
5380

tcttttactt tcaccagcgt ttctgggtga gcaaaaacag gaaggcaaaa tgccgcaaaa
5440

aagggaataa gggcgacacg gaaatgttga atactcatac tcttcctttt tcaatattat
5500

tgaagcattt atcagggtta ttgtctcatg agcggataca tatttgaatg tatttagaaa
5560

aataaacaaa taggggttcc gcgcacattt ccccgaaaag tgccacctaa attgtaagcg
5620

ttaatatttt gttaaaattc gcgttaaatt tttgttaaat cagctcattt tttaaccaat
5680

aggccgaaat cggcaaaatc ccttataaat caaaagaata gaccgagata gggttgagtg
5740

ttgttccagt ttggaacaag agtccactat taaagaacgt ggactccaac gtcaaagggc
5800

gaaaaaccgt ctatcagggc gatggcccac tacgtgaacc atcaccctaa tcaagttttt
5860

tggggtcgag gtgccgtaaa gcactaaatc ggaaccctaa agggagcccc cgatttagag
5920

cttgacgggg aaagccggcg aacgtggcga gaaaggaagg gaagaaagcg aaaggagcgg
5980

gcgctagggc gctggcaagt gtagcggtca cgctgcgcgt aaccaccaca cccgccgcgc
6040

ttaatgcgcc gctacagggc gcgtcccatt cgccattcag gctgcgcaac tgttgggaag
6100

ggcgatcggt gcgggcctct tcgctattac gccagctggc gaaaggggga tgtgctgcaa
6160

ggcgattaag ttgggtaacg ccagggtttt cccagtcacg acgttgtaaa acgacggcca
6220

gtgagcgcgc gtaatacgac tcactatagg gcgaattgga gctccaccgc ggtg
6274


<210> 9
<211> 255
<212> PRT
<213> Artificial

<220>
<223> phCMV-deltaCE1-1a plasmid

<400> 9

Met Asn Ser Asp Leu Met Gly Tyr Ile Pro Leu Val Gly Ala Pro Leu
1               5                   10                  15


Gly Gly Ala Ala Arg Ala Leu Ala His Gly Val Arg Val Leu Glu Asp
            20                  25                  30


Gly Val Asn Tyr Ala Thr Gly Asn Leu Pro Gly Cys Phe Phe Ser Ile
        35                  40                  45


Phe Leu Leu Ala Leu Leu Ser Cys Leu Thr Val Pro Ala Ser Ala Tyr
    50                  55                  60


Gln Val Arg Asn Ser Ser Gly Leu Tyr His Val Thr Asn Asp Cys Pro

65                  70                  75                  80

Asn Ser Ser Ile Val Tyr Glu Ala Ala Asp Ala Ile Leu His Thr Pro
                85                  90                  95

Gly Cys Val Pro Cys Val Arg Glu Gly Asn Ala Ser Arg Cys Trp Val
            100                 105                 110

Ala Val Thr Pro Thr Val Ala Thr Arg Asp Gly Lys Leu Pro Thr Thr
        115                 120                 125

Gln Leu Arg Arg His Ile Asp Leu Leu Val Gly Ser Ala Thr Leu Cys
    130                 135                 140

Ser Ala Leu Tyr Val Gly Asp Leu Cys Gly Ser Val Phe Leu Val Gly
145                 150                 155                 160

Gln Leu Phe Thr Phe Ser Pro Arg Arg His Trp Thr Thr Gln Ser Cys
                165                 170                 175

Asn Cys Ser Ile Tyr Pro Gly His Ile Thr Gly His Arg Met Ala Trp
            180                 185                 190

Asp Met Met Met Asn Trp Ser Pro Thr Ala Ala Leu Val Val Ala Gln
        195                 200                 205

Leu Leu Arg Ile Pro Gln Ala Ile Met Asp Met Ile Ala Gly Ala His
    210                 215                 220

Trp Gly Val Leu Ala Gly Ile Ala Tyr Phe Ser Met Val Gly Asn Trp
225                 230                 235                 240

Ala Lys Val Leu Val Val Leu Leu Leu Phe Ala Gly Val Asp Ala
                245                 250                 255

<210>  10
<211>  7754
<212>  DNA
<213>  Artificial

<220>
<223>  phCMV-deltaCE1E2-1a plasmid


<220>
<221>  promoter
<222>  (1)..(768)
<223>  human cytomegalovirus (hCMV) immediate-early promoter

<220>
<221> misc_feature
<222> (769)..(1429)
<223> rabbit beta-globin intron II

<220>
<221> misc_feature
<222> (1425)..(1433)
<223> BamHI/ClaI junction after ligation of pTM-E1E2-745bit
      (ClaI-blunted/StuI) in BamHI-blunted of hCMV-G

<220>
<221> misc_feature
<222> (1430)..(2115)
<223> IRES sequence of EMCV

<220>
<221> CDS
<222> (2116)..(3966)

<220>
<221> misc_feature
<222> (2125)..(2304)
<223> deltaC part of the polyprotein

<220>
<221> misc_feature
<222> (2305)..(2880)
<223> Mature E1 protein

<220>
<221> misc_feature
<222> (2881)..(3966)
<223> Mature E2 protein

<220>
<221> misc_feature
<222> (3979)..(4354)
<223> Sinbis Virus junk sequence

<220>
<221> misc_feature
<222> (4361)..(4368)
<223> StuI/BamHI junction after ligation of pTM-E1E2-745bit
      (ClaI-blunted/StuI) in BamHI-blunted of hCMV-G

<400> 10
gcggccgctc tagagagctt ggcccattgc atacgttgta tccatatcat aatatgtaca
60

tttatattgg ctcatgtcca acattaccgc catgttgaca ttgattattg actagttatt
120

aatagtaatc aattacgggg tcattagttc atagcccata tatggagttc cgcgttacat
180

aacttacggt aaatggcccg cctggctgac cgcccaacga cccccgccca ttgacgtcaa
240

taatgacgta tgttcccata gtaacgccaa tagggacttt ccattgacgt caatgggtgg
300

agtatttacg gtaaactgcc cacttggcag tacatcaagt gtatcatatg ccaagtacgc
360
cccctattga cgtcaatgac ggtaaatggc ccgcctggca ttatgcccag tacatgacct
420
tatgggactt tcctacttgg cagtacatct acgtattagt catcgctatt accatggtga
480
tgcggttttg gcagtacatc aatgggcgtg gatagcggtt tgactcacgg ggatttccaa
540
gtctccaccc cattgacgtc aatgggagtt tgttttggca ccaaaatcaa cgggactttc
600
caaaatgtcg taacaactcc gccccattga cgcaaatggg cggtaggcgt gtacggtggg
660
aggtctatat aagcagagct cgtttagtga accgtcagat cgcctggaga cgccatccac
720
gctgttttga cctccataga agacaccggg accgatccag cctccggtcg accgatcctg
780
agaacttcag ggtgagtttg gggacccttg attgttcttt ctttttcgct attgtaaaat
840
tcatgttata tggagggggc aaagttttca gggtgttgtt tagaatggga agatgtccct
900
tgtatcacca tggaccctca tgataatttt gtttctttca ctttctactc tgttgacaac
960
cattgtctcc tcttattttc ttttcatttt ctgtaacttt ttcgttaaac tttagcttgc
1020
atttgtaacg aatttttaaa ttcacttttg tttatttgtc agattgtaag tactttctct
1080
aatcactttt ttttcaaggc aatcagggta tattatattg tacttcagca cagttttaga
1140
gaacaattgt tataattaaa tgataaggta gaatatttct gcatataaat tctggctggc
1200
gtggaaatat tcttattggt agaaacaact acaccctggt catcatcctg cctttctctt
1260
tatggttaca atgatataca ctgtttgaga tgaggataaa atactctgag tccaaaccgg
1320
gcccctctgc taaccatgtt catgccttct tctctttcct acagctcctg ggcaacgtgc
1380
tggttgttgt gctgtctcat cattttggca aagaattcct cgacggatcc gatgaaggac
1440
agttctttcc agacattgtt gaattgatct cgatcccgcg aaattaatac gactcactat
1500

agggagacca caacggtttc cctctagcgg gatcaattcc gcccctctcc ctcccccccc
1560

cctaacgtta ctggccgaag ccgcttggaa taaggccggt gtgcgtttgt ctatatgtta
1620

ttttccacca tattgccgtc ttttggcaat gtgagggccc ggaaacctgg ccctgtcttc
1680

ttgacgagca ttcctagggg tctttcccct ctcgccaaag gaatgcaagg tctgttgaat
1740

gtcgtgaagg aagcagttcc tctggaagct tcttgaagac aaacaacgtc tgtagcgacc
1800

ctttgcaggc agcggaaccc cccacctggc gacaggtgcc tctgcggcca aaagccacgt
1860

gtataagata cacctgcaaa ggcggcacaa ccccagtgcc acgttgtgag ttggatagtt
1920

gtggaaagag tcaaatggct ctcctcaagc gtattcaaca aggggctgaa ggatgcccag
1980

aaggtacccc attgtatggg atctgatctg gggcctcggt gcacatgctt tacatgtgtt
2040

tagtcgaggt taaaaaacgt ctaggccccc cgaaccacgg ggacgtggtt ttcctttgaa
2100

aaacacgata atacc atg aat tcc gac ctc atg ggg tac ata ccg ctc gtc
2151
                 Met Asn Ser Asp Leu Met Gly Tyr Ile Pro Leu Val
                 1               5                   10

ggc gcc cct ctt gga ggc gct gcc agg gcc ctg gcg cat ggc gtc cgg
2199
Gly Ala Pro Leu Gly Gly Ala Ala Arg Ala Leu Ala His Gly Val Arg
        15                  20                  25

gtt ctg gaa gac ggc gtg aac tat gca aca ggg aac ctt cct ggt tgc
2247
Val Leu Glu Asp Gly Val Asn Tyr Ala Thr Gly Asn Leu Pro Gly Cys
    30                  35                  40

ttt ttc tct atc ttc ctt ctg gcc ctg ctc tct tgc ctg act gtg ccc
2295
Phe Phe Ser Ile Phe Leu Leu Ala Leu Leu Ser Cys Leu Thr Val Pro
45                  50                  55                  60

gct tca gcc tac caa gtg cgc aat tcc tcg ggg ctt tac cat gtc acc
2343
Ala Ser Ala Tyr Gln Val Arg Asn Ser Ser Gly Leu Tyr His Val Thr
                65                  70                  75

aat gat tgc cct aat tcg agt att gtg tac gag gcg gcc gat gcc atc
2391
Asn Asp Cys Pro Asn Ser Ser Ile Val Tyr Glu Ala Ala Asp Ala Ile
            80                  85                  90

ctg cac act ccg ggg tgt gtc cct tgc gtt cgc gag ggt aac gcc tcg
2439
Leu His Thr Pro Gly Cys Val Pro Cys Val Arg Glu Gly Asn Ala Ser
        95                  100                 105

agg tgt tgg gtg gcg gtg acc ccc acg gtg gcc acc agg gac ggc aaa
2487
Arg Cys Trp Val Ala Val Thr Pro Thr Val Ala Thr Arg Asp Gly Lys
    110                 115                 120

ctc ccc aca acg cag ctt cga cgt cat atc gat ctg ctt gtc ggg agc
2535
Leu Pro Thr Thr Gln Leu Arg Arg His Ile Asp Leu Leu Val Gly Ser
125                 130                 135                 140

gcc acc ctc tgc tca gcc ctc tac gtg ggg gac ctg tgc ggg tct gtt
2583
Ala Thr Leu Cys Ser Ala Leu Tyr Val Gly Asp Leu Cys Gly Ser Val
                145                 150                 155

ttt ctt gtt ggt caa ctg ttt acc ttc tct ccc agg cgc cac tgg acg
2631
Phe Leu Val Gly Gln Leu Phe Thr Phe Ser Pro Arg Arg His Trp Thr
            160                 165                 170

acg caa agc tgc aat tgt tct atc tat ccc ggc cat ata acg ggt cat
2679
Thr Gln Ser Cys Asn Cys Ser Ile Tyr Pro Gly His Ile Thr Gly His
        175                 180                 185

cgc atg gca tgg gat atg atg atg aac tgg tcc cct acg gca gcg ttg
2727
Arg Met Ala Trp Asp Met Met Met Asn Trp Ser Pro Thr Ala Ala Leu
    190                 195                 200

gtg gta gct cag ctg ctc cgg atc cca caa gcc atc atg gac atg atc
2775
Val Val Ala Gln Leu Leu Arg Ile Pro Gln Ala Ile Met Asp Met Ile
205                 210                 215                 220

gct ggt gct cac tgg gga gtc ctg gcg ggc ata gcg tat ttc tcc atg
2823
Ala Gly Ala His Trp Gly Val Leu Ala Gly Ile Ala Tyr Phe Ser Met
                225                 230                 235

gtg ggg aac tgg gcg aag gtc ctg gta gtg ctg ctg tta ttt gcc ggc
2871
Val Gly Asn Trp Ala Lys Val Leu Val Val Leu Leu Leu Phe Ala Gly
            240                 245                 250

gtc gac gcg gaa acc cac gtc acc ggg gga agt gcc ggc cac acc acg
2919
Val Asp Ala Glu Thr His Val Thr Gly Gly Ser Ala Gly His Thr Thr
        255                 260                 265

gct ggg ctt gtt ggt ctc ctt aca cca ggc gcc aag cag aac atc caa
2967
Ala Gly Leu Val Gly Leu Leu Thr Pro Gly Ala Lys Gln Asn Ile Gln
    270                 275                 280

ctg atc aac acc aac ggc agt tgg cac atc aat agc acg gcc ttg aac
3015
Leu Ile Asn Thr Asn Gly Ser Trp His Ile Asn Ser Thr Ala Leu Asn
285                 290                 295                 300

tgc aac gat agc ctt acc acc ggc tgg tta gca ggg ctc ttc tat cgc
3063
Cys Asn Asp Ser Leu Thr Thr Gly Trp Leu Ala Gly Leu Phe Tyr Arg
                305                 310                 315

cac aaa ttc aac tct tca ggc tgt cct gag agg ttg gcc agc tgc cga
3111
His Lys Phe Asn Ser Ser Gly Cys Pro Glu Arg Leu Ala Ser Cys Arg
            320                 325                 330

cgc ctt acc gat ttt gcc cag ggc tgg ggt ccc atc agt tat gcc aac
3159
Arg Leu Thr Asp Phe Ala Gln Gly Trp Gly Pro Ile Ser Tyr Ala Asn
        335                 340                 345

gga agc ggc ctt gac gaa cgc ccc tac tgt tgg cac tac cct cca aga
3207
Gly Ser Gly Leu Asp Glu Arg Pro Tyr Cys Trp His Tyr Pro Pro Arg
    350                 355                 360

cct tgt ggc att gtg ccc gca aag agc gtg tgt ggc ccg gta tat tgc
3255
Pro Cys Gly Ile Val Pro Ala Lys Ser Val Cys Gly Pro Val Tyr Cys
365                 370                 375                 380

ttc act ccc agc ccc gtg gtg gtg gga acg acc gac agg tcg ggc gcg
3303
Phe Thr Pro Ser Pro Val Val Val Gly Thr Thr Asp Arg Ser Gly Ala
                385                 390                 395

cct acc tac agc tgg ggt gca aat gat acg gat gtc ttc gtc ctt aac
3351
Pro Thr Tyr Ser Trp Gly Ala Asn Asp Thr Asp Val Phe Val Leu Asn
            400                 405                 410

aac acc agg cca ccg ctg ggc aat tgg ttc ggt tgt acc tgg atg aac
3399
Asn Thr Arg Pro Pro Leu Gly Asn Trp Phe Gly Cys Thr Trp Met Asn
        415                 420                 425

tca act gga ttc acc aaa gtg tgc gga gcg ccc cct tgt gtc atc gga
3447
Ser Thr Gly Phe Thr Lys Val Cys Gly Ala Pro Pro Cys Val Ile Gly
    430                 435                 440

ggg gtg ggc aac aac acc ttg ctc tgc ccc act gat tgc ttc cgc aaa
3495
Gly Val Gly Asn Asn Thr Leu Leu Cys Pro Thr Asp Cys Phe Arg Lys
445                 450                 455                 460

cat ccg gaa gcc aca tac tct cgg tgc ggc tcc ggt ccc tgg att aca
3543
His Pro Glu Ala Thr Tyr Ser Arg Cys Gly Ser Gly Pro Trp Ile Thr
                465                 470                 475

ccc agg tgc atg gtc gac tac ccg tat agg ctt tgg cac tat cct tgt
3591
Pro Arg Cys Met Val Asp Tyr Pro Tyr Arg Leu Trp His Tyr Pro Cys
            480                 485                 490

act atc aat tac acc ata ttc aaa gtc agg atg tac gtg gga ggg gtc
3639
Thr Ile Asn Tyr Thr Ile Phe Lys Val Arg Met Tyr Val Gly Gly Val
        495                 500                 505

gag cac agg ctg gaa gcg gcc tgc aac tgg acg cgg ggc gaa cgc tgt
3687
Glu His Arg Leu Glu Ala Ala Cys Asn Trp Thr Arg Gly Glu Arg Cys
    510                 515                 520

gat ctg gaa gac agg gac agg tcc gag ctc agc cca ttg ctg ctg tcc
3735
Asp Leu Glu Asp Arg Asp Arg Ser Glu Leu Ser Pro Leu Leu Leu Ser
525                 530                 535                 540

acc aca cag tgg cag gtc ctt ccg tgt tct ttc acg acc ctg cca gcc
3783
Thr Thr Gln Trp Gln Val Leu Pro Cys Ser Phe Thr Thr Leu Pro Ala
                545                 550                 555

ttg tcc acc ggc ctc atc cac ctc cac cag aac att gtg gac gtg cag
3831
Leu Ser Thr Gly Leu Ile His Leu His Gln Asn Ile Val Asp Val Gln
            560                 565                 570

tac ttg tac ggg gtg ggg tca agc atc gcg tcc tgg gcc att aag tgg
3879
Tyr Leu Tyr Gly Val Gly Ser Ser Ile Ala Ser Trp Ala Ile Lys Trp
        575                 580                 585

gag tac gtc gtt ctc ctg ttc ctt ctg ctt gca gac gcg cgc gtc tgc
3927
Glu Tyr Val Val Leu Leu Phe Leu Leu Leu Ala Asp Ala Arg Val Cys
    590                 595                 600

tcc tgc ttg tgg atg atg tta ctc ata tcc caa gcg gag taagctcctt
3976
Ser Cys Leu Trp Met Met Leu Leu Ile Ser Gln Ala Glu
605                 610                 615

gggcccaatg atccgaccag caaaactcga tgtacttccg aggaactgat gtgcataatg
4036

catcaggctg gtacattaga tccccgctta ccgcgggcaa tatagcaaca ctaaaaactc
4096

gatgtacttc cgaggaagcg cagtgcataa tgctgcgcag tgttgccaca taaccactat
4156

attaaccatt tatctagcgg acgccaaaaa ctcaatgtat ttctgaggaa gcgtggtgca
4216

taatgccacg cagcgtctgc ataactttta ttatttcttt tattaatcaa caaaattttg
4276

tttttaacat ttcaaaaaaa aaaaaaaaaa aaaaaaaaaa aaaaaaaaaa gggaattcct
4336

cgattaatta agcggccgct cgagagggat ccgtcgagga attcactcct caggtgcagg
4396

ctgcctatca gaaggtggtg gctggtgtgg ccaatgccct ggctcacaaa taccactgag
4456

atctttttcc ctctgccaaa aattatgggg acatcatgaa gccccttgag catctgactt
4516

ctggctaata aaggaaattt attttcattg caatagtgtg ttggaatttt ttgtgtctct
4576

cactcggaag gacatatggg agggcaaatc atttaaaaca tcagaatgag tatttggttt
4636

agagtttggc aacatatgcc catatgctgg ctgccatgaa caaaggttgg ctataaagag
4696

gtcatcagta tatgaaacag ccccctgctg tccattcctt attccataga aaagccttga
4756

cttgaggtta gatttttttt atattttgtt ttgtgttatt tttttcttta acatccctaa
4816

aattttcctt acatgtttta ctagccagat ttttcctcct ctcctgacta ctcccagtca
4876

tagctgtccc tcttctctta tggagatccc tcgacggatc ggccgcaatt cgtaatcatg
4936

tcatagctgt ttcctgtgtg aaattgttat ccgctcacaa ttccacacaa catacgagcc
4996

ggaagcataa agtgtaaagc ctggggtgcc taatgagtga gctaactcac attaattgcg
5056

ttgcgctcac tgcccgcttt ccagtcggga aacctgtcgt gccagctgca ttaatgaatc
5116

ggccaacgcg cggggagagg cggtttgcgt attgggcgct cttccgcttc ctcgctcact
5176

gactcgctgc gctcggtcgt tcggctgcgg cgagcggtat cagctcactc aaaggcggta
5236

atacggttat ccacagaatc aggggataac gcaggaaaga acatgtgagc aaaaggccag
5296

caaaaggcca ggaaccgtaa aaaggccgcg ttgctggcgt ttttccatag gctccgcccc
5356

cctgacgagc atcacaaaaa tcgacgctca agtcagaggt ggcgaaaccc gacaggacta
5416

taaagatacc aggcgtttcc ccctggaagc tccctcgtgc gctctcctgt tccgaccctg
5476

ccgcttaccg gatacctgtc cgcctttctc ccttcgggaa gcgtggcgct ttctcatagc
5536

tcacgctgta ggtatctcag ttcggtgtag gtcgttcgct ccaagctggg ctgtgtgcac
5596

gaaccccccg ttcagcccga ccgctgcgcc ttatccggta actatcgtct tgagtccaac
5656

ccggtaagac acgacttatc gccactggca gcagccactg gtaacaggat tagcagagcg
5716

aggtatgtag gcggtgctac agagttcttg aagtggtggc ctaactacgg ctacactaga
5776

agaacagtat ttggtatctg cgctctgctg aagccagtta ccttcggaaa aagagttggt
5836

agctcttgat ccggcaaaca aaccaccgct ggtagcggtg gtttttttgt ttgcaagcag
5896

cagattacgc gcagaaaaaa aggatctcaa gaagatcctt tgatcttttc tacggggtct
5956

gacgctcagt ggaacgaaaa ctcacgttaa gggattttgg tcatgagatt atcaaaaagg
6016

atcttcacct agatcctttt aaattaaaaa tgaagtttta aatcaatcta aagtatatat
6076

gagtaaactt ggtctgacag ttaccaatgc ttaatcagtg aggcacctat ctcagcgatc
6136

tgtctatttc gttcatccat agttgcctga ctccccgtcg tgtagataac tacgatacgg
6196

gagggcttac catctggccc cagtgctgca atgataccgc gagacccacg ctcaccggct
6256

ccagatttat cagcaataaa ccagccagcc ggaagggccg agcgcagaag tggtcctgca
6316

actttatccg cctccatcca gtctattaat tgttgccggg aagctagagt aagtagttcg
6376

ccagttaata gtttgcgcaa cgttgttgcc attgctacag gcatcgtggt gtcacgctcg
6436

tcgtttggta tggcttcatt cagctccggt tcccaacgat caaggcgagt tacatgatcc
6496

cccatgttgt gcaaaaaagc gggttagctc cttcggtcct ccgatcgttg tcagaagtaa
6556

gttggccgca gtgttatcac tcatggttat ggcagcactg cataattctc ttactgtcat
6616

gccatccgta agatgctttt ctgtgactgg tgagtactca accaagtcat tctgagaata
6676

gtgtatgcgg cgaccgagtt gctcttgccc ggcgtcaata cgggataata ccgcgccaca
6736

tagcagaact ttaaaagtgc tcatcattgg aaaacgttct tcggggcgaa aactctcaag
6796

gatcttaccg ctgttgagat ccagttcgat gtaacccact cgtgcaccca actgatcttc
6856

agcatctttt actttcacca gcgtttctgg gtgagcaaaa acaggaaggc aaaatgccgc
6916

aaaaaaggga ataagggcga cacggaaatg ttgaatactc atactcttcc tttttcaata
6976

ttattgaagc atttatcagg gttattgtct catgagcgga tacatatttg aatgtattta
7036

gaaaaataaa caaatagggg ttccgcgcac atttccccga aaagtgccac ctaaattgta
7096

agcgttaata ttttgttaaa attcgcgtta aatttttgtt aaatcagctc attttttaac
7156

caataggccg aaatcggcaa aatcccttat aaatcaaaag aatagaccga gatagggttg
7216

agtgttgttc cagtttggaa caagagtcca ctattaaaga acgtggactc caacgtcaaa
7276

gggcgaaaaa ccgtctatca gggcgatggc ccactacgtg aaccatcacc ctaatcaagt
7336

tttttggggt cgaggtgccg taaagcacta aatcggaacc ctaaagggag cccccgattt
7396

agagcttgac ggggaaagcc ggcgaacgtg gcgagaaagg aagggaagaa agcgaaagga
7456

gcgggcgcta gggcgctggc aagtgtagcg gtcacgctgc gcgtaaccac cacacccgcc
7516

gcgcttaatg cgccgctaca gggcgcgtcc cattcgccat tcaggctgcg caactgttgg
7576

gaagggcgat cggtgcgggc ctcttcgcta ttacgccagc tggcgaaagg gggatgtgct
7636

gcaaggcgat taagttgggt aacgccaggg ttttcccagt cacgacgttg taaaacgacg
7696

gccagtgagc gcgcgtaata cgactcacta tagggcgaat tggagctcca ccgcggtg
7754

<210> 11
<211> 617
<212> PRT
<213> Artificial

<220>

<223>  phCMV-deltaCE1E2-1a plasmid

<400>  11

Met Asn Ser Asp Leu Met Gly Tyr Ile Pro Leu Val Gly Ala Pro Leu
1               5                   10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Tyr
    50                  55                  60

Gln Val Arg Asn Ser Ser Gly Leu Tyr His Val Thr Asn Asp Cys Pro
65                  70                  75                  80

Asn Ser Ser Ile Val Tyr Glu Ala Ala Asp Ala Ile Leu His Thr Pro
                85                  90                  95

Gly Cys Val Pro Cys Val Arg Glu Gly Asn Ala Ser Arg Cys Trp Val
            100                 105                 110

Ala Val Thr Pro Thr Val Ala Thr Arg Asp Gly Lys Leu Pro Thr Thr
        115                 120                 125

Gln Leu Arg Arg His Ile Asp Leu Leu Val Gly Ser Ala Thr Leu Cys
    130                 135                 140

Ser Ala Leu Tyr Val Gly Asp Leu Cys Gly Ser Val Phe Leu Val Gly
145                 150                 155                 160

Gln Leu Phe Thr Phe Ser Pro Arg Arg His Trp Thr Thr Gln Ser Cys
                165                 170                 175

Asn Cys Ser Ile Tyr Pro Gly His Ile Thr Gly His Arg Met Ala Trp
            180                 185                 190

Asp Met Met Met Asn Trp Ser Pro Thr Ala Ala Leu Val Val Ala Gln
        195                 200                 205

Leu Leu Arg Ile Pro Gln Ala Ile Met Asp Met Ile Ala Gly Ala His
    210                 215                 220

Trp Gly Val Leu Ala Gly Ile Ala Tyr Phe Ser Met Val Gly Asn Trp

225                 230                 235                 240

Ala Lys Val Leu Val Val Leu Leu Leu Phe Ala Gly Val Asp Ala Glu
                245                 250                 255

Thr His Val Thr Gly Gly Ser Ala Gly His Thr Thr Ala Gly Leu Val
            260                 265                 270

Gly Leu Leu Thr Pro Gly Ala Lys Gln Asn Ile Gln Leu Ile Asn Thr
        275                 280                 285

Asn Gly Ser Trp His Ile Asn Ser Thr Ala Leu Asn Cys Asn Asp Ser
    290                 295                 300

Leu Thr Thr Gly Trp Leu Ala Gly Leu Phe Tyr Arg His Lys Phe Asn
305                 310                 315                 320

Ser Ser Gly Cys Pro Glu Arg Leu Ala Ser Cys Arg Arg Leu Thr Asp
                325                 330                 335

Phe Ala Gln Gly Trp Gly Pro Ile Ser Tyr Ala Asn Gly Ser Gly Leu
            340                 345                 350

Asp Glu Arg Pro Tyr Cys Trp His Tyr Pro Pro Arg Pro Cys Gly Ile
        355                 360                 365

Val Pro Ala Lys Ser Val Cys Gly Pro Val Tyr Cys Phe Thr Pro Ser
    370                 375                 380

Pro Val Val Val Gly Thr Thr Asp Arg Ser Gly Ala Pro Thr Tyr Ser
385                 390                 395                 400

Trp Gly Ala Asn Asp Thr Asp Val Phe Val Leu Asn Asn Thr Arg Pro
                405                 410                 415

Pro Leu Gly Asn Trp Phe Gly Cys Thr Trp Met Asn Ser Thr Gly Phe
            420                 425                 430

Thr Lys Val Cys Gly Ala Pro Pro Cys Val Ile Gly Gly Val Gly Asn
        435                 440                 445

Asn Thr Leu Leu Cys Pro Thr Asp Cys Phe Arg Lys His Pro Glu Ala
    450                 455                 460

Thr Tyr Ser Arg Cys Gly Ser Gly Pro Trp Ile Thr Pro Arg Cys Met
465                 470                 475                 480

Val Asp Tyr Pro Tyr Arg Leu Trp His Tyr Pro Cys Thr Ile Asn Tyr
                485                 490                 495

Thr Ile Phe Lys Val Arg Met Tyr Val Gly Gly Val Glu His Arg Leu
            500                 505                 510

Glu Ala Ala Cys Asn Trp Thr Arg Gly Glu Arg Cys Asp Leu Glu Asp
        515                 520                 525

Arg Asp Arg Ser Glu Leu Ser Pro Leu Leu Leu Ser Thr Thr Gln Trp
    530                 535                 540

Gln Val Leu Pro Cys Ser Phe Thr Thr Leu Pro Ala Leu Ser Thr Gly
545                 550                 555                 560

Leu Ile His Leu His Gln Asn Ile Val Asp Val Gln Tyr Leu Tyr Gly
                565                 570                 575

Val Gly Ser Ser Ile Ala Ser Trp Ala Ile Lys Trp Glu Tyr Val Val
            580                 585                 590

Leu Leu Phe Leu Leu Leu Ala Asp Ala Arg Val Cys Ser Cys Leu Trp
        595                 600                 605

Met Met Leu Leu Ile Ser Gln Ala Glu
    610                 615

<210>  12
<211>  7178
<212>  DNA
<213>  Artificial

<220>
<223>  phCMV-deltaCE2-1a plasmid


<220>
<221>  promoter
<222>  (1)..(768)
<223>  human cytomegalovirus (hCMV) immediate-early promoter

<220>
<221>  misc_feature
<222>  (769)..(1429)
<223>  rabbit beta-globin intron II

<220>
<221>  misc_feature
<222>  (1425)..(1430)

<223>  BamHI 5'junction after ligation of deltaCE2 fragment in
BamHI
       site of hCMV-G

<220>
<221>  misc_feature
<222>  (1430)..(2115)
<223>  IRES sequence of EMCV

<220>
<221>  CDS
<222>  (2116)..(3390)

<220>
<221>  misc_feature
<222>  (2125)..(2304)
<223>  deltaC part of the polyprotein

<220>
<221>  misc_feature
<222>  (2305)..(3390)
<223>  Mature E2 protein

<220>
<221>  misc_feature
<222>  (3403)..(3778)
<223>  Sinbis Virus junk sequence

<220>
<221>  misc_feature
<222>  (3787)..(3792)
<223>  BamHI 3' junction after ligation of deltaCE1 fragment in
BamHI
       site of hCMV-G

<400>  12
   gcggccgctc tagagagctt ggcccattgc atacgttgta tccatatcat aatatgtaca
   60
   tttatattgg ctcatgtcca acattaccgc catgttgaca ttgattattg actagttatt
   120
   aatagtaatc aattacgggg tcattagttc atagcccata tatggagttc cgcgttacat
   180
   aacttacggt aaatggcccg cctggctgac cgcccaacga cccccgccca ttgacgtcaa
   240
   taatgacgta tgttcccata gtaacgccaa tagggacttt ccattgacgt caatgggtgg
   300
   agtatttacg gtaaactgcc cacttggcag tacatcaagt gtatcatatg ccaagtacgc
   360
   cccctattga cgtcaatgac ggtaaatggc ccgcctggca ttatgcccag tacatgacct
   420
   tatgggactt tcctacttgg cagtacatct acgtattagt catcgctatt accatggtga
   480
   tgcggttttg gcagtacatc aatgggcgtg gatagcggtt tgactcacgg ggatttccaa
   540
   gtctccaccc cattgacgtc aatgggagtt tgttttggca ccaaaatcaa cgggactttc
   600
   caaaatgtcg taacaactcc gccccattga cgcaaatggg cggtaggcgt gtacggtggg
   660
   aggtctatat aagcagagct cgtttagtga accgtcagat cgcctggaga cgccatccac
   720
   gctgttttga cctccataga agacaccggg accgatccag cctccggtcg accgatcctg
   780
   agaacttcag ggtgagtttg gggacccttg attgttcttt ctttttcgct attgtaaaat
   840
   tcatgttata tggagggggc aaagttttca gggtgttgtt tagaatggga agatgtccct
   900
   tgtatcacca tggaccctca tgataatttt gtttctttca ctttctactc tgttgacaac
   960
   cattgtctcc tcttattttc ttttcatttt ctgtaacttt ttcgttaaac tttagcttgc
   1020
   atttgtaacg aatttttaaa ttcacttttg tttatttgtc agattgtaag tactttctct
   1080
   aatcactttt ttttcaaggc aatcagggta tattatattg tacttcagca cagttttaga
   1140
   gaacaattgt tataattaaa tgataaggta gaatatttct gcatataaat tctggctggc
   1200
   gtggaaatat tcttattggt agaaacaact acaccctggt catcatcctg cctttctctt
   1260
   tatggttaca atgatataca ctgtttgaga tgaggataaa atactctgag tccaaaccgg
   1320
   gcccctctgc taaccatgtt catgccttct tctctttcct acagctcctg ggcaacgtgc
   1380
   tggttgttgt gctgtctcat cattttggca aagaattcct cgacggatcc gatgaaggac
   1440
   agttctttcc agacattgtt gaattgatct cgatcccgcg aaattaatac gactcactat
   1500
   agggagacca caacggtttc cctctagcgg gatcaattcc gcccctctcc ctcccccccc
   1560
   cctaacgtta ctggccgaag ccgcttggaa taaggccggt gtgcgtttgt ctatatgtta
   1620
   ttttccacca tattgccgtc ttttggcaat gtgagggccc ggaaacctgg ccctgtcttc
   1680
   ttgacgagca ttcctagggg tctttcccct ctcgccaaag gaatgcaagg tctgttgaat
   1740
   gtcgtgaagg aagcagttcc tctggaagct tcttgaagac aaacaacgtc tgtagcgacc
   1800
   ctttgcaggc agcggaaccc cccacctggc gacaggtgcc tctgcggcca aaagccacgt
   1860
   gtataagata cacctgcaaa ggcggcacaa ccccagtgcc acgttgtgag ttggatagtt
   1920
   gtggaaagag tcaaatggct ctcctcaagc gtattcaaca aggggctgaa ggatgcccag
   1980
   aaggtacccc attgtatggg atctgatctg gggcctcggt gcacatgctt tacatgtgtt
   2040
   tagtcgaggt taaaaaacgt ctaggccccc cgaaccacgg ggacgtggtt ttcctttgaa
   2100
   aaacacgata atacc atg aat tcc gac ctc atg ggg tac ata ccg ctc gtc
   2151
   Met Asn Ser Asp Leu Met Gly Tyr Ile Pro Leu Val
   1 5 10
   ggc gcc cct ctt gga ggc gct gcc agg gcc ctg gcg cat ggc gtc cgg
   2199
   Gly Ala Pro Leu Gly Gly Ala Ala Arg Ala Leu Ala His Gly Val Arg
   15 20 25
   gtt ctg gaa gac ggc gtg aac tat gca aca ggg aac ctt cct ggt tgc
   2247
   Val Leu Glu Asp Gly Val Asn Tyr Ala Thr Gly Asn Leu Pro Gly Cys
   30 35 40
   ttt ttc tct atc ttc ctt ctg gcc ctg ctc tct tgc ctg act gtg ccc
   2295
   Phe Phe Ser Ile Phe Leu Leu Ala Leu Leu Ser Cys Leu Thr Val Pro
   45 50 55 60
   gct tca gcc gaa acc cac gtc acc ggg gga agt gcc ggc cac acc acg
   2343
   Ala Ser Ala Glu Thr His Val Thr Gly Gly Ser Ala Gly His Thr Thr
   65 70 75
   gct ggg ctt gtt ggt ctc ctt aca cca ggc gcc aag cag aac atc caa
   2391
   Ala Gly Leu Val Gly Leu Leu Thr Pro Gly Ala Lys Gln Asn Ile Gln
   80 85 90
   ctg atc aac acc aac ggc agt tgg cac atc aat agc acg gcc ttg aac
   2439
   Leu Ile Asn Thr Asn Gly Ser Trp His Ile Asn Ser Thr Ala Leu Asn
   95 100 105
   tgc aac gat agc ctt acc acc ggc tgg tta gca ggg ctc ttc tat cgc
   2487
   Cys Asn Asp Ser Leu Thr Thr Gly Trp Leu Ala Gly Leu Phe Tyr Arg
   110 115 120
   cac aaa ttc aac tct tca ggc tgt cct gag agg ttg gcc agc tgc cga
   2535
   His Lys Phe Asn Ser Ser Gly Cys Pro Glu Arg Leu Ala Ser Cys Arg
   125                 130                 135                 140
   cgc ctt acc gat ttt gcc cag ggc tgg ggt ccc atc agt tat gcc aac
   2583
   Arg Leu Thr Asp Phe Ala Gln Gly Trp Gly Pro Ile Ser Tyr Ala Asn
                   145                 150                 155
   gga agc ggc ctt gac gaa cgc ccc tac tgt tgg cac tac cct cca aga
   2631
   Gly Ser Gly Leu Asp Glu Arg Pro Tyr Cys Trp His Tyr Pro Pro Arg
               160                 165                 170
   cct tgt ggc att gtg ccc gca aag agc gtg tgt ggc ccg gta tat tgc
   2679
   Pro Cys Gly Ile Val Pro Ala Lys Ser Val Cys Gly Pro Val Tyr Cys
           175                 180                 185
   ttc act ccc agc ccc gtg gtg gtg gga acg acc gac agg tcg ggc gcg
   2727
   Phe Thr Pro Ser Pro Val Val Val Gly Thr Thr Asp Arg Ser Gly Ala
       190                 195                 200
   cct acc tac agc tgg ggt gca aat gat acg gat gtc ttc gtc ctt aac
   2775
   Pro Thr Tyr Ser Trp Gly Ala Asn Asp Thr Asp Val Phe Val Leu Asn
   205                 210                 215                 220
   aac acc agg cca ccg ctg ggc aat tgg ttc ggt tgt acc tgg atg aac
   2823
   Asn Thr Arg Pro Pro Leu Gly Asn Trp Phe Gly Cys Thr Trp Met Asn
                   225                 230                 235
   tca act gga ttc acc aaa gtg tgc gga gcg ccc cct tgt gtc atc gga
   2871
   Ser Thr Gly Phe Thr Lys Val Cys Gly Ala Pro Pro Cys Val Ile Gly
               240                 245                 250
   ggg gtg ggc aac aac acc ttg ctc tgc ccc act gat tgc ttc cgc aaa
   2919
   Gly Val Gly Asn Asn Thr Leu Leu Cys Pro Thr Asp Cys Phe Arg Lys
           255                 260                 265
   cat ccg gaa gcc aca tac tct cgg tgc ggc tcc ggt ccc tgg att aca
   2967
   His Pro Glu Ala Thr Tyr Ser Arg Cys Gly Ser Gly Pro Trp Ile Thr
       270                 275                 280
   ccc agg tgc atg gtc gac tac ccg tat agg ctt tgg cac tat cct tgt
   3015
   Pro Arg Cys Met Val Asp Tyr Pro Tyr Arg Leu Trp His Tyr Pro Cys
   285                 290                 295                 300
   act atc aat tac acc ata ttc aaa gtc agg atg tac gtg gga ggg gtc
   3063
   Thr Ile Asn Tyr Thr Ile Phe Lys Val Arg Met Tyr Val Gly Gly Val
                   305                 310                 315
   gag cac agg ctg gaa gcg gcc tgc aac tgg acg cgg ggc gaa cgc tgt
   3111
   Glu His Arg Leu Glu Ala Ala Cys Asn Trp Thr Arg Gly Glu Arg Cys
               320                 325                 330
   gat ctg gaa gac agg gac agg tcc gag ctc agc cca ttg ctg ctg tcc
   3159
   Asp Leu Glu Asp Arg Asp Arg Ser Glu Leu Ser Pro Leu Leu Leu Ser
           335                 340                 345
   acc aca cag tgg cag gtc ctt ccg tgt tct ttc acg acc ctg cca gcc
   3207
   Thr Thr Gln Trp Gln Val Leu Pro Cys Ser Phe Thr Thr Leu Pro Ala
       350                 355                 360
   ttg tcc acc ggc ctc atc cac ctc cac cag aac att gtg gac gtg cag
   3255
   Leu Ser Thr Gly Leu Ile His Leu His Gln Asn Ile Val Asp Val Gln
   365                 370                 375                 380
   tac ttg tac ggg gtg ggg tca agc atc gcg tcc tgg gcc att aag tgg
   3303
   Tyr Leu Tyr Gly Val Gly Ser Ser Ile Ala Ser Trp Ala Ile Lys Trp
                   385                 390                 395
   gag tac gtc gtt ctc ctg ttc ctt ctg ctt gca gac gcg cgc gtc tgc
   3351
   Glu Tyr Val Val Leu Leu Phe Leu Leu Leu Ala Asp Ala Arg Val Cys
               400                 405                 410
   tcc tgc ttg tgg atg atg tta ctc ata tcc caa gcg gag taagctcctt
   3400
   Ser Cys Leu Trp Met Met Leu Leu Ile Ser Gln Ala Glu
           415                 420                 425
   gggcccaatg atccgaccag caaaactcga tgtacttccg aggaactgat gtgcataatg
   3460
   catcaggctg gtacattaga tccccgctta ccgcgggcaa tatagcaaca ctaaaaactc
   3520
   gatgtacttc cgaggaagcg cagtgcataa tgctgcgcag tgttgccaca taaccactat
   3580
   attaaccatt tatctagcgg acgccaaaaa ctcaatgtat ttctgaggaa gcgtggtgca
   3640
   taatgccacg cagcgtctgc ataactttta ttatttcttt tattaatcaa caaaattttg
   3700
   tttttaacat ttcaaaaaaa aaaaaaaaaa aaaaaaaaaa aaaaaaaaaa gggaattcct
   3760
   cgattaatta agcggccgct cgagagggat ccgtcgagga attcactcct caggtgcagg
   3820
   ctgcctatca gaaggtggtg gctggtgtgg ccaatgccct ggctcacaaa taccactgag
   3880
   atctttttcc ctctgccaaa aattatgggg acatcatgaa gccccttgag catctgactt
   3940
   ctggctaata aaggaaattt attttcattg caatagtgtg ttggaatttt ttgtgtctct
   4000
   cactcggaag gacatatggg agggcaaatc atttaaaaca tcagaatgag tatttggttt
   4060
   agagtttggc aacatatgcc catatgctgg ctgccatgaa caaaggttgg ctataaagag
   4120
   gtcatcagta tatgaaacag ccccctgctg tccattcctt attccataga aaagccttga
   4180
   cttgaggtta gatttttttt atattttgtt ttgtgttatt tttttcttta acatccctaa
   4240
   aattttcctt acatgtttta ctagccagat ttttcctcct ctcctgacta ctcccagtca
   4300
   tagctgtccc tcttctctta tggagatccc tcgacggatc ggccgcaatt cgtaatcatg
   4360
   tcatagctgt ttcctgtgtg aaattgttat ccgctcacaa ttccacacaa catacgagcc
   4420
   ggaagcataa agtgtaaagc ctggggtgcc taatgagtga gctaactcac attaattgcg
   4480
   ttgcgctcac tgcccgcttt ccagtcggga aacctgtcgt gccagctgca ttaatgaatc
   4540
   ggccaacgcg cggggagagg cggtttgcgt attgggcgct cttccgcttc ctcgctcact
   4600
   gactcgctgc gctcggtcgt tcggctgcgg cgagcggtat cagctcactc aaaggcggta
   4660
   atacggttat ccacagaatc aggggataac gcaggaaaga acatgtgagc aaaaggccag
   4720
   caaaaggcca ggaaccgtaa aaaggccgcg ttgctggcgt ttttccatag gctccgcccc
   4780
   cctgacgagc atcacaaaaa tcgacgctca agtcagaggt ggcgaaaccc gacaggacta
   4840
   taaagatacc aggcgtttcc ccctggaagc tccctcgtgc gctctcctgt tccgaccctg
   4900
   ccgcttaccg gatacctgtc cgcctttctc ccttcgggaa gcgtggcgct ttctcatagc
   4960
   tcacgctgta ggtatctcag ttcggtgtag gtcgttcgct ccaagctggg ctgtgtgcac
   5020
   gaaccccccg ttcagcccga ccgctgcgcc ttatccggta actatcgtct tgagtccaac
   5080
   ccggtaagac acgacttatc gccactggca gcagccactg gtaacaggat tagcagagcg
   5140
   aggtatgtag gcggtgctac agagttcttg aagtggtggc ctaactacgg ctacactaga
   5200
   agaacagtat ttggtatctg cgctctgctg aagccagtta ccttcggaaa aagagttggt
   5260
   agctcttgat ccggcaaaca aaccaccgct ggtagcggtg gtttttttgt ttgcaagcag
   5320
   cagattacgc gcagaaaaaa aggatctcaa gaagatcctt tgatcttttc tacggggtct
   5380
   gacgctcagt ggaacgaaaa ctcacgttaa gggattttgg tcatgagatt atcaaaaagg
   5440
   atcttcacct agatcctttt aaattaaaaa tgaagtttta aatcaatcta aagtatatat
   5500
   gagtaaactt ggtctgacag ttaccaatgc ttaatcagtg aggcacctat ctcagcgatc
   5560
   tgtctatttc gttcatccat agttgcctga ctccccgtcg tgtagataac tacgatacgg
   5620
   gagggcttac catctggccc cagtgctgca atgataccgc gagacccacg ctcaccggct
   5680
   ccagatttat cagcaataaa ccagccagcc ggaagggccg agcgcagaag tggtcctgca
   5740
   actttatccg cctccatcca gtctattaat tgttgccggg aagctagagt aagtagttcg
   5800
   ccagttaata gtttgcgcaa cgttgttgcc attgctacag gcatcgtggt gtcacgctcg
   5860
   tcgtttggta tggcttcatt cagctccggt tcccaacgat caaggcgagt tacatgatcc
   5920
   cccatgttgt gcaaaaaagc ggttagctcc ttcggtcctc cgatcgttgt cagaagtaag
   5980
   gttggccgca gtgttatcac tcatggttat ggcagcactg cataattctc ttactgtcat
   6040
   gccatccgta agatgctttt ctgtgactgg tgagtactca accaagtcat tctgagaata
   6100
   gtgtatgcgg cgaccgagtt gctcttgccc ggcgtcaata cgggataata ccgcgccaca
   6160
   tagcagaact ttaaaagtgc tcatcattgg aaaacgttct tcggggcgaa aactctcaag
   6220
   gatcttaccg ctgttgagat ccagttcgat gtaacccact cgtgcaccca actgatcttc
   6280
   agcatctttt actttcacca gcgtttctgg gtgagcaaaa acaggaaggc aaaatgccgc
   6340
   aaaaaaggga ataagggcga cacggaaatg ttgaatactc atactcttcc tttttcaata
   6400
   ttattgaagc atttatcagg gttattgtct catgagcgga tacatatttg aatgtattta
   6460
   gaaaaataaa caaatagggg ttccgcgcac atttccccga aaagtgccac ctaaattgta
   6520
   agcgttaata ttttgttaaa attcgcgtta aatttttgtt aaatcagctc attttttaac
   6580
   caataggccg aaatcggcaa aatcccttat aaatcaaaag aatagaccga gatagggttg
   6640
   agtgttgttc cagtttggaa caagagtcca ctattaaaga acgtggactc caacgtcaaa
   6700
   gggcgaaaaa ccgtctatca gggcgatggc ccactacgtg aaccatcacc ctaatcaagt
   6760
   tttttggggt cgaggtgccg taaagcacta aatcggaacc ctaaagggag cccccgattt
   6820
   agagcttgac ggggaaagcc ggcgaacgtg gcgagaaagg aagggaagaa agcgaaagga
   6880
   gcgggcgcta gggcgctggc aagtgtagcg gtcacgctgc gcgtaaccac cacacccgcc
   6940
   gcgcttaatg cgccgctaca gggcgcgtcc cattcgccat tcaggctgcg caactgttgg
   7000
   gaagggcgat cggtgcgggc ctcttcgcta ttacgccagc tggcgaaagg gggatgtgct
   7060
   gcaaggcgat taagttgggt aacgccaggg ttttcccagt cacgacgttg taaaacgacg
   7120
   gccagtgagc gcgcgtaata cgactcacta tagggcgaat tggagctcca ccgcggtg
   7178

<210> 13
<211> 425
<212> PRT
<213> Artificial

<220>
<223> phCMV-deltaCE2-1a plasmid

<400> 13

Met Asn Ser Asp Leu Met Gly Tyr Ile Pro Leu Val Gly Ala Pro Leu
1               5                   10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Glu
    50                  55                  60

Thr His Val Thr Gly Gly Ser Ala Gly His Thr Thr Ala Gly Leu Val
65                  70                  75                  80

Gly Leu Leu Thr Pro Gly Ala Lys Gln Asn Ile Gln Leu Ile Asn Thr
                85                  90                  95

Asn Gly Ser Trp His Ile Asn Ser Thr Ala Leu Asn Cys Asn Asp Ser
            100                 105                 110

Leu Thr Thr Gly Trp Leu Ala Gly Leu Phe Tyr Arg His Lys Phe Asn
        115                 120                 125

Ser Ser Gly Cys Pro Glu Arg Leu Ala Ser Cys Arg Arg Leu Thr Asp
    130                 135                 140

Phe Ala Gln Gly Trp Gly Pro Ile Ser Tyr Ala Asn Gly Ser Gly Leu
145                 150                 155                 160

Asp Glu Arg Pro Tyr Cys Trp His Tyr Pro Pro Arg Pro Cys Gly Ile
                165                 170                 175

Val Pro Ala Lys Ser Val Cys Gly Pro Val Tyr Cys Phe Thr Pro Ser
            180                 185                 190

Pro Val Val Val Gly Thr Thr Asp Arg Ser Gly Ala Pro Thr Tyr Ser
        195                 200                 205

Trp Gly Ala Asn Asp Thr Asp Val Phe Val Leu Asn Asn Thr Arg Pro
    210                 215                 220

Pro Leu Gly Asn Trp Phe Gly Cys Thr Trp Met Asn Ser Thr Gly Phe
225                 230                 235                 240

Thr Lys Val Cys Gly Ala Pro Pro Cys Val Ile Gly Gly Val Gly Asn
                245                 250                 255

Asn Thr Leu Leu Cys Pro Thr Asp Cys Phe Arg Lys His Pro Glu Ala
            260                 265                 270

Thr Tyr Ser Arg Cys Gly Ser Gly Pro Trp Ile Thr Pro Arg Cys Met

        275                 280                 285

Val Asp Tyr Pro Tyr Arg Leu Trp His Tyr Pro Cys Thr Ile Asn Tyr
    290                 295                 300

Thr Ile Phe Lys Val Arg Met Tyr Val Gly Gly Val Glu His Arg Leu
305                 310                 315                 320

Glu Ala Ala Cys Asn Trp Thr Arg Gly Glu Arg Cys Asp Leu Glu Asp
                325                 330                 335

Arg Asp Arg Ser Glu Leu Ser Pro Leu Leu Leu Ser Thr Thr Gln Trp
            340                 345                 350

Gln Val Leu Pro Cys Ser Phe Thr Thr Leu Pro Ala Leu Ser Thr Gly
        355                 360                 365

Leu Ile His Leu His Gln Asn Ile Val Asp Val Gln Tyr Leu Tyr Gly
    370                 375                 380

Val Gly Ser Ser Ile Ala Ser Trp Ala Ile Lys Trp Glu Tyr Val Val
385                 390                 395                 400

Leu Leu Phe Leu Leu Leu Ala Asp Ala Arg Val Cys Ser Cys Leu Trp
                405                 410                 415

Met Met Leu Leu Ile Ser Gln Ala Glu
            420                 425

<210> 14
<211> 7551
<212> DNA
<213> Artificial

<220>
<223> phCMV-DCE1E2p7 plasmid

<400> 14
gcggccgctc tagagagctt ggcccattgc atacgttgta tccatatcat aatatgtaca
60

tttatattgg ctcatgtcca acattaccgc catgttgaca ttgattattg actagttatt
120

aatagtaatc aattacgggg tcattagttc atagcccata tatggagttc cgcgttacat
180

aacttacggt aaatggcccg cctggctgac cgcccaacga cccccgccca ttgacgtcaa
240

taatgacgta tgttcccata gtaacgccaa tagggacttt ccattgacgt caatgggtgg
300

agtatttacg gtaaactgcc cacttggcag tacatcaagt gtatcatatg ccaagtacgc
360

cccctattga cgtcaatgac ggtaaatggc ccgcctggca ttatgcccag tacatgacct
420

tatgggactt tcctacttgg cagtacatct acgtattagt catcgctatt accatggtga
480

tgcggttttg gcagtacatc aatgggcgtg gatagcggtt tgactcacgg ggatttccaa
540

gtctccaccc cattgacgtc aatgggagtt tgttttggca ccaaaatcaa cgggactttc
600

caaaatgtcg taacaactcc gccccattga cgcaaatggg cggtaggcgt gtacggtggg
660

aggtctatat aagcagagct cgtttagtga accgtcagat cgcctggaga cgccatccac
720

gctgttttga cctccataga agacaccggg accgatccag cctccggtcg accgatcctg
780

agaacttcag ggtgagtttg gggacccttg attgttcttt ctttttcgct attgtaaaat
840

tcatgttata tggagggggc aaagttttca gggtgttgtt tagaatggga agatgtccct
900

tgtatcacca tggaccctca tgataatttt gtttctttca ctttctactc tgttgacaac
960

cattgtctcc tcttattttc ttttcatttt ctgtaacttt ttcgttaaac tttagcttgc
1020

atttgtaacg aatttttaaa ttcacttttg tttatttgtc agattgtaag tactttctct
1080

aatcactttt ttttcaaggc aatcagggta tattatattg tacttcagca cagttttaga
1140

gaacaattgt tataattaaa tgataaggta gaatatttct gcatataaat tctggctggc
1200

gtggaaatat tcttattggt agaaacaact acaccctggt catcatcctg cctttctctt
1260

tatggttaca atgatataca ctgtttgaga tgaggataaa atactctgag tccaaaccgg
1320

gcccctctgc taaccatgtt catgccttct tctctttcct acagctcctg ggcaacgtgc
1380

tggttgttgt gctgtctcat cattttggca aagaattcct cgacggatcc gatgaaggac
1440

agttctttcc agacattgtt gaattgatct cgatcccgcg aaattaatac gactcactat
1500

agggagacca caacggtttc cctctagcgg gatcaattcc gcccctctcc ctcccccccc
1560

cctaacgtta ctggccgaag ccgcttggaa taaggccggt gtgcgtttgt ctatatgtta
1620

ttttccacca tattgccgtc ttttggcaat gtgagggccc ggaaacctgg ccctgtcttc
1680

ttgacgagca ttcctagggg tctttcccct ctcgccaaag gaatgcaagg tctgttgaat
1740

gtcgtgaagg aagcagttcc tctggaagct tcttgaagac aaacaacgtc tgtagcgacc
1800

ctttgcaggc agcggaaccc cccacctggc gacaggtgcc tctgcggcca aaagccacgt
1860

gtataagata cacctgcaaa ggcggcacaa ccccagtgcc acgttgtgag ttggatagtt
1920

gtggaaagag tcaaatggct ctcctcaagc gtattcaaca aggggctgaa ggatgcccag
1980

aaggtacccc attgtatggg atctgatctg gggcctcggt gcacatgctt tacatgtgtt
2040

tagtcgaggt taaaaaacgt ctaggccccc cgaaccacgg ggacgtggtt ttcctttgaa
2100

aaacacgata ataccatgaa ttccgacctc atggggtaca taccgctcgt cggcgcccct
2160

cttggaggcg ctgccagggc cctggcgcat ggcgtccggg ttctggaaga cggcgtgaac
2220

tatgcaacag ggaaccttcc tggttgcttt ttctctatct tccttctggc cctgctctct
2280

tgcctgactg tgcccgcttc agcctaccaa gtgcgcaatt cctcggggct ttaccatgtc
2340

accaatgatt gccctaattc gagtattgtg tacgaggcgg ccgatgccat cctgcacact
2400

ccggggtgtg tcccttgcgt tcgcgagggt aacgcctcga ggtgttgggt ggcggtgacc
2460

cccacggtgg ccaccaggga cggcaaactc cccacaacgc agcttcgacg tcatatcgat
2520

ctgcttgtcg ggagcgccac cctctgctca gccctctacg tgggggacct gtgcgggtct
2580

gtttttcttg ttggtcaact gtttaccttc tctcccaggc gccactggac gacgcaaagc
2640

tgcaattgtt ctatctatcc cggccatata acgggtcatc gcatggcatg ggatatgatg
2700

atgaactggt cccctacggc agcgttggtg gtagctcagc tgctccggat cccacaagcc
2760

atcatggaca tgatcgctgg tgctcactgg ggagtcctgg cgggcatagc gtatttctcc
2820

atggtgggga actgggcgaa ggtcctggta gtgctgctgt tatttgccgg cgtcgacgcg
2880

gaaacccacg tcaccggggg aagtgccggc cacaccacgg ctgggcttgt tggtctcctt
2940

acaccaggcg ccaagcagaa catccaactg atcaacacca acggcagttg gcacatcaat
3000

agcacggcct tgaactgcaa cgatagcctt accaccggct ggttagcagg gctcttctat
3060

cgccacaaat tcaactcttc aggctgtcct gagaggttgg ccagctgccg acgccttacc
3120

gattttgccc agggctgggg tcccatcagt tatgccaacg gaagcggcct tgacgaacgc
3180

ccctactgtt ggcactaccc tccaagacct tgtggcattg tgcccgcaaa gagcgtgtgt
3240

ggcccggtat attgcttcac tcccagcccc gtggtggtgg gaacgaccga caggtcgggc
3300

gcgcctacct acagctgggg tgcaaatgat acggatgtct tcgtccttaa caacaccagg
3360

ccaccgctgg gcaattggtt cggttgtacc tggatgaact caactggatt caccaaagtg
3420

tgcggagcgc ccccttgtgt catcggaggg gtgggcaaca acaccttgct ctgccccact
3480

gattgcttcc gcaaacatcc ggaagccaca tactctcggt gcggctccgg tccctggatt
3540

acacccaggt gcatggtcga ctacccgtat aggctttggc actatccttg tactatcaat
3600

tacaccatat tcaaagtcag gatgtacgtg ggaggggtcg agcacaggct ggaagcggcc
3660

tgcaactgga cgcggggcga acgctgtgat ctggaagaca gggacaggtc cgagctcagc
3720

ccattgctgc tgtccaccac acagtggcag gtccttccgt gttctttcac gaccctgcca
3780

gccttgtcca ccggcctcat ccacctccac cagaacattg tggacgtgca gtacttgtac
3840

ggggtggggt caagcatcgc gtcctgggcc attaagtggg agtacgtcgt tctcctgttc
3900

cttctgcttg cagacgcgcg cgtctgctcc tgcttgtgga tgatgttact catatcccaa
3960

gcggaggcgg ctttggagaa cctcgtaata ctcaatgcag catccctggc cgggacgcac
4020

ggtcttgtgt ccttcctcgt gttcttctgc tttgcgtggt atctgaaggg taggtgggtg
4080

cccggagcgg tctacgcctt ctacgggatg tggcctctcc tcctgctcct gctggcgttg
4140

cctcagcggg catacgcatg aagatccctg cagctcgaga ggtcaggtgc aggctgccta
4200

tcagaaggtg gtggctggtg tggccaatgc cctggctcac aaataccact gagatctttt
4260

tccctctgcc aaaaattatg gggacatcat gaagcccctt gagcatctga cttctggcta
4320

ataaaggaaa tttattttca ttgcaatagt gtgttggaat tttttgtgtc tctcactcgg
4380

aaggacatat gggagggcaa atcatttaaa acatcagaat gagtatttgg tttagagttt
4440

ggcaacatat gcccatatgc tggctgccat gaacaaaggt tggctataaa gaggtcatca
4500

gtatatgaaa cagccccctg ctgtccattc cttattccat agaaaagcct tgacttgagg
4560

ttagattttt tttatatttt gttttgtgtt atttttttct ttaacatccc taaaattttc
4620

cttacatgtt ttactagcca gatttttcct cctctcctga ctactcccag tcatagctgt
4680

ccctcttctc ttatggagat ccctcgacgg atcggccgca attcgtaatc atgtcatagc
4740

tgtttcctgt gtgaaattgt tatccgctca caattccaca caacatacga gccggaagca
4800

taaagtgtaa agcctggggt gcctaatgag tgagctaact cacattaatt gcgttgcgct
4860

cactgcccgc tttccagtcg ggaaacctgt cgtgccagct gcattaatga atcggccaac
4920

gcgcggggag aggcggtttg cgtattgggc gctcttccgc ttcctcgctc actgactcgc
4980

tgcgctcggt cgttcggctg cggcgagcgg tatcagctca ctcaaaggcg gtaatacggt
5040

tatccacaga atcaggggat aacgcaggaa agaacatgtg agcaaaaggc cagcaaaagg
5100

ccaggaaccg taaaaaggcc gcgttgctgg cgtttttcca taggctccgc ccccctgacg
5160

agcatcacaa aaatcgacgc tcaagtcaga ggtggcgaaa cccgacagga ctataaagat
5220

accaggcgtt tccccctgga agctccctcg tgcgctctcc tgttccgacc ctgccgctta
5280

ccggatacct gtccgccttt ctcccttcgg gaagcgtggc gctttctcat agctcacgct
5340

gtaggtatct cagttcggtg taggtcgttc gctccaagct gggctgtgtg cacgaacccc
5400

ccgttcagcc cgaccgctgc gccttatccg gtaactatcg tcttgagtcc aacccggtaa
5460

gacacgactt atcgccactg gcagcagcca ctggtaacag gattagcaga gcgaggtatg
5520

taggcggtgc tacagagttc ttgaagtggt ggcctaacta cggctacact agaagaacag
5580

tatttggtat ctgcgctctg ctgaagccag ttaccttcgg aaaaagagtt ggtagctctt
5640

gatccggcaa acaaaccacc gctggtagcg gtggtttttt tgtttgcaag cagcagatta
5700

cgcgcagaaa aaaaggatct caagaagatc ctttgatctt ttctacgggg tctgacgctc
5760

agtggaacga aaactcacgt taagggattt tggtcatgag attatcaaaa aggatcttca
5820

cctagatcct tttaaattaa aaatgaagtt ttaaatcaat ctaaagtata tatgagtaaa
5880

cttggtctga cagttaccaa tgcttaatca gtgaggcacc tatctcagcg atctgtctat
5940

ttcgttcatc catagttgcc tgactccccg tcgtgtagat aactacgata cgggagggct
6000

taccatctgg ccccagtgct gcaatgatac cgcgagaccc acgctcaccg gctccagatt
6060

tatcagcaat aaaccagcca gccggaaggg ccgagcgcag aagtggtcct gcaactttat
6120

ccgcctccat ccagtctatt aattgttgcc gggaagctag agtaagtagt tcgccagtta
6180

atagtttgcg caacgttgtt gccattgcta caggcatcgt ggtgtcacgc tcgtcgtttg
6240

gtatggcttc attcagctcc ggttcccaac gatcaaggcg agttacatga tcccccatgt
6300

tgtgcaaaaa agcgggttag ctccttcggt cctccgatcg ttgtcagaag taagttggcc
6360

gcagtgttat cactcatggt tatggcagca ctgcataatt ctcttactgt catgccatcc
6420

gtaagatgct tttctgtgac tggtgagtac tcaaccaagt cattctgaga atagtgtatg
6480

cggcgaccga gttgctcttg cccggcgtca atacgggata ataccgcgcc acatagcaga
6540

actttaaaag tgctcatcat tggaaaacgt tcttcggggc gaaaactctc aaggatctta
6600

ccgctgttga gatccagttc gatgtaaccc actcgtgcac ccaactgatc ttcagcatct
6660

tttactttca ccagcgtttc tgggtgagca aaaacaggaa ggcaaaatgc cgcaaaaaag
6720

ggaataaggg cgacacggaa atgttgaata ctcatactct tcctttttca atattattga
6780

agcatttatc agggttattg tctcatgagc ggatacatat ttgaatgtat ttagaaaaat
6840

aaacaaatag gggttccgcg cacatttccc cgaaaagtgc cacctaaatt gtaagcgtta
6900

atattttgtt aaaattcgcg ttaaattttt gttaaatcag ctcatttttt aaccaatagg
6960

ccgaaatcgg caaaatccct tataaatcaa aagaatagac cgagataggg ttgagtgttg
7020

ttccagtttg gaacaagagt ccactattaa agaacgtgga ctccaacgtc aaagggcgaa
7080

aaaccgtcta tcagggcgat ggcccactac gtgaaccatc accctaatca agttttttgg
7140

ggtcgaggtg ccgtaaagca ctaaatcgga accctaaagg gagcccccga tttagagctt
7200

gacggggaaa gccggcgaac gtggcgagaa aggaagggaa gaaagcgaaa ggagcgggcg
7260

ctagggcgct ggcaagtgta gcggtcacgc tgcgcgtaac caccacaccc gccgcgctta
7320

atgcgccgct acagggcgcg tcccattcgc cattcaggct gcgcaactgt tgggaagggc
7380

gatcggtgcg ggcctcttcg ctattacgcc agctggcgaa agggggatgt gctgcaaggc
7440

gattaagttg ggtaacgcca gggttttccc agtcacgacg ttgtaaaacg acggccagtg
7500

agcgcgcgta atacgactca ctatagggcg aattggagct ccaccgcggt g
7551

<210> 15
<211> 682
<212> PRT
<213> Artificial

<220>
<223> delta CE E2p7 polyprotein

<400> 15

Met Asn Ser Asp Leu Met Gly Tyr Ile Pro Leu Val Gly Ala Pro Leu
1               5                   10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Tyr
    50                  55                  60

Gln Val Arg Asn Ser Ser Gly Leu Tyr His Val Thr Asn Asp Cys Pro
65                  70                  75                  80

Asn Ser Ser Ile Val Tyr Glu Ala Ala Asp Ala Ile Leu His Thr Pro
                85                  90                  95

Gly Cys Val Pro Cys Val Arg Glu Gly Asn Ala Ser Arg Cys Trp Val
            100                 105                 110

Ala Val Thr Pro Thr Val Ala Thr Arg Asp Gly Lys Leu Pro Thr Thr
        115                 120                 125

Gln Leu Arg Arg His Ile Asp Leu Leu Val Gly Ser Ala Thr Leu Cys
    130                 135                 140

Ser Ala Leu Tyr Val Gly Asp Leu Cys Gly Ser Val Phe Leu Val Gly
145                 150                 155                 160

Gln Leu Phe Thr Phe Ser Pro Arg Arg His Trp Thr Thr Gln Ser Cys
                165                 170                 175

Asn Cys Ser Ile Tyr Pro Gly His Ile Thr Gly His Arg Met Ala Trp
            180                 185                 190

Asp Met Met Met Asn Trp Ser Pro Thr Ala Ala Leu Val Val Ala Gln
        195                 200                 205

Leu Leu Arg Ile Pro Gln Ala Ile Met Asp Met Ile Ala Gly Ala His
    210                 215                 220

Trp Gly Val Leu Ala Gly Ile Ala Tyr Phe Ser Met Val Gly Asn Trp
225                 230                 235                 240

Ala Lys Val Leu Val Val Leu Leu Leu Phe Ala Gly Val Asp Ala Glu
                245                 250                 255

Thr His Val Thr Gly Gly Ser Ala Gly His Thr Thr Ala Gly Leu Val
            260                 265                 270

Gly Leu Leu Thr Pro Gly Ala Lys Gln Asn Ile Gln Leu Ile Asn Thr
        275                 280                 285

Asn Gly Ser Trp His Ile Asn Ser Thr Ala Leu Asn Cys Asn Asp Ser
    290                 295                 300

Leu Thr Thr Gly Trp Leu Ala Gly Leu Phe Tyr Arg His Lys Phe Asn
305                 310                 315                 320

Ser Ser Gly Cys Pro Glu Arg Leu Ala Ser Cys Arg Arg Leu Thr Asp
                325                 330                 335

Phe Ala Gln Gly Trp Gly Pro Ile Ser Tyr Ala Asn Gly Ser Gly Leu
            340                 345                 350

Asp Glu Arg Pro Tyr Cys Trp His Tyr Pro Pro Arg Pro Cys Gly Ile
        355                 360                 365

Val Pro Ala Lys Ser Val Cys Gly Pro Val Tyr Cys Phe Thr Pro Ser
    370                 375                 380

Pro Val Val Val Gly Thr Thr Asp Arg Ser Gly Ala Pro Thr Tyr Ser
385                 390                 395                 400

Trp Gly Ala Asn Asp Thr Asp Val Phe Val Leu Asn Asn Thr Arg Pro
                405                 410                 415

Pro Leu Gly Asn Trp Phe Gly Cys Thr Trp Met Asn Ser Thr Gly Phe

420                 425                 430

Thr Lys Val Cys Gly Ala Pro Pro Cys Val Ile Gly Gly Val Gly Asn
        435                 440                 445

Asn Thr Leu Leu Cys Pro Thr Asp Cys Phe Arg Lys His Pro Glu Ala
    450                 455                 460

Thr Tyr Ser Arg Cys Gly Ser Gly Pro Trp Ile Thr Pro Arg Cys Met
465                 470                 475                 480

Val Asp Tyr Pro Tyr Arg Leu Trp His Tyr Pro Cys Thr Ile Asn Tyr
                485                 490                 495

Thr Ile Phe Lys Val Arg Met Tyr Val Gly Gly Val Glu His Arg Leu
            500                 505                 510

Glu Ala Ala Cys Asn Trp Thr Arg Gly Glu Arg Cys Asp Leu Glu Asp
        515                 520                 525

Arg Asp Arg Ser Glu Leu Ser Pro Leu Leu Leu Ser Thr Thr Gln Trp
    530                 535                 540

Gln Val Leu Pro Cys Ser Phe Thr Thr Leu Pro Ala Leu Ser Thr Gly
545                 550                 555                 560

Leu Ile His Leu His Gln Asn Ile Val Asp Val Gln Tyr Leu Tyr Gly
                565                 570                 575

Val Gly Ser Ser Ile Ala Ser Trp Ala Ile Lys Trp Glu Tyr Val Val
            580                 585                 590

Leu Leu Phe Leu Leu Leu Ala Asp Ala Arg Val Cys Ser Cys Leu Trp
        595                 600                 605

Met Met Leu Leu Ile Ser Gln Ala Glu Ala Ala Leu Glu Asn Leu Val
    610                 615                 620

Ile Leu Asn Ala Ala Ser Leu Ala Gly Thr His Gly Leu Val Ser Phe
625                 630                 635                 640

Leu Val Phe Phe Cys Phe Ala Trp Tyr Leu Lys Gly Arg Trp Val Pro
                645                 650                 655

Gly Ala Val Tyr Ala Phe Tyr Gly Met Trp Pro Leu Leu Leu Leu
            660                 665                 670

Leu Ala Leu Pro Gln Arg Ala Tyr Ala Glx
        675                     680